(19) Europäisches Patentamt
European Patent Office
Office européen des brevets

(11) EP 2 636 748 A1

(12) # EUROPEAN PATENT APPLICATION
published in accordance with Art. 153(4) EPC

(43) Date of publication:
**11.09.2013 Bulletin 2013/37**

(21) Application number: **11837764.7**

(22) Date of filing: **07.11.2011**

(51) Int Cl.:
***C12P 19/26*** (2006.01) ***A61K 47/36*** (2006.01)
***C08B 37/00*** (2006.01)

(86) International application number:
**PCT/JP2011/006223**

(87) International publication number:
**WO 2012/060110 (10.05.2012 Gazette 2012/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.11.2010 JP 2010249082**

(71) Applicant: **Ezaki Glico Co., Ltd.**
**Osaka-shi**
**Osaka 555-8502 (JP)**

(72) Inventors:
- **TAKAHA, Takeshi**
  **Osaka-shi**
  **Osaka 555-8502 (JP)**
- **KUBO, Akiko**
  **Osaka-shi**
  **Osaka 555-8502 (JP)**
- **YANASE, Michiyo**
  **Osaka-shi**
  **Osaka 555-8502 (JP)**

(74) Representative: **UEXKÜLL & STOLBERG**
**Patentanwälte**
**Beselerstraße 4**
**22607 Hamburg (DE)**

(54) **AMINO SUGAR-CONTAINING GLUCAN, METHOD FOR PRODUCING SAME AND USE OF SAME**

(57) An object of the present invention is to provide a glucosamine-containing glucan, a modified product and a conjugate thereof.

The glucosamine-containing glucan of the present invention is a glucosamine-containing glucan wherein the glucan has a plurality of non-reducing ends and at least one glucosamine residue is bound via an α-1,4-bond to each of two or more non-reducing ends of the branched α-1,4-glucan, but no glucosamine residue is present at a position other than the non-reducing ends of the branched α-1,4-glucan, wherein the degree of polymerization of the glucan is 15 or more and 4 x $10^5$ or less. Theglucosamine-containingglucan of the present invention can be provided by allowing an α-glucan phosphorylase to act on an aqueous solution comprising a branched α-1,4-glucan and glucosamine-1-phosphate.

EP 2 636 748 A1

**Description**

TECHNICAL FIELD

The present invention relates to a glucan having at least one (preferably two or more) aminomonosaccharide residue on each of at least one non-reducing end, a modified product and a conjugate thereof, as well as a method for producing the same, and utilization of the same. More preferably, the present invention relates to a glucan having at least one (preferably two or more) glucosamine residue on each of at least one non-reducing end, a modified product and a conjugate thereof, as well as a method for producing the same, and utilization of the same. The glucan, amodified product and a conjugate thereof of the present invention can have a function to non-covalently bond to a nucleic acid to increase the apparent molecular weight of the nucleic acid.

BACKGROUND ART

A medically effective ingredient of medicaments is rapidly changing from a chemically synthesized stable low-molecular weight compound to an unstable substance which is easily degraded in blood, such as a protein, an antibody and a nucleic acid. For this reason, there is a necessity of stabilizing these unstable medically effective ingredients to keep the blood concentration of the medically effective ingredient high for an elongated time. In addition, in order to decrease side effects of drugs, a necessity of delivering drugs to a target tissue efficiently has been increasing. Under such a background, a so-called drug delivery system (DDS) technique has been utilized in earnest (Non-Patent Documents 1 to 4). A DDS technique refers to a technique and a system for allowing a medically effective ingredient to act on "a required site" at "a required amount" for "a required period of time", that is, controlling it with an ideal pharmacokinetics for a drug to maximally exert the effect.

In the DDS technique, a modifying material for a medically effective ingredient is important. The term "modifying material for a medically effective ingredient" in the present specification refers to a material which modifies a medically effective ingredient by covalently binding, or via non-covalent type interaction, with a medically effective ingredient. By utilizing the modifying material, a variety of properties (for example, pharmacokinetics (for example, absorption, distribution, metabolism and excretion), pharmacological effect, stability and the like) of the medically effective ingredient can be modified. As a substance which has been used previously as the modifying material for the medically effective ingredient, there are a variety of substances, most generally it is a macromolecular material. For example, polyethylene glycol (PEG) which is a synthetic macromolecule, and derivatives thereof are widely utilized as a modifying material for medically effective ingredients. Many medically effective ingredient-modifying materials having a functional group for binding the medically effective ingredient on a terminus of a PEG chain have been developed, and such modifying materials are actually utilized in producing a medicament. Specific application examples include pegylated interferon α (product name: PEGASYS). Since interferon α has a small molecular weight and is easily excreted into urine, there was a problem that it has a short half-life in blood. However, the half-life in blood was successfully enhanced dramatically by covalently binding interferon α to a PEG chain having a molecular weight of 40,000 to form an interferon α-conjugate having a high molecular weight. As described above, the remarkable effect is recognized in the modification of the medically effective ingredient or the nanoparticulate carrier for DDSs, with a macromolecular material.

However, on the other hand, a problem has been pointed out. For example, when a high- molecular weight synthetic macromolecule which has no degradability in a living body and will not undergo renal glomerular filtration is administered to blood, there is a risk that the macromolecule is accumulated in a particular organ and a risk that side effects due to the accumulation are generated. The reason is that a molecule having a molecular weight of a few tens thousands or less present in blood, undergoes renal glomerular filtration and is rapidly excreted into urine, but a molecule having a molecular weight of a few tens thousands or more does not undergo renal glomerular filtration and its excretion into urine is limited. For this reason, a modifying material of the medically effective ingredient which can be safely utilized is expected.

In addition, when PEG is utilized as a carrier, PEG is usually linked to a medically effective ingredient through a covalent bond. While a covalent bond is excellent in stability, since a portion of the structure of the medically effective ingredient changes, there is a problem that the physiological activity of the medically effective ingredient changes. In addition, when the medically effective ingredient has a plurality of functional groups, there is a problem that groups having different binding position and binding number of PEG chains are synthesized. Such problems become fatal problem in a biomedicine, especially in a proteinaceous medicament and a peptidic medicament. The reason is that it is very difficult to produce products of the same quality reproducibly. Chemical creativity and originality are needed to covalently bind a PEG molecule to a specific amino acid residue, and there are various problems that a specific amino acid sequence may be required, the kind of the medically effective ingredient maybe limited, and the like.

On the other hand, the development of a nucleic acid medicament such as an siRNA and an RNA aptamer has been progressing in recent years. Since a DNA and an RNA, which are components of a nucleic acid medicament, are

easily degraded by DNA degrading enzyme and RNA degrading enzyme, respectively, it is required to prevent degradation using a suitable carrier. In addition, in the case of a nucleic acid medicament comprising a low-molecular weight nucleic acid medically effective ingredient, it is required to bind it with a macromolecular carrier to extend the blood retention time. Further, it is preferable that these macromolecular carriers for a nucleic acid can be given a targeting function, if necessary. A macromolecular carrier which is safe and has nucleic acid binding property and can solve such problems has been sought.

Polysaccharides have been used as a food raw material for a long time and, in recent years, have begun to be paid attention as a macromolecular material which is environment friendly, as a safe material having biocompatibility and, further, as a functional material. The naturally occurring polysaccharides can be classified into neutral polysaccharides typified by a starch,cellulose, and dextrin; and acidic polysaccharides typified by alginic acid and hyaluronic acid. On the other hand, basic polysaccharides do not occur naturally. A method for producing basic polysaccharides artificially includes a method by which chitin, a naturally derived polysaccharide which is a homopolymer of N-acetylglucosamine, is chemically deacetylated. Such a polysaccharide is generally referred to as chitosan, which is the only basic polysaccharide which is industrially applicable.

Chitosan has many amino groups in the molecule, and strongly binds to a nucleic acid to form a very huge chitosan:nucleic acid associate referred to as polyplex. There is a problem that such a bond between a polyanion and a polycation is so strong that the dissociation of them is difficult.

Chitosan is produced by a method such as boiling chitin in a concentrated alkali solution to convert the acetamide group on the carbon at 2-position in a chitin backbone into a free primary amino group. In this method, however, chitosan is degraded into molecules having lower molecular weight by the cleavage of the chitin main chain, and the strict control of a complete deacetylation is difficult, therefore, a polysaccharide wherein a plurality of glucosamines and N-acetylglucosamines are randomly arranged. The number of glucosamines in this polysaccharide molecule can not be controlled strictly. In addition, in this method, it is not possible to selectively deacetylate only the non-reducing ends of this polysaccharide to produce a polysaccharide wherein glucosamines are bound only to non-reducing ends. That is, in this method, it is not possible to control the strength and ease of dissociation of the bond between chitosan and a nucleic acid. In addition, chitin and chitosan cannot be degraded rapidly in a body. Therefore, chitin and chitosan, which have molecular weights not less than the fractionation size of renal glomerular filtration, have a risk of accumulating in a body. As described above, while chitosan, which is the only cationic polysaccharide, has nucleic acid binding property, it has a limited utility as a carrier for nucleic acid medicaments.

An amino sugar-containing glucan wherein amino sugar residues are bound only to the non-reducing ends has not been found in nature. Even using a chemical method, the synthesis of an amino sugar-containing glucan wherein amino sugar residues are bound only to the non-reducing ends is not possible, and the chemical synthesis of an amino sugar-containing glucan wherein amino sugar residues are bound only to the non-reducing ends has not been disclosed. In addition, a polysaccharide wherein the number of the bound amino sugar residues can be strictly controlled is not known.

Non- Patent Document 5 discloses that glucosamine- 1- phosphate (GlcN- 1- P) can be synthesized by a chemical reduction reaction of 2- azido- 2- deoxy- glucopyranosyl- 1- phosphate, and that a maltooligosaccharide having a glucosamine residue at the non- reducing end is produced by allowing potato- derived $\alpha$- glucan phosphorylase to act on a mixture of glucosamine- 1- phosphate and a maltooligosaccharide. However, Non- Patent Document 5 does not disclose or suggest applying a similar method to a high- molecular weight glucan and a branched glucan. It does not disclose that the number of the glucosamine residues in a glucan molecule can be controlled. Further, it does not disclose or suggest that controlling the number of the glucosamine residue in a glucan molecule has an important effect on the strength and form of the bond with a negatively- charged substance such as a nucleic acid. Non- Patent Document 5 does not suggest or disclose that a glucan having one glucosamine residue at the non- reducing end has a binding property to a negatively- charged substance such as a nucleic acid, and that such a glucan has a function to increase the apparent molecular weight of a negatively- charged substance such as a nucleic acid. In addition, when glucosamine-containing glucan is obtained by the method described in Non- Patent Document 5, it is not possible to separate the glucosamine- 1- phosphate used in the reaction from the product glucosamine- containing glucan. Since a medicinal material does not allow the mixing of impurities, a glucosamine- containing glucan containing unremoved glucosamine- 1- phosphate has a critical drawback of unavailability as a medicament.

[Prior Art Documents]

[Non-Patent Documents]

[Non-Patent Document 1] Hiroaki Okada, "Drug delivery using functional DDS carriers" Chapter 1, General Statement: Kinousei DDS carrier wo mochiita seizai sekkei ni yoru soyaku (Drug Discovery by Preparation Design Using

Functional DDS Carrier), CMC Publishing Co., Ltd., 2008, 1-23
[Non-Patent Document 2] Masayuki Yokoyama, "Polymeric materials for drug carriers, Special Topic, DDS ni riyou sareru kobunshi kagaku (Polymer Chemistry Utilized in DDS)", Drug Delivery System 23-6, 2008: 610-617
[Non-Patent Document 3] Maria Laura Immordino et al., International Journal of Nanomedicine 2006: 1(3) 297-315
[Non-Patent Document 4] J. Milton Harris and Robert B. Chess, NATURE REVIEWS, DRUG DISCOVERY, VOLUME 2, MARCH 2003, 214-221 [Non-Patent Document 5] Nawaji et al., Carbohydr. Res. 2008, 343, 2692-2696

SUMMARY OF THE INVENTION

PROBLEMS TO BE SOLVED BY THE INVENTION

The present invention is intended to solve the above-mentioned problems.

An ideal modifying material for the negatively-charged medically effectiveingredientwhich can be safely utilized is thought to have the following characteristics:

(1) The modifying material shall be a macromolecular material which can be degraded in a living body;
(2) The modifying material shall have such a stable quality that it can be usable as a medicament. That is, the structure can be specified, and a modifying material having the same quality shall be able to produce every time;
(3) The modifying material shall be a macromolecular material which can have the arbitrary number of amino groups which can bind to or interact with a negatively-charged medically effective ingredient at specific sites in the molecule.

A modifying material for a nucleic acid is thought to be preferable to have the following characteristics:

(4) The modifying material shall have a nucleic acid binding property and have a function to increase the apparent molecular weight of the nucleic acid.

The present inventors thought that a macromolecular substance most excellent as the modifying material for the medically effective ingredient is a glucan (in the present specification, the glucan refers to an α- 1, 4- glucan, and an α- 1, 4- glucan which is branched with an α- 1, 6- bond (s) ) . Since glycogen or a starch, which is accumulated in an animal's and plant's body as a polysaccharide for storage, is one kind of glucan, it is a component which is always present in a body of a human, and is excellent in biocompatibility. Further, the glucan undergoes hydrolysis by α- amylase in a body, and is converted into glucose or a maltooligosaccharide being a component which is always present in a body. For this reason, the glucan can be said to be the safest macromolecular material.

The glucan has another advantage that design of its structure is relatively easy. Methods for controlling the molecular weight, degree of branching, cyclization and the like of the glucan are known. Completely linear glucans in which glucose residues are bound only with an α- 1, 4- bond, glucans which are branched at high frequency by some glucose residues bound with an α- 1, 6- bond, and the like are available. In a branched glucan, every time an α- 1, 6- bond is increased, one new non- reducing end is generated. The number of α- 1, 6- bonds can be suitably adjusted according to desire upon synthesis of a glucan molecule.

On the other hand, the greatest problem when the glucan is utilized as the modifying material for the medically effective ingredient is that it does not have a functional group which can bind to or interact with the medically effective ingredient. It is possible to introduce a cationic or anionic functional group into a large number of hydroxyl groups present in a glucan by a chemical reaction. However, when such a procedure is used, the position of introduction of the functional group to the glucan is random and it is difficult to obtain a modifying material of the same quality. Therefore, this procedure is not preferable for utilization in medicaments.

For this reason, it is sought to provide a modifying material suitable for utilizing in medicaments for a medically effective ingredient, especially for a negatively-charged medically effective ingredient.

MEANS FOR SOLVING THE PROBLEMS

In order to solve the aforementioned problems, the present inventors thought that specific introduction of an amino sugar (for example, glucosamine) into a non-reducing end of a high-molecular weight glucan chain is the extremely ideal method of modifying a glucan. If an amino sugar (for example, glucosamine) can be selectively introduced into a non-reducing end of a high-molecular weight glucan, since the introduction position is not random, the same quality material can be produced reproductively, and the resulting product is suitable for utilization in medicaments. In addition, there is no anxiety for toxicity by administering an amino sugar. The reason is that glucosamine is a monosaccharide having an amino group, sold as a supplement or a health food as a single component or as a mixture with chondroitin (chondroitin sulfate), and has low anxiety for safety. Further, the amino group of an amino sugar (for example, glucosamine) is available for binding with a medically effective ingredient. Furthermore, an amino sugar (for example, glucosamine) becomes a sugar residue having a positive charge in an aqueous solution. For this reason, by introducing an amino sugar residue into a glucan chain, it is possible to give a property to interact with a negatively-charged molecule (for example, a medically effective ingredient which is negatively charged in an aqueous solution) to a glucan chain. Example of negatively-charged molecules includes a nucleic acid. A biodegradable carrier which binds to a molecule negatively charged in an aqueous solution and has no anxiety for staying in a body has not been known to date. For this reason, the glucan of the present invention can be said to be a useful carrier in a bio DDS technique using a nucleic acid which has been paid attention in recent years.

The present inventors found that α- glucan phosphorylase can utilize as a substrate an amino sugar- 1- phosphate (for example, glucosamine- 1- phosphate) which is not its original substrate, can transfer an amino sugar residue to a non- reducing end of a glucan receptor, and in spite of this, this enzyme can hardly catalyze the reverse reaction thereof, and completed the present invention based on this finding. α- glucan phosphorylase is an enzyme which, when acts on glucose- 1- phosphate which is the original substrate, catalyzes both a reaction wherein a glucose residue is transferred to a glucan receptor (glucan synthesizing reaction) and a reaction wherein the glucose on the non- reducing end of a glucan is phosphorolysed to generate glucose- 1- phosphate (glucan degrading reaction) . However, surprisingly, when this enzyme uses an amino sugar- 1- phosphate (for example, glucosamine- 1- phosphate) as a substrate, this enzyme catalyzes the reaction wherein an amino sugar residue is transferred to a glucan receptor (glucan synthesizing reaction) , but hardly catalyzes the reaction wherein an amino sugar residue on the non- reducing end of a glucan is phosphorolysed to generate an amino sugar- 1- phosphate (glucan degrading reaction) . While glucan degrading reaction releases again the bound amino sugar residue, the present inventors found that in the method of the present invention, α- glucan phosphorylase hardly catalyzes the glucan degrading reaction. That is, the catalytic activity for the synthesizing reaction is much higher than the catalytic activity for the degradation reaction. Due to this characteristic, if a glucan has a plurality of non- reducing ends, α- glucan phosphorylase can bindaminosugarresidues (forexample, glucosamine residues) one by one to the plurality of non- reducing ends of the glucan. By utilizing this characteristic of this enzyme, the amino sugar- containing glucan of the present invention became producible for the first time. Especially, it could be obtained for the first time by utilizing this characteristic of this enzyme that a non- reducing end- modified glucan having high frequency of (for example, 50% or more) binding rate of amino sugar residues (for example, glucosamine residues) to the non- reducing ends, starting with a glucan having a large number of (for example, 5 or more) non- reducing ends.

In the present specification, an amino sugar means that it is a sugar having an amino group. The sugar constructing the backbone monosaccharide of an amino sugar can be a monosaccharide (for example, glucose) or an oligosaccharide. In the present specification, an oligosaccharide means a compound wherein 2 or more and 10 or less monosaccharides are linked. The degree of polymerization of an amino oligosaccharide bound to one non-reducing end of the amino sugar-containing glucan of the present invention can be, for example, about 2 or more, about 3 or more, about 4 or more, about 5 or more or the like, and can be, for example, about 10 or less, about 9 or less, about 8 or less, about 7 or less, about 6 or less, about 5 or less, about 4 or less, about 3 or less, about 2 or less or the like. In one embodiment, the amino oligosaccharide bound to one non-reducing end of the amino sugar-containing glucan of the present invention has 2 sugars (that is, a dimer). In the present specification, an amino monosaccharide refers to an amino sugar wherein the sugar backbone is a monosaccharide. In the present specification, an amino oligosaccharide refers to an amino sugar wherein the sugar backbone is an oligosaccharide.

In addition, since a bond by an electrostatic interaction does not bind molecules directly, it is thought that if a glucan is degraded from the conjugate to separate the medically effective ingredient, the medically effective ingredient retains the original stereostructure again. For this reason, the bond by an electrostatic interaction is thought as an ideal binding mode between a medically effective ingredient and a carrier. However, the bond by an electrostatic interaction is prone to be affected by the concentration of salts, and expected to be unstable in a living body. Therefore, it is desirable to control strictly the position and number of amino sugars (for example, glucosamines) bound to a glucan molecule in order to control the strength of the bond with a nucleic acid and the form of the associate.

Further, the present inventors found that a glucosamine- containing glucan wherein at least one glucosamine residue is bound to each of one or more (preferably two or more) non- reducing ends of a glucan has a function to non-covalently bind to a nucleic acid to increase the apparent molecular weight of the nucleic acid.

Furthermore, the present inventors found that the strength of the bond with a nucleic acid and the form of the associate can be controlled by controlling the number ofaminomonosaccharide residues such as glucosamine residues bound to a glucan molecule.

The present inventors found that a glucan wherein amino sugar residues are bound only to the ends of a plurality of glucan chains having appropriate degree of freedom can be produced, and that a medically effective ingredient and a carrier can be bound via a stable electrostatic interaction, by development of a novel amino sugar-containing glucan.

In the amino sugar- containing glucan, a modified product thereof and a conjugate thereof of the present invention, an amino sugar residue is bound only to a non- reducing end of the glucan. In preferred embodiments of the present invention, the amino sugar residue is glucosamine residue, galactosamine residue or mannosamine residue,

or an oligomeric (for example, a dimeric) residue of sole or the combination thereof, and most preferably, glucosamine residue. Intheglucosamine- containingglucan, a modified product thereof and a conjugate thereof of the present invention, a glucosamine residue is bound only to a non- reducing end of the glucan. In the present invention, transfer of an amino sugar (for example, glucosamine) residue is performed using α- glucan phosphorylase (EC 2.4.1.1) . For this reason, binding of an amino sugar (for example, glucosamine) residue to a glucan is an α- 1, 4- bond. That is, the carbon atom at 4- potision of a glucosyl residue at the end of glucan and the carbon atom at 1- position of an amino sugar (for example, glucosamine) residue are α- bound through an oxygen atom. An enzyme which can transfer an amino sugar (for example, glucosamine) to a glucan directly in a high yield has not been found other than this enzyme. Since the present amino sugar- containing glucan, a modified product thereof and a conjugate thereof have an amino sugar on a terminus, a glucan terminus comes to be positively charged in an aqueous solution, and a physicochemical nature of the glucan is changed. The present amino sugar- containing glucan, a modified product thereof and a conjugate thereof are expected to be widely utilized in foods, cosmetics, medicaments and the like.

The introduction amount of amino sugar (for example, glucosamine) residues to a glucan can be controlled by the branching frequency of the glucan used and the frequency of introduction of amino sugar (for example, glucosamine) residues to a non-reducing end. When one wants to increase an amount of introduction of an amino sugar (for example, glucosamine) residue into a glucan, it is possible to increase the introduction amount by using a glucan having a high branching frequency and increasing the frequency of introduction of amino sugars (for example, glucosamines) to the non-reducing end. When one wants to decrease an amount of introduction of an amino sugar (for example, glucosamine) residue into a glucan, it is possible to decrease the introduction amount by using a glucan having a low branching frequency or decreasing the frequency of introduction of amino sugars (for example, glucosamines) to the non-reducing end. The lower limit of the introduction amount of amino sugar (for example, glucosamine) residues to a glucan is the state wherein one amino sugar (for example, glucosamine) residue is introduced per glucan molecule, which can be attained by introducing an amino sugar (for example, glucosamine) residue to the non-reducing end of the glucan having no branching. Further, since the introduction position is at an end, it is thought to exert no influence on degradability of a glucan by α-amylase. In the case of a glucan which is highly branched, since non-reducing ends are distributed in an outermost layer of a glucan molecule, introduced amino sugar (for example, glucosamine) residues are distributed in an outermost layer of a glucan molecule after introduction of an amino sugar (for example, glucosamine) residue, and this is ideal for interaction and binding with the medically effective ingredient. As described above, a glucan which has an amino sugar (forexample, glucosamine) residue selectively bound to a non-reducing end can be an excellent modifying material for the medically effective ingredient.

For example, the present invention provides the followings:

(Item 1)
An amino sugar- containing glucan, wherein at least one amino monosaccharide residue is bound via an α- 1, 4- bond to each of at least one non- reducing end of a glucan, but no amino sugar residue is present at the position other than the non- reducing end of the glucan, wherein the glucan is a branched α- 1, 4- glucan or a linear α- 1, 4- grucan, and the degree of polymerization of the glucan is about 10 or more and about 1 x $10^5$ or less, and preferably about 15 or more and about 4 x $10^5$ or less.
(Item 2)
The amino sugar- containing glucan according to item 1, wherein the glucan is a branched α- 1, 4- glucan, the glucan has a plurality of non- reducing ends, and at least one amino monosaccharide residue is bound via an α- 1, 4- bond to each of at least one (preferably two ore more) non- reducing ends of the branched α- 1, 4- glucan. That is, the amino sugar- containing glucan is an amino sugar- containing branched glucan wherein the glucan has a plurality of non- reducing ends and at least one amino monosaccharide residue is bound via an α- 1, 4- bond to each of at least one (preferably two or more) non- reducing ends of the branched α- 1, 4- glucan, but no amino sugar residue is present at the position other than the non- reducing end of the branched α- 1, 4- glucan, wherein the degree of polymerization of the branched α- 1, 4- glucan is about 10 or more and about 1 x $10^5$ or less, and about 15 or more and about 4 x $10^5$ or less.
(Item 3)
The amino sugar- containing glucan according to item 2, wherein the branched α- 1, 4- glucan is selected from the group consisting of a branched maltooligosaccharide, a starch, amylopectin, glycogen, dextrin, enzymatically synthesized branched glucan and highly branched cyclic glucan.
(Item 4)
A hydroxyl group-modified product of the amino sugar-containing glucan according to any one of items 1 to 3, wherein the modification on the hydroxyl group is a modification on some or all of alcoholic hydroxyl groups of the glucan, and the modification on the hydroxyl group is independently selected from the group consisting of hydroxyalkylation, alkylation, acetylation, carboxymethylation, sulfation and phosphorylation.
(Item 5)

A reducing end-modified product of the amino sugar-containing glucan according to any one of items 1 to 3 or a hydroxyl group-modified product thereof.
(Item 6)
An aminogroup-modified productofthe amino sugar-containing glucan according to any one of items 1 to 3, a hydroxyl group-modified product thereof or a reducing end-modified product thereof, wherein the modification on the amino group is a modification on some or all of amino groups of the amino monosaccharide residues, the modification on the amino group is attained by a reaction of the amino group and an amino group-modifying reagent, and the amino group-modifying reagent has at least one carboxyl group and at least one other functional group.
(Item 7)
A non-reducing end-modified product of the amino sugar-containing glucan according to any one of items 1 to 3, a hydroxyl group-modified product thereof, a reducing end-modified product thereof or an amino group-modified product thereof, wherein a targeting molecule is bound to at least one of non-reducing ends to which the glucosamine residue of the glucan is not bound, or to the 4-position of the glucosamine residue, wherein the targeting molecule is selected from the group consisting of mannose, galactose, glucuronic acid, N-acetylglucosamine, xylose, fucose, galactosamine, an antibody, an antibody fragment, a receptor, a receptor fragment and a receptor ligand.
(Item 8)
A method for producing an amino sugar- containing glucan, characterized by allowing an $\alpha$- glucan phosphorylase to act on an aqueous solution comprising a glucan and an amino sugar- 1- phosphate, wherein the glucan is a branched $\alpha$- 1, 4- glucan or a linear $\alpha$- 1, 4- glucan, and the degree of polymerization of the glucan is about 10 or more and about 1 x $10^5$ or less, and preferably about 15 or more and about 4 x $10^5$ or less.
(Item 9)
The method according to item 8, wherein the $\alpha$- glucan phosphorylase has 95% or more sequence identity with the amino acid sequence of $\alpha$- glucan phosphorylase derived from *Aquifex aeolicus* VF5, and has activity of transferring an amino sugar to a non- reducing end of a branched glucan to form an $\alpha$- 1, 4- bond.
(Item 10)
A medicament comprising the amino sugar-containing glucan according to any one of items 1 to 3, a hydroxyl group-modified product thereof, a reducing end-modified product thereof, an amino group-modified product thereof, or a non-reducing end-modified product thereof, and a medically effective ingredient.
(Item 11)
The medicament according to item 10, wherein the medically effective ingredient is selected from the group consisting of a low-molecular weight organic compound, a protein, a peptide, an antibody, an antibody fragment, a receptor, a receptor fragment, a DNA, an RNA, a siRNA, an miRNA and an RNA aptamer.
(Item 12)
A composition for clinical diagnosis comprising the amino sugar-containing glucan according to any one of items 1 to 3, a hydroxyl group-modified product thereof, a reducing end-modified product thereof, an amino group-modified product thereof, or a non-reducing end-modified product thereof.
(Item 13)
A nanoparticulate carrier for a DDS comprising the amino sugar-containing glucan according to any one of items 1 to 3, a hydroxyl group-modified product thereof, a reducing end-modified product thereof, an amino group-modified product thereof, or a non-reducing end-modified product thereof.
(Item 14)
The carrier according to item 13, wherein the nanoparticulate carrier for a DDS is selected from the group consisting of a liposome, a virus particle, a macromolecule micelle and a nanogel composed of macromolecule bearing hydrophobic groups.
(Item 15)
A complex formed with a nucleic acid molecule comprising a gene which can be expressed in a cell and the amino sugar-containing branched glucan according to item 1.
(Item 16)
The complex according to item 15, wherein the nucleic acid molecule is selected from the group consisting of a DNA, an RNA, a siRNA, an miRNA and an RNA aptamer.
(Item 17)
A complex formed with the complex carrier according to item 15 or 16, and a cationic polymer or a cationic lipid.
(Item 18)
The complex according to item 17, wherein the cationic polymer comprises at least one cationic polymer selected from the group consisting of polyethyleneimine, polylysine, polyarginine, polyamidoamine dendrimer, poly (aminostyrene) , chitosan, a cationic glucan and DEAE- dextran.
(Item 19)
A method for delivering a nucleic acid molecule into an isolated cell, comprising contacting the complex according

to any one of items 15 to 18 with the cell.

In one embodiment, the amino sugar is glucosamine, and in that case, the present invention is as follows:

(Item 1A)
A glucosamine- containing glucan, wherein at least one glucosamine residue is bound via an α- 1, 4- bond to each of at least one non- reducing end of a glucan, but no glucosamine residue is present at the position other than the non- reducing end of the glucan, wherein the glucan is a branched α- 1, 4- glucan or a linear α- 1, 4- grucan, and the degree of polymerization of the glucan is about 10 or more and about 1 x $10^5$ or less, and preferably about 15 or more and about 4 x $10^5$ or less. That is, the glucosamine- containing glucan is a glucosamine- containing branched glucan wherein the glucan has a plurality of non- reducing ends and at least one glucosamine residue is bound via an α- 1, 4- bond to each of at least one (preferably two or more) non- reducing ends of the branched α- 1, 4- glucan, but no glucosamine residue is present at the position other than the non- reducing ends of the glucan, wherein the degree of polymerization of the branched α- 1, 4- glucan is about 10 or more and about 1 x $10^5$ or less, and preferably about 15 or more and about 4 x $10^5$ or less.
(Item 2A)
The glucosamine- containing glucan according to item 1A, wherein the glucan is a branched α- 1, 4- glucan, the glucan has a plurality of non- reducing ends, and at least one glucosamine residue is bound to each of at least one non- reducing end of the branched α- 1, 4- glucan.
(Item 3A)
The glucosamine- containing glucan according to item 2A, wherein the branched α- 1, 4- glucan is selected from the group consisting of a branched maltooligosaccharide, a starch, amylopectin, glycogen, dextrin, enzymatically synthesized branched glucan and highly branched cyclic glucan.
(Item 4A)
A hydroxyl group-modified product of the glucosamine-containing glucan according to any one of items 1A to 3A, wherein the modification on the hydroxyl group is a modification on some or all of alcoholic hydroxyl groups of the glucan, and the modification on the hydroxyl group is independently selected from the group consisting of hydroxyalkylation, alkylation, acetylation, carboxymethylation, sulfation and phosphorylation.
(Item 5A)
A reducing end-modified product of the glucosamine-containing glucan according to any one of items 1A to 3A or a hydroxyl group-modified product thereof.
(Item 6A)
An amino group-modified product of the glucosamine-containing glucan according to any one of items 1A to 3A, a hydroxyl group-modified product thereof or a reducing end-modified product thereof, wherein the modification on the amino group is a modification on some or all of amino groups of the glucosamine residues, the modification on the amino group is attained by a reaction of the amino group and an amino group-modifying reagent, and the amino group-modifying reagent has at least one carboxyl group and at least one other functional group.
(Item 7A)
A non-reducing end-modified product of the glucosamine-containing glucan according to any one of items 1A to 3A, a hydroxyl group-modified product thereof, a reducing end-modified product thereof or an amino group-modified product thereof, wherein a targeting molecule is bound to at least one of non-reducing ends to which the glucosamine residue of the glucan is not bound, wherein the targeting molecule is selected from the group consisting of mannose, galactose, glucuronic acid, N-acetylglucosamine, xylose, fucose, galactosamine, an antibody, an antibody fragment, a receptor, a receptor fragment and a receptor ligand.
(Item 8A)
A method for producing a glucosamine- containing glucan, characterized by allowing an α- glucan phosphorylase to act on an aqueous solution comprising a glucan and glucosamine- 1- phosphate, wherein the glucan is a branched α- 1, 4- glucan or a linear α- 1, 4- glucan, and the degree of polymerization of the glucan is about 10 or more and about 1 x $10^5$ or less, and preferably about 15 or more and about 4 x $10^5$ or less.
(Item 9A)
The method according to item 8A, wherein the α- glucan phosphorylase has 95% or more sequence identity with the amino acid sequence of α- glucan phosphorylase derived from *Aquifex aeolicus* VF5, and has activity of transferring glucosamine to a non- reducing end of a branched glucan to form an α- 1, 4- bond.
(Item 10A)
A medicament comprising the glucosamine-containing glucan according to any one of items 1A to 3A, a hydroxyl group-modified product thereof, a reducing end-modified product thereof, or an amino group-modifiedproduct thereof, and a medically effective ingredient.
(Item 11A)

The medicament according to item 10A, wherein the medically effective ingredient is selected from the group consisting of a low-molecular weight organic compound, a protein, a peptide, an antibody, an antibody fragment, a receptor, a receptor fragment, a DNA, an RNA, an miRNA and an RNA aptamer.

(Item 12A)

A composition for clinical diagnosis comprising the glucosamine-containing glucan according to any one of items 1A to 3A, a hydroxyl group-modified product thereof, a reducing end-modified product thereof, or an amino group-modified product thereof.

(Item 13A)

A nanoparticulate carrier for a DDS comprising the glucosamine-containing glucan according to any one of items 1A to 3A, a hydroxyl group-modified product thereof, a reducing end-modified product thereof, or an amino group-modified product thereof.

(Item 14A)

The carrier according to item 13A, wherein the nanoparticulate carrier for a DDS is selected from the group consisting of a liposome, a virus particle, a macromolecule micelle and a nanogel composed of macromolecule bearing hydrophobic groups.

(Item 15A)

A complex formed with a nucleic acid molecule comprising a gene which can be expressed in a cell and the glucosamine-containing branched glucan according to any one of items 1A to 3A.

(Item 16A)

The complex according to item 15A, wherein the nucleic acid molecule is selected from the group consisting of a DNA, an RNA, a siRNA, an miRNA and an RNA aptamer.

(Item 17A)

A complex formed with the complex carrier according to item 15A or 16A, and a cationic polymer or a cationic lipid.

(Item 18A)

The complex according to item 17A, wherein the cationic polymer comprises at least one cationic polymer selected from the group consisting of polyethyleneimine, polylysine, polyarginine, polyamidoamine dendrimer, poly (aminostyrene) , chitosan, a cationic glucan and DEAE- dextran.

(Item 19A)

A method for delivering a nucleic acid molecule into a cell, comprising contacting the complex according to any one of item 15A to 18A with the cell.

EFFECTS OF THE INVENTION

In the amino sugar-containing glucan, a hydroxyl group-modified product thereof, a reducing end-modified product thereof, an amino group-modified product thereof and a conjugate thereof of the present invention, an amino sugar residue is bound only to a non-reducing end of the glucan. Since the present amino sugar-containing glucan, a hydroxyl group-modified product thereof, a reducing end-modified product thereof, an amino group-modified product thereof and a conjugate thereof have amino sugar on a terminus, a glucan terminus comes to be positively charged in an aqueous solution, and a physicochemical nature of the glucan is changed. The present amino sugar-containing glucan, a hydroxyl group-modified product thereof, a reducing end-modified product thereof, an amino group-modified product thereof and a conjugate thereof are expected to be widely utilized in foods, cosmetics, medicaments and the like. These glucans have the characteristics that, when used as a carrier for a medically effective ingredient, due to the bond by an electrostatic interaction, these glucans do not change in quality largely the chemical structure of the medically effective ingredient, and can bind a plurality of amino sugars to the non-reducing end per carrier molecule, therefore, these glucans can retain the medically effective ingredient stably.

Since the amino sugar-containing glucan, a hydroxyl group-modified product thereof, a reducing end-modified product thereof, and an amino group-modified product thereof of the present invention can increase a half-life in blood than that of an unmodified glucan, and ultimately completely degraded in a living body and excreted from kidney, they have extremely high safety. For this reason, they are useful as a modifying material for a medically effective ingredient, a clinical diagnostic agent, a contrast agent and a nanoparticulate carrier for DDS. In the amino sugar-containing glucan, a hydroxyl group-modified product thereof, a reducing end-modified product thereof, and an amino group-modified product thereof of the present invention, since their structure can be controlled by an enzymatic reaction, they are also excellent in quality stability.

In the amino sugar-containing glucan and a modified product thereof of the present invention, since an amino sugar residue is bound only to a non-reducing end of the glucan, a site which interacts with other molecules and a site which suppresses degradation can be structurally separated. Further, the amino sugar-containing glucan and a modified product thereof of the present invention have been appropriately suppressed the degradation compared to naturally-occurring glucan, the interaction with other molecules is not too strong, and can appropriately release other molecule

as the glucan moiety is degraded, therefore, they can give an appropriate degradability to other molecule by association with the other molecule. In addition, the amino sugar-containing glucan and a modified product thereof and a conjugate thereof of the present invention are degraded with an enzyme in a body, and therefore it does not cause the problem of residual property due to staying in a body for an excessively long term.

When the amino sugar-containing glucan and a modified product thereof of the present invention are associated with a negatively-charged substance such as a nucleic acid molecule, it is possible to control the molecular weight of the associate. Since in view of safety, stability of the result, and reproducibility, it is preferable that the molecular weight of an administered product in DDS can be controlled, the amino sugar-containing glucan and a modified product thereof of the present invention can effectively be utilized as an excellent carrier for DDS.

## BRIEF DESCRIPTION OF THE DRAWINGS

[Fig. 1] Fig. 1 is a schematic view of a reaction of transferring a glucosamine residue to a non-reducing end of a branched glucan. The structure of the part shown with an asterisk is shown in the square frame.

[Fig. 2] Fig. 2 is results of analysis of an enzymatically treated product of a branched glucan obtained in Production Example 2, and an enzymatically treated product of the product obtained by allowing *Aquifex aeolicus* VF5- derived α- glucan phosphorylase in Production Example 1 to act on glucosamine- 1- phosphate and the branched glucan of Production Example 2 (the product obtained in Example 1) , with a HPAEC- PAD apparatus manufactured by DIONEX. The horizontal axis represents the elusion time (minutes) . A shows the result obtained by analyzing the degradation product after degrading the branchedportions of the branched glucan with the sufficient amount (that is, excess amount) of isoamylase to cleave all the branches. Regarding numbers above the peaks in Fig. 2A, 6 indicates maltohexaose, and 7 indicates maltoheptaose. The peaks shown with black solid circles thereabove indicate, from left to right, maltooligosaccharides having the degree of polymerization of glucose of from 8 to 14. Fig. 2B shows the result obtained by analyzing the degradation productobtained after degrading the branched glucan with an excess amount of isoamylase, and then further degrading it with an excess amount of microorganism-derived α- glucosidase. Since α- glucosidase is an enzyme which degrades glucan in glucose units starting from the non- reducing end, the branched glucan was completely degraded and the peak of glucose (shown by "Glucose" in Fig. 2B) was detected. Fig. 2C shows the result obtained by analyzing the degradation product obtained after degrading the glucosamine- containing branched glucan obtained in the present Example 1 with an excess amount of isoamylase. While the peaks shown with black solid circles in Fig. 2C correspond to the peaks detected at the same elusion time as the peaks shown with black solid circles at the corresponding positions in Fig. 2A, the peaks shown with "x" having different intervals therefrom were detected. Fig. 2D shows the result obtained by analyzing the degradation productobtained after degrading the glucosamine- containing branched glucan obtained in the present Example 1 with isoamylase and further degrading with an excess amount of microorganism- derived α-glucosidase. The group of peaks shown with black solid circles disappeared due to the degradation, and as a result, the peak of glucose (shown by "Glucose" in Fig. 2D) appeared, however, the peaks shown with "x" did not disappeare. That is, the partially degraded product indicated by the peaks shown with "x" exhibited resistance to degradation with α- glucosidase. Fig. 2E shows the result obtained by analyzing the degradation product obtained after degrading the glucosamine- containing branched glucan obtained in the present Example 1 with an excess amount of isoamylase, and further degrading with an excess amount of microorganism- derived α- glucosidase and an excess amount of α- amylase simultaneously. As a result, in addition to the peak of glucose, the peaks shown with asterisks were detected. The peaks shown with asterisks indicate glucosamine- containing oligosaccharide (trisaccharide) , which is the smallest unit which cannot be degraded with α- amylase and α- glucosidase, and therefore, it can be understood that a glucan which has a glucosamine residue bound to the non- reducing end was obtained.

[Fig. 3] Fig. 3 is a figure showing formation of a complex of the glucosamine-containing glucan of Example 1 and a DNA. Relative to 0.5 μg of a lambda DNA-Hind III fragment, ion-exchanged water for Sample 1, 0.1 mg of branched glucan for Sample 2, 0.5 mg of branched glucan for Sample 3, 0.1 mg of glucosamine-containing branched glucan for Sample 4, 0.5 mg of glucosamine-containing branched glucan for Sample 5, the same molar concentration of glucosamine as that of glucosamine unit contained in Sample 4 for Sample 6, the same molar concentration of glucosamine as that of glucosamine unit contained in Sample 5 for Sample 7 were added respectively, and they were allowed to stand at room temperature in 10 μl for 5 minutes, then 1% agarose gel electrophoresis were performed. While bands (a) were detected for Samples 1 to 3, DNA did not migrate through agarose gel when glucosamine-containing branched glucan were added (b). Therefore, it could be understood that the cationized glucosamine-containing branched glucan can form a complex with a DNA. In addition, in Samples 6 and 7 to which glucosamine were added, the mobility were not changed as compared with the case where the glucosamine was not added, and it could be understood that glucosamine has no effect to increase the molecular weight of a DNA.

[Fig. 4] Fig. 4 is a figure showing the binding force between oligo (dT)- cellulose and the glucosamine- containing branched glucans of Example 3 (BN5 and BN6) or the glucosamine- containing branched glucan of Example 4 (PN6) . Oligo (dT)- cellulose was suspended in NaCl solutions of each concentration (0.1 M, 0.2 M, 0.3 M, 0.5 M, 1 M, or 2 M) , and the glucosamine- containing branched glucan obtained in Example 3 or Example 4 was added and allowed to stand at room temperature for 30 minutes, then centrifuged, and measured the amount of glucosamine-containing branched glucans which were not bound with oligo (dT)- cellulose contained in the supernatant fraction, thereby calculated the percentage (%) of the amount of bound cationized glucan (total sugar amount) relative to the amount of added glucosamine- containing glucan (total sugar amount) . The obtained results are shown as a graph. It can be understood that PN6 separates from a nucleic acid at 1 M or more of NaCl. It can be understood that BN6 separates from a nucleic acid at 0.5 M or more of NaCl. It can be understood that BN5 separates from a nucleic acid at 0.2 or more. There was a tendency that the more number of glucosamine residues contained in one molecule of glucosamine- containing branched glucan, the higher the NaCl concentration at which these residues separates. Therefore, the binding force with a nucleic acid could be controlled by controlling the frequency at which glucosamine was bound to a branched glucan. In the case where polylysine was used, polylysine bound with a nucleic acid strongly, and did not separate from the nucleic acid even in 2 M of NaCl.

[Fig. 5] Fig. 5 is a figure showing the transfection efficiency of macrophage cell line in the case where the glucosamine-containing branched glucan of Example 4 (PN6) was used. The glucosamine-containing branched glucan was incubated with a plasmid containing LUC gene encoding firefly-derived luciferase for 24 hours, and thereafter, luciferase activity was measured. As shown in Fig. 5, it can be understood that PN6 shows a transfection function, andespecially, the highest transfection efficiency was shown when N/P ratio was 200. While polyethyleneimine showed high transfection efficiencies at N/P ratios of 10 and 50, the efficiency remarkably lowered when N/P ratio was 200. Polylysine showed the highest transfection efficiency when N/P ratio was 2, and the efficiency remarkably lowered when N/P ratio was higher than 10.

[Fig. 6] Fig. 6 is a figure evaluating the cytotoxicity in transfection of macrophage cell line when the glucosamine-containing branched glucan of Example 4 (PN6) was used. The protein concentration in the supernatant of the cell lysate obtained in Example 11 was measured and used as a measure of toxicity. As shown in Fig. 6, it is shown that PN6 has low toxicity to a cell. When polyethyleneimine and polylysine were used, remarkable toxicity was observed at N/P ratios of 50 and 200.

## MODE FOR CARRYING THE INVENTION

The present invention will be explained in detail below.

Throughout the present specification, it should be understood that expression in a singular form includes a concept of a plural form thereof, unless otherwise indicated. In addition, it should be understood that a term used in the present specification is used in a sense which is usually used in the art, unless otherwise indicated.

(General Techniques)

The molecular biological procedures, biochemical procedures, and microbiological procedures used in the present specification are well known and routine in the art, and described in, for example, Sambrook, J. et al., (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al., (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al., (1999). PCR Applications: Protocols for Functional Genomics, Academic Press, Bessatsu Jikken-igaku "Idenshidounyu & Hatsugen kaiseki jikkenhou" (a separate volume of Experimental Medicine "Experimental methods for gene introduction and expression analysis"), Yodosha Co., Ltd., 1997, and the like, the related portions thereof (which can be the whole) are incorporated herein by reference.

DNA synthetic techniques and nucleic acid chemistries for producing an artificially-synthesized gene are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approac, IRL Press; Adams, R.L. et al., (1992). The Biochemistry of the Nucleic Acids, Chapman & Hall; Shabarova, Z. et al., (1994). Advanced Organic Chemistry of Nucleic Acids, Weinheim; Blackburn, G.M. et al., (1996).

Nucleic Acids in Chemistry and Biology, Oxford University Press; Hermanson, G.T. (I996). Bioconjugate Techniques, Academic Press, and the like, the related portions thereof are incorporated herein by reference.

When a gene is referred to in the present specification, a "vector" or a "recombinant vector" refers to a vector which can transfer an objective polynucleotide sequence into an objective cell. Examples of such vectors include a vector which can autonomously replicate in a host cell, such as a prokaryotic cell, a yeast, an animal cell, a plant cell, an insect cell, an animal individual and a plant individual, or can be incorporated into a chromosome, and has a promoter at a position suitable for transcribing the polynucleotide of the present invention. Among the vectors, those vectors which are suitable for cloning are referred to as "cloning vector". Such a cloning vector usually comprises a multiple-cloning site containing a plurality of restriction enzyme sites. Such a restriction enzyme site and a multiple-cloning site are well known in the art, and those skilled in the art can select as appropriate and use them in accordance with the purpose. Such techniques are described in the documents described in the present specification (for example, Sambrook, et al., supra). Preferred vectors include, but are not limited to, a plasmid, a phage, a cosmid, an episome, a viral particle or a virus, and an integratable DNA fragment (that is, a fragment which can be integrated into a host genome by homologous recombination).

One type of vectors is a "plasmid", which refers to a circular duplex DNA loop to which an additional DNA segment can be linked. Another type of vector is a viral vector, wherein an additional DNA segment can be linked into a viral genome. Specific vectors (for example, bacterial vector having a bacterial origin of replication and an episome mammalian vector) can autonomously replicate in a host cell to which these vectors are introduced. Other vectors (for example, non-episome mammalian vector) are integrated to a genome of a host cell upon introduction to the host cell, and thereby, replicated together with the host genome. In addition, specific vectors can direct expression of a gene to which these vectors are operably linked. Such vectors are referred to as "expression vectors" in the present specification.

Therefore, in the present specification, an "expression vector" or an "expression plasmid" refers to a nucleic acid sequence wherein various regulation elements, in addition to a structural gene and a promoter regulating its expression, are connected in a cell of a host in an operable condition. An expression vector is, preferably, a vehicle which is operably linked to an objective structural gene such that the objective structural gene is transcripted and translated, and if necessary, contains factors necessary for replication in the host cell and selection of a recombinant. Regulation elements can include, preferably, a terminator, a selectable marker such as a drug resistant gene, and an enhancer. It is well- known to those skilled in the art that the type of an expression vector of an organism (e.g. mammal) , and the kind of regulation element used can vary depending on a host cell. When secretion production of an expressed product ($\alpha$- glucan phosphorylase or a medically effective ingredient) is intended, a polynucleotide encoding a secretion signal peptide is linked upstream of a DNA coding for the objective protein in the correct reading frame.

Preferred expression vectors include pTRC99A (manufactured by Pharmacia) that is also expressible in *Escherichia coli,* and the like. In order to operably link an $\alpha$-glucan phosphorylase gene to factors necessary for transcription and translation in the aforementioned expression vector, an objective $\alpha$-glucan phosphorylase gene should be processed in some cases. Examples include the case where the distance between a promoter and a coding region is too long, and reduction in a transcription efficiency is predicted, the case where the distance between a ribosome binding site and a translation initiation codon is not suitable, and the like. The process means include digestion with a restriction enzyme, digestion with an exonuclease such as Bal31 and ExoIII, or introduction of site-directed mutagenesis using a single-stranded DNA such as M13 or PCR.

As "recombinant vectors" for a prokaryotic cell which can be used in the present application, pcDNA3 (+) , pBluescript- SK (+/- ) , pGEM- T, pEF- BOS, pEGFP, pHAT, pUC18, pFT- DEST™ 42GATEWAY, pENTR™/D- TOPO (Invitrogen) , and the like are exemplified. A prokaryotic cell can be used in amplification, modification and the like of a gene.

"Recombinant vectors" for an eukaryote cell which can be used in the present application include, but are not limited to, pECFP (Clontech) , pAcGFP (Clontech) , pEYFP (Clontech) , pDsRED (Clontech) , pTRE (Clontech) , pCMV (Clontech) , pcDNA (Invitrogen) , pTarget (Promega) , and the like.

In the present specification, a "mammalian expression vector" refers to a nucleic acid sequence wherein various regulation elements such as a promoter regulating expression of the gene of the present invention are operably linked in a host cell. The term "regulation sequence" used in the specification of the present application refers to a DNA sequence having a functional promoter and any related transcription elements (for example, an enhancer, a CCAAT box, a TATAbox, an SPI site, and the like). The term "operably linked" used in the specification of the present application refers that a polynucleotide related to a gene and various regulation elements, such as a promoter and an enhancer, which regulate expression of the gene are connected in a host cell in an operable condition such that the gene can be expressed. A mammalian expression vector can preferably include a mammalian gene, a promoter, a terminator, a drug resistant gene and an enhancer. It is well-known to those skilled in the art that the type of expression vector and the kind of regulation element used can vary depending on a host cell.

Mammalian expression vectors as described above can be made using gene recombination techniques well-known to those skilled in the art. For construction of a mammalian expression vector, for example, a pECFP-type vector, a pcDNA-type vector or the like are preferably used, but not limited to them.

In the present specification, a "terminator" is a sequence which is situated downstream of a protein coding region of a gene, and is involved in termination of transcription upon transcription of a DNA into an mRNA, and in the addition of a poly A sequence. It is known that a terminator contributes to the stability of an mRNA and influences the expression level of a gene.

In the present specification, a "promoter" refers to a region on a DNA which determines a transcription initiation site of a gene, and directly regulates the transcription frequency, and is a base sequence to which a RNA polymerase binds, thereby, initiating transcription. Aputative promoter region varies with every structural gene, and is usually upstream of a structural gene without limitation, and may be downstream of a structural gene.

While a promoter may be inducible, constitutive, site-directed, or stage-specific, a constitutive promoter or an inducible promoter is preferable. Any promoter is possible as long as which can be expressed in a host cell such as a mammalian cell, *Escherichia coli,* or a yeast.

In the present specification, expression of a promoter is "constitutive" means the nature by which the promoter is expressed at an almost fixed amount in all the tissues in an organism at whatever stage of growth/proliferation of the organism. Specifically, in the definition of the present invention, when analyzed with Northern blot, if almost the same degree of expression amount is observed in either of the same or corresponding sites, for example, at any time point (for example, at two or more points (for example, on day 5 and day 15)), the expression is said to be constitutive. It is believed that a constitutive promoter plays a role in maintenance of homeostasis of an organism in a normal growing environment. "Responsive" expression of the promoter of the present invention means the nature by which the expression amount is changed when at least one factor is given to an organism. Especially, the nature by which expression amount is increased is referred to be "inducible" by a factor, and the nature by which expression amount is decreased is referred to be "decreasing" by a factor. Since "decreasing" expression has a premise that expression is observed under a normal condition, it is an idea overlapping with "constitutive" expression. These natures can be determined by extracting an RNA from any portion of an organism and analyzing expression amount by Northern blot analysis, or quantifying the expressed protein by western blot. A mammalian cell or a mammal (including a specific tissue or the like) transformed with a vector integrated a promoter inducible by a factor together with a nucleic acid encoding the site-directed recombination inducing factor of the present invention can perform a site-directed recombination of a site-directed recombination sequence under a certain condition by using a stimulation factor having an inducible activity to the promoter.

The polynucleotide of the present invention can be linked intact or with modification into an appropriate expression vector using a method well-known to those skilled in the art, and introduced to a host cell by a known gene recombination technique. The introduced gene exists integrated to a DNA in the host cell. It is noted that a DNA in a host cell includes not only a DNA included in a chromosome but also a DNA included in various organelle (for example, a mitochondrion and the like) contained in a host cell.

When *Escherichia coli* is used as a host cell, promoters derived from *Escherichia coli,* phage and the like, such as trp promoter (Ptrp), lac promoter (Plac), PL promoter, PR promoter, and PSE promoter, SPO1 promoter, SPO2 promoter, penP promoter, and the like can be included. In addition, an artificially designed and modulated promoters or the like such as a promoter consisting of two serially aligned Ptrps (Ptrp x2), tac promoter, lacT7 promoter, let I promoter can also be used.

In the present specification, an "origin of replication" refers to a specific region on a chromosome from which DNA replication starts. An origin of replication can either be provided by constructing the vector so as to include an endogenous origin, or by a chromosomal replication mechanism of a host cell. The latter may be sufficient when the vector is integrated to a host cell chromosome. Alternatively, those skilled in the art can transform a mammalian cell by co- transforming with a selectable marker and the DNA of the present invention, rather than by using a vector including a viral origin of replication. Examples of suitable selectable markers are dihydrofolate reductase (DHFR) or thymidine kinase (see U. S . Patent No. 4, 399, 216) .

In the present specification, "operably linked" refers to that expression (operation) of a desired sequence is placed under the control of a transcription and translation regulating sequence (e.g. promoter, enhancer and the like) or a translation regulating sequence. In order that a promoter is operably linked to a gene, usually, a promoter is disposed immediately upstream of the gene, but it is not necessary that the promoter is disposed adjacent to the gene.

In the present specification, the technique of introducing a nucleic acid molecule into a cell may be any technique. Examples of such techniques include, for example, transformation, transduction, and transfection. Such techniques of introducing a nucleic acid molecule are well-known in the art, and are conventional, and are described, for example, in Ausubel F.A. et al., ed. (1988), Current Protocols in Molecular Biology, Wiley, New York, NY; Sambrook J et al., (1987) Molecular Cloning: A Laboratory Manual, 2nd Ed. and 3rd Ed., Cold Spring Harbor Laboratory Press, Cold Spring Harbor, NY; Bessatsu Jikken-igaku "Idenshidounyu & Hatsugen kaiseki jikkenhou" (a separate volume of Experimental Medicine "Experimental methods for gene introduction and expression analysis"), Yodosha Co., Ltd., 1997 and the like. The introduction of a gene can be confirmed using the methods described in the present specification such as Northern blot analysis and Western blot analysis, or other well-known and conventional techniques.

Further, as a method for introducing a vector, any method as described above for introducing a DNA to a cell

can be used, the method include, for example, transfection, transduction, transformation and the like (for example, calcium phosphate method, liposome method, DEAE- dextran method, electroporation method, method using a particle gun (a gene gun) and the like) .

In the present specification, a "transformant" refers to a whole or a part of a living body such as a cell produced by transformation. Transformants are exemplified by a prokaryotic cell, a yeast, an animal cell, a plant cell, an insect cell, and the like. A transformant can also be referred to as a transformated cell, a transformated tissue, or a transformated host, depending on the subject. The cell used in the present invention can be a transformant.

When a prokaryotic cell is used in gene manipulation or the like in the present invention, prokaryotic cells are exemplified by the prokaryotic cells belonging to a genus *Escherichia, Serratia, Bacillus, Brevibacterium, Corynebacterium, Microbacterium, Pseudomonas,* or the like, for example, *Escherichia coli* XL1-Blue, *Escherichia coli* XL2-Blue, and *Escherichia coli* DH1.

As used in the present specification, as a method for introducing a recombinant vector, any methods which introduce a DNA can be used, the methods include, for example, calcium chloride method, electroporation method [Methods. Enzymol., 194, 182 (1990)], lipofection method, spheroplast method [Proc. Natl. Acad. Sci. USA, 84, 1929 (1978)], lithium acetate method, and the like.

In the present specification, "detection" or "quantification" of gene expression (for example, mRNA expression, and polypeptide expression) can be achieved using a suitable method including, for example, measurement of mRNA and immunological measuring method. Molecular biological measuring methods are exemplified by, for example, Northern blot method, dot blot method, PCR method and the like. Immunological measuring methods are exemplified, for example, ELISA method using a microtiter plate, RIA method, immunofluorescence method, western blot method, immunohistostaining, and the like, as the method. Further, quantifying methods are exemplified by ELISA method, RIA method or the like. It can be done by a gene analyzing method using an array (for example, a DNA array, and a protein array) . A DNA array is widely reviewed in (Shujunsha Ed. , Saibo Kogaku Bessatsu, "DNA Microarray to Saishin PCR Hou"). A protein array is detailed in Nat Genet. 2002 Dec; 32 Suppl: 526-32. Methods for analyzing gene expression include, but are not limited to, RT-PCR, RACE method, SSCP method, immunoprecipitation method, two-hybrid system, *in vitro* translation and the like, in addition to the above. Such additional analysis methods are described in, for example, Genomu kaiseki jikkenhou, Nakamura Yusuke lab manual (Understand the Basics of Genomics Experiments - Yusuke Nakamura Laboratory Manual), Editor Yusuke Namamura, Yodosha Co., Ltd. (2002) and the like, the whole description of which is incorporated herein by reference.

(1. Materials)

(1.1) Glucans and modified products of glucan

"Glucan", when used in the present specification, is a polysaccharide having D- glucose as a constituent unit. In the present invention, it is preferable to use, as the glucan, an α- D- glucan. The bonds which link glucose residues in an α- D- glucan predominantly consist of an α- 1, 4- glucosidic bond, and can contain an α- 1, 6- glucosidic bond. An α- D- glucan containing a α- 1, 6- glucosidic bond has a branched structure. A glucan having at least one α- 1, 6- glucosidic bond in the molecule is referred to as a branched glucan, and a glucan having no α- 1, 6- glucosidic bond in the molecule is referred to as a linear glucan. In the present specification, unless otherwise indicated, it is preferable that the weight average molecular weight of the "glucan" is about $1 \times 10^3$ or more. Preferable glucans used in the present invention are a linear glucan and a branched glucan, more preferably a linear α- 1, 4- glucan and an α- 1, 4- glucan which is branched with an α- 1, 6- bond (also referred to as a branched α- 1, 4 glucan) . It is preferable that the glucan used in the present invention does not contain an α- 1, 3- bond.

The linear α- D- 1, 4- glucan refers to a polysaccharide in which two or more saccharide units of D- glucose units are bound only with an α- 1, 4- glucosidic bond (s) . In the present specification, unless otherwise indicated, the linear α- D- 1, 4- glucan is referred to as a linear glucan or a linear α- 1, 4- glucan. The linear glucan has one non-reducing end. Examples of the linear glucan suitably utilized in the present invention include amylose.

In the present specification, the term "amylose" refers to a linear molecule constructed of glucose units connected via α- 1, 4- bonds. An amylose is contained in natural starch. Amylose may be a natural amylose extracted from a natural starch, or may be an amylose synthesized by an enzymatic reaction (also referred to as "enzymatically synthesized amylose" in the present specification) . Natural amylose may contain a branched part in some cases, but enzymatically synthesized amylose does not contain a branch. Further, natural amyloses have a large polydispersity and vary in the molecular weight, but an enzymatically synthesized amylose (particularly, an enzymatically synthesized amylose synthesized by the SP- GP method described in International Publication WO 02/097107 pamphlet) has a small polydispersity and has an extremely uniform molecular weight. For this reason, in the present invention, it is preferable to use an enzymatically synthesized amylose. The degree of polymerization of the amylose used in the present invention is preferably about 10 or more, more preferably about 50 or more, still more preferably about 100 or more, and most preferably

about 180 or more. The degree of polymerization of the amylose used in the present invention is preferably about 1 x $10^5$ or less, more preferably about 1 x $10^4$ or less, still more preferably about 1 x $10^3$ or less and most preferably about 500 or less.

In the present specification, the term "branched α- D- glucan" refers to a glucan in which a linear glucan, in which D- glucose units are connected with an α- 1, 4- glucosidic bond (s) , is branched with a bond other than an α- 1, 4- glucosidic bond. In the present specification, unless otherwise indicated, the branched α- D- glucan is referred to as a branched glucan. A branching bond is either an α- 1, 6- glucosidic bond, an α- 1, 3- glucosidic bond, or an α- 1, 2- glucosidic bond, and most preferably is an α- 1, 6- glucosidic bond. It is preferable that a branched α- D- glucan used in the present invention does not contain an α- 1, 3- glucosidic bond and an α- 1, 2- glucosidic bond. The branched glucan usually has the same number of non- reducing ends as the number of branching bonds. When the branched glucan is treated with an enzyme which selectively breaks only an α- 1, 6- glucosidic bond (for example, isoamylase, pullulanase, or the like) , the branched glucan can be degraded into a mixture of linear α- 1, 4- glucans. These are referred to as a unit chain of the branched glucan, and the degree of polymerization thereof is referred to as a unit chain length.

Examples of the branched glucan suitably utilized in the present invention include starches, amylopectin, glycogen, dextrin, enzymatically synthesized branched glucan and highly branched cyclic glucan.

In the present specification, the term "starch" is a mixture of amylose and amylopectin. As a starch, any starch can be used as long as it is commonly commercially available. The ratio of the amylose and amylopectin contained in a starch is different depending on the kind of plant producing the starch. Almost all starches possessed by glutinous rice, glutinous corn and the like are an amylopectin. On the other hand, a starch consisting only of amyloses, containing no amylopectin, can not be obtained from a common plant. Starch is classified into natural starch, a degraded starch and modified starch.

Natural starch is classified into tuber starch and cereal starch depending on the raw material. Examples of tuber starches include potato starch, tapioca starch, sweet potato starch, kudzu starch, bracken starch and the like. Examples of cereal starches include corn starch, wheat starch, rice starch and the like. Examples of natural starches are high amylose starches (for example, high amylose corn starch) or waxy starches. The starch can be a soluble starch. A soluble starch refers to a water-soluble starch obtained by subjecting a variety of treatment on natural starch. The starch may be selected from the group consisting of soluble starch, waxy starch and high amylose starch. The starch may be a modified starch.

The degree of polymerization of the starch used in the present invention is preferably about 1 x $10^3$ or more, more preferably about 5 x $10^3$ or more, still more preferably about 1 x $10^4$ or more, and most preferably about 2 x $10^4$ or more. The degree of polymerization of the starch used in the present invention is preferably about 1 x $10^7$ or less, more preferably about 3 x $10^6$ or less, still more preferably about 1 x $10^6$ or less and most preferably about 3 x $10^5$ or less.

An amylopectin is a branched molecule in which a glucose unit (s) is bound via an α-1,6 bond to glucose units which are bound via an α-1,4 bond(s). An amylopectin is contained in natural starch. As an amylopectin, for example, waxy corn starch, which consists of 100% amylopectin, can be used. The degree of polymerization of the amylopectin used in the present invention is preferably about 1 x $10^3$ or more, more preferably about 5 x $10^3$ or more, still more preferably about 1 x $10^4$ or more, and most preferably about 2 x $10^4$ or more. The degree of polymerization of the amylopectin used in the present invention is preferably about 1 x $10^7$ or less, more preferably about 3 x $10^6$ or less, still more preferably about 1 x $10^6$ or less and most preferably about 1 x $10^5$ or less.

A glycogen is one kind of glucan constructed of glucose, and is a glucan having a high frequency of branching. A glycogen is widely distributed as a storage polysaccharide for animals in almost all cells in the granule state. In a plant, glycogen is present, for example, in the seed of sweet corn species of corn. In a glycogen, typically, sugar chains consisting of glucoses bound via an α- 1, 4- bond (s) which have an average degree of polymerization of 12 to 18 are bound by an α- 1, 6- bond (s) at a ratio of around one chain every about 3 units of glucose, to a sugar chain consisting of glucoses bound via an α- 1, 4- bond (s) . In addition, similarly, a sugar chain consisting of glucoses bound by an α- 1, 4- bond (s) is bound by an α- 1, 6- bond to a branch chain bound by an α- 1, 6- bond (s) . For this reason, glycogen forms a network structure. It is also possible to enzymatically synthesize a glycogen. The degree of polymerization of the glycogen used in the present invention is preferably about 500 or more, more preferably about 1 x $10^3$ or more, still more preferably about 2 x $10^3$ or more, and most preferably about 3 x $10^3$ or more. The degree of polymerization of the glycogen used in the present invention is preferably about 1 x $10^7$ or less, more preferably about 3 x $10^6$ or less, still more preferably about 1 x $10^6$ or less and most preferably about 3 x $10^5$ or less.

Dextrin is one kind of glucan constructed of glucose, and is a glucan having a medium complexity between those of starch and those of maltose. Dextrin is obtained by partially degrading starch by an acid, an alkyl or an enzyme. The degree of polymerization of the dextrin used in the present invention is preferably about 10 or more, more preferably about 20 or more, still more preferably about 30 or more, and most preferably about 50 or more. The degree of polymerization of the dextrin used in the present invention is preferably about 1 x $10^4$ or less, more preferably about 9 x $10^3$ or less, still more preferably about 7 x $10^3$ or less and most preferably about 5 x $10^3$ or less.

The enzymatically synthesized branched glucan refers to a branched glucan synthesized using an enzyme. By adding a branching enzyme to the reaction solution upon synthesis of amylose by the SP-GP method, the product can be branched. The extent of branching can be regulated by the added amount of the branching enzyme. Since the enzymatically synthesized branched glucan has a uniform structure as compared with a natural branched glucan, it is very advantageous when used as a pharmaceutical material. For example, the degree of polymerization of the enzymatically synthesized branched glucan used in the present invention is preferably about 10 or more, more preferably about 20 or more, still more preferably about 30 or more, and most preferably about 500 or more. The degree of polymerization of the enzymatically synthesized branched glucan used in the present invention is preferably about 2 x $10^5$ or less, more preferably about 1 x $10^5$ or less, still more preferably about 5 x $10^4$ or less and most preferably about 3 x $10^4$ or less.

In the present specification, the term "highly branched cyclic glucan" refers to a glucan having an internally branched cyclic structural moiety and an externally branched structural moiety and having a degree of polymerization of 50 or more. The highly branched cyclic glucan may have at least two non-reducing ends as a whole molecule. The degree of polymerization of the highly branched cyclic glucan as a whole molecule that can be used in the present invention is preferably about 50 or more, more preferably about 60 or more, and still more preferably about 100 or more. The degree of polymerization of the highly branched cyclic glucan as a whole molecule that can be used in the present invention is preferably about 1 x $10^4$ or less, more preferably about 7 x $10^3$ or less, and still more preferably about 5 x $10^3$ or less.

The degree of polymerization of the internally branched cyclic structural moiety present in the highly branched cyclic glucan is preferably about 10 or more, more preferably about 15 or more, and further preferably about 20 or more. The degree of polymerization of the internally branched cyclic structural moiety present in the highly branched cyclic glucan is preferably about 500 or less, more preferably about 300 or less, and further preferably about 100 or less.

The degree of polymerization of the externally branched structural moiety present in the highly branched cyclic glucan is preferably about 40 or more, more preferably about 100 or more, further preferably about 300 or more, and further more preferably about 500 or more. The degree of polymerization of the externally branched structural moiety present in the highly branched cyclic glucan is preferably about 3 x $10^3$ or less, more preferably about 1 x $10^3$ or less, further preferably about 500 or less, and further more preferably about 300 or less.

The number of α- 1, 6- glucosidic bonds in the internally branched cyclic structural moiety present in the highly branched cyclic glucan may be at least one, and for example, can be one or more, 5 or more, 10 or more or the like; the number of α- 1, 6- glucosidic bonds in the internally branched cyclic structural moiety can be, for example, about 200 or less, about 50 or less, about 30 or less, about 15 or less, about 10 or less or the like.

The number of branches (that is, the number of α- 1, 6- glucosidic bonds) in the externally branched structural moiety present in the highly branched cyclic glucan is preferably about 2 or more, more preferably about 3 or more, further preferably about 5 or more, and especially preferably about 10 or more. The number of branches (that is, the number of α- 1, 6- glucosidic bonds) in the externally branched structural moiety present in the highly branched cyclic glucan is preferably about 5 x $10^3$ or less, more preferably about 4 x $10^3$ or less, and further preferably about 3 x $10^3$ or less.

As the highly branched cyclic glucan, a highly branched cyclic glucan having one kind of a degree of polymerization may be used alone, or a mixture of highly branched cyclic glucans having a variety of degree of polymerization may be used. Preferably, the degrees of polymerization of the highly branched cyclic glucan is such that the ratio of the degrees of polymerization of the maximum degree of polymerization to the minimum degree of polymerization is about 100 or less, more preferably about 50 or less, and further more preferably about 10 or less.

The highly branched cyclic glucan is preferably a glucan having an internally branched cyclic structural moiety and an externally branched structural moiety and having a degree of polymerization in a range of 50 to 5 x $10^3$, wherein the internally branched cyclic structural moiety is a cyclic structural moiety formed with an α- 1, 4- glucosidic bond and an α- 1, 6- glucosidic bond, and the externally branched structural moiety is a non- cyclic structural moiety bound to the internally branched cyclic structural moiety. The degree of polymerization of each unit chain of this externally branched structural moiety is, on average, preferably about 10 or more and preferably about 20 or less. The highly branched cyclic glucan and a method for producing the same are described in detail in Japanese Laid- Open Publication No. 8- 134104 (Japanese Patent No. 3107358) , and this glucan can be produced according to the description of it. The highly branched cyclic glucan is commercially available, for example, as "Cluster Dextrin" from Ezaki Glico Co., Ltd. The degree of polymerization of the highly branched cyclic glucan used in the present invention is preferably about 50 or more, more preferably about 70 or more, further preferably about 100 or more, most preferably about 150 or more. The degree of polymerization of the highly branched cyclic glucan used in the present invention is preferably about 1 x $10^4$ or less, more preferably about 7 x $10^3$ or less, further preferably about 5 x $10^3$ or less, and most preferably about 4 x $10^3$ or less.

In a specific embodiment, the branched glucan can be particulate. It is known that particles having a diameter of about 4 nm or less are excreted from kidney, particles having a diameter of about 4 nm to about 200 nm are circulated in blood for a long time, particles having a diameter of about 200 nmto about 7 μm are captured by a reticuloendothelial system, and particles having a diameter of about 7 μm or more obstruct capillary blood vessels. A reticuloendothelial

system is distributed in liver and spleen. For this reason, by controlling the particle size of the branched glucan, the pharmacokinetics of the amino sugar-containing glucan and a modified product thereof and a conjugate thereof of the present invention *in vivo* can be controlled. When one intends to circulate the particles in blood for a long time, the particle size of a particulate branched glucan is, as the diameter, preferably about 4 nm or more, more preferably about 10 nm or more, preferably about 200 nm or less, and more preferably about 100 nm or less. The molecular weight of the particulate branched glucan having such a particle size is preferably about $5 \times 10^5$ or more, more preferably about $1 \times 10^6$ or more, preferably about $5 \times 10^7$ or less, and more preferably about $2 \times 10^7$ or less. For example, since it is known that particles having a diameter of 20 to 50 nm are accumulated in cancer cells, when it is intended that the particles are accumulated in cancer cells, the particle size of the particulate branched glucan is, as the diameter, preferably about 10 nm or more, more preferably about 15 nm or more, preferably about 100 nm or less, and more preferably about 50 nm or less. The molecular weight of the particulate branched glucan having such a particle size is preferably about $5 \times 10^5$ or more, more preferably about $1 \times 10^6$ or more, preferably about $2 \times 10^7$ or less, and more preferably about $5 \times 10^6$ or less.

The number of branches in the α- glucan (i.e. the number of α- 1, 6- glucosidic bonds) is preferably about 1 or more, more preferably about 10 or more, further preferably about 30 or more. The number of branches of the α- glucan (i.e. the number of α- 1, 6- glucosidic bonds) is preferably about $5 \times 10^3$ or less, more preferably about $3 \times 10^3$ or less, further preferably about $1 \times 10^3$ or less.

In the branched α- glucan used in the present invention, the ratio of the number of α- 1, 6- glucosidic bonds relative to the number of α- 1, 4- glucosidic bonds ("number of α- 1, 6- glucosidic bonds": "number of α- 1, 4- glucosidic bonds") is preferably 1: 1 to 1: $1 \times 10^3$, more preferably 1: 1.1 to 1: 500, further preferably 1: 1.2 to 1: 100, further more preferably 1 : 1.5 to 1: 50, and most preferably 1: 2 to 1: 20. The ratio may be, in some cases, 1: 10 to 1: $5 \times 10^3$, 1: 50 to 1: $1 \times 10^3$, or 1: 100 to 1: 500.

In the present invention, the lower limit of the branching frequency of the branched glucan is preferably 0.2% or more, preferably 1% or more, more preferably 2% or more, and most preferably 5% or more. While there is no specific upper limit of the branching frequency, it can be, for example, 99% or less, 90% or less, 85% or less, 65% or less, 50% or less, or the like. The branching frequency is calculated by {(number of α- 1, 6- bonds) / (sum of α- 1, 4- bonds and α- 1, 6- bonds in glucan) } x 100.

The α- 1, 6- glucosidic bonds may be randomly distributed in the α- glucan or may be homogeneously distributed in the α- glucan. Adistribution to such an extent that a linear chain part (s) of 5 or more saccharide units can be formed in the α- glucan is preferable.

In the present invention, a modified product of the glucan may be used in place of the glucan. Examples of the modified product of the glucan include a modified starch and an esterified product of the glucan explained above. Furthermore, the modified product of the glucan may be a hydroxyl group- modified product or a reducing end- modified product. In addition, as described later, after at least one amino monosaccharide residue is α- 1, 4- bound to each of at least one non- reducing end of the glucan, a glucan moiety may be modified.

The modified starch is a starch which was made to have a nature that it is easier to use by subjecting a natural starch to treatment such as hydrolysis, esterification or gelatinization. Wide variety of modified starches having a variety of combinations of a gelatinization initiation temperature, a viscosity of a starch paste, a degree of transparency of a starch paste, stability against retrogradation and the like are available. There are various types of modified starches. An example of such a starch is a starch obtained by immersing starch granules in an acid at a gelatinization temperature or lower of the starch, thereby cutting a starch molecule but not destroying starch granules.

Examples of the modified product of the glucan other than the modified starch include a modified product in which at least one of alcoholic hydroxyl groups of an unmodified glucan is modified (hereinafter, in the present specification, referred to as a "hydroxyl group-modifiedproduct of glucan"), a modified product in which some of non-reducing ends of the glucan is modified (hereinafter, in the present specification, referred to as a " non-reducing end-modified product of glucan") and a modified product in which the reducing end of a glucan is modified (hereinafter, in the present specification, referred to as a "reducing end-modified product of glucan").

Examples of the modification at a hydroxyl group include hydroxyalkylation, alkylation, acylation, carboxymethylation, sulfation and phosphorylation. It is preferable that modification at a hydroxyl group is a modification which can be removed with an enzyme in a body. The hydroxyl group-modified product of the glucan is preferably an acylated glucan, and further preferably an acetylated glucan. The frequency of introduction of the modifying group(s) into alcoholic hydroxyl groups can be arbitrarily set at the time of a modification reaction of the glucan. The frequency of introduction of the modifying group (s) into alcoholic hydroxyl groups is expressed as DS, and DS1 means the state where one modifying group per glucose residue is introduced. DS can be calculated by DS = (number of modifying group)/(number of glucose residue). Since there is an OH group at the 2-position, the 3-position and the 6-position in an unmodified glucose residue, theoretically, maximum 3 modifying groups per glucose residue can be introduced. For this reason, the upper limit of DS is usually 3. The frequency of introduction of the modifying group(s) into alcoholic hydroxyl groups is about DS 0.01 or more, more preferably about DS 0.03 or more, further preferably about DS 0.05 or more, particularly

preferably about DS 0.07 or more, and most preferably about DS 0.1 or more. The frequency of introduction of modifying group (s) is preferably about DS 1.5 or less, more preferably about DS 1.3 or less, further preferably about DS 1.1 or less, particularly preferably about DS 1.0 or less, and most preferably about DS 0.9 or less. By modifying the glucan, degradation of the glucan in blood or in a body is suppressed.

Examples of modification at a non-reducing end include binding with a targeting molecule such as a mannose residue or a galactose residue. Modification at a non-reducing end will be explained in detail in the following 2.6 and 3 sections. A non-reducing end-modified product is preferably a conjugate with a mannose residue or a conjugate with a galactose residue.

Examples of modification at a reducing end include binding with a substance selected from the group consisting of a monosaccharide, a non-reducing carbohydrate, a biocompatible macromolecule, a liposome constituent component, a glycoside and an amine group-containing low-molecular weight substance. Modification at a non-reducing end will be explained in detail in the following 2.6 and 3 sections.

(1.2) Amino sugar

Amino sugar is a generic name of sugars wherein at least one hydroxyl group is replaced with an amino group. It is preferable that the sugar moiety of an amino sugar is a monosaccharide, and preferably a hexose. When the sugar moiety is a hexose, the number of amino groups in the amino sugar can be any integer of 1 to 4, preferably 1 or 2, and most preferably 1. In the present invention, amino sugars wherein the hydroxyl group at the 2-position of the sugar is replaced with an amino group are preferable. Example of representative amino sugars includes glucosamine, galactosamine, and mannosamine. Glucosamine is the most preferable as the amino sugar in the present invention. A plural kind of amino sugars may be used in mixed, or one kind of amino sugar may be used.

(2.Method for producing an amino sugar-containing glucan)

(2.1) Amino sugar-1-phosphate

In the method of the present invention, amino sugar- 1- phosphate can be used. Preferably, glucosamine- 1- phosphate, galactosamine- 1- phosphate or mannosamine- 1- phosphate is used. The amino sugar- 1- phosphate may be commercially available amino sugar- 1- phosphate, or amino sugar- 1- phosphate synthesized by a chemical method, an enzymatic method, or a biological method such as fermentation. The method for producing amino sugar- 1- phosphate is known in the art. For example, a method for synthesizing glucosamine- 1- phosphate is disclosed in, for example, Nawaji, et al., Carbohydr. Res. 2008, 343, 2692- 2696.

As an amino sugar- 1- phosphate, any of amino sugar- 1- phosphate not in a salt form and amino sugar- 1- phosphate in the form of a salt can be used. For example, as glucosamine- 1- phosphate, any of glucosamine- 1- phosphate not in a salt form and glucosamine- 1- phosphate in the form of a salt can be used. For example, a metal salt of glucosamine- 1- phosphate can be used, and an alkali metal salt of glucosamine- 1- phosphate (for example, disodium glucosamine- 1- phosphate and dipotassium glucosamine- 1- phosphate) can be used.

(2.2) α-Glucan phosphorylase

In the present specification, the term "α- glucan phosphorylase" means an enzymes having α- glucan phosphorylase activity. α- Glucan phosphorylase is classified in EC 2.4.1.1. α- Glucan phosphorylase activity refers to an activity catalyzing a reaction producing glucose- 1- phosphate and partial degraded products of an α- 1, 4- glucan from inorganic phosphate and the α- 1, 4- glucan, or the reverse reaction thereof. α- Glucan phosphorylase can also catalyze an α- 1, 4- glucan synthesizing reaction which is the reverse reaction relative to phosphorolysis. In which direction a reaction proceeds depends on the amount of substrate.

In the present invention, any α-glucan phosphorylase can be used as long as it has a function to transfer a glucosamine residue to a non-reducing end of glucan. α-glucan phosphorylase used in the present invention can be derived from a bacterium, a yeast, an animal or a plant. α-glucan phosphorylase of the present invention can be derived from, for example, potato, sweet potato, *Fava bean, Arabidopsis thaliana,* spinach, corn, rice, wheat, *Citrus hybrid cultivar, Aquifex aeolicus, Thermotoga maritima, Thermococcuszilligii, Thermoanaerobacterpseudethanolicus,* or the like.

It is preferable that α-glucan phosphorylase used in the present invention is α-glucan phosphorylase derived from *Aquifex aeolicus* VF5. In a specific embodiment, potato-derived α-glucan phosphorylase or *Thermococcus zilligii* AN1-derived α-glucan phosphorylase may be used.

The base sequence of α-glucan phosphorylases derived from *Aquifex aeolicus* VF5 is set forth in SEQ ID NO: 1, and its amino acid sequence is set forth in positions 1-692 of SEQ ID NO: 2. The amino acid sequence of α-glucan

phosphorylases derived from *Aquifex aeolicus* VF5 has about 21% to about 24 % sequence identity with the amino acid sequence of plant α-glucan phosphorylases, about 34% sequence identity with the amino acid sequence of α-glucan phosphorylases derived from *Thermus thermophilus,* and about 38% sequence identity with the amino acid sequence of α-glucan phosphorylases derived from *Thermococcus litoralis.* It has about 38% sequence identity with the amino acid sequence of α-glucan phosphorylases derived from *Thermotoga maritima,* about 38% sequence identity with the amino acid sequence of maltodextrin phosphorylases derived from *Thermococcus* zilligii, and about 33% sequence identity with those of *Thermoanaerobacter pseudethanolicus.*

The base sequence of type L α-glucan phosphorylases derived from potato is set forth in SEQ ID NO: 3, and its amino acid sequence is set forth in positions 15-930 of SEQ ID NO: 4. The base sequence of α-glucan phosphorylases derived from *Thermococcus zilligii* AN1 is set forth in SEQ ID NO: 5, and its amino acid sequence is set forth in positions 1-717 of SEQ ID NO: 6.

In the present specification, an enzyme "derived from" an organism, means not only that the enzyme is directly isolated from the organism, but also refers to an enzyme obtained by utilizing the organism in any form. For example, when a gene encoding an enzyme obtained from an organism is introduced into *Escherichia coli,* and the enzyme is isolated from that *Escherichia coli,* the enzyme is referred to as being "derived from" the organism.

In the present specification, "identity" of a sequence (for example, an amino acid sequence, a base sequence and the like) refers to the degree of occurrence of the same amino acid (base when base sequences are compared) between two sequences. Identity can be generally determined by comparing two amino acid sequences or two base sequences, and comparing these two sequences which are aligned in an optimal format, which can contain additions or deletions.

In the present specification, the identity of sequences is calculated using maximum matching of GENETYX-WIN Ver.4.0 (Genetics Co., Ltd.). This program aligns sequence data to be analyzed, and sequence data to be compared so that amino acid pairs matched between sequences become greatest while substitution and deletion are considered, and thereupon, gives a score to each of Matches, Mismatches, and Gaps, calculates a sum, outputs alignment at the smallest sum, and calculates identity thereupon (Reference:Takeishi, K., and Gotoh, O. 1984. Sequence Relationships among Various 4.5 S RNA Species J. Biochem. 92:1173-1177).

For example, the amino acid sequence of α-glucan phosphorylases used in the present invention can be same with SEQ ID NO: 2, 4 or 6, i.e., it can have 100% identity. In another embodiment, as long as having activity to transfer glucosamine residue to non-reducing end of a glucan, this amino acid sequence may be altered in up to a certain number of amino acids compared with a reference amino acid sequence. Such alterations can be selected from the group consisting of a deletion, a substitution (including conservative substitution and non-conservative substitution), or an insertion of at least 1 (for example, 1 or several) amino acids. This alteration may occur at a position of an amino terminus or a carboxyl terminus of the amino acid sequence of SEQ ID NO: 2, 4 or 6, or may occur at any position other than these termini. Alteration of an amino acid residue may be interspersed with one residue, or a few residues may be contiguous. For example, α-glucan phosphorylases used in the present invention may be added with amino acid residues (preferably about 20 or less residues, more preferably about 10 or less residues, and further preferably about 5 or less residues) at either terminus of the amino acid sequence of SEQ ID NO: 2, 4 or 6, for the reasons such as to make ease of purification of the enzyme, to increase stability, or the like.

The α-glucan phosphorylase used in the present invention has an amino acid sequence which has preferably about 50% or more, more preferably about 60% or more, further more preferably about 70% or more, still more preferably about 80% or more, particularly more preferably about 90% or more, and most preferably about 95% or more sequence identity with the amino acid sequence of SEQ ID NO: 2, 4 or 6 and has an activity transferring a glucosamine residue to a non-reducing end of the glucan. The α-glucan phosphorylase used in the present invention can have an amino acid sequence which has about 96 % or more, about 97% or more, about 98 % or more, or about 99 % more sequence identity with amino acid sequence of SEQ ID NO: 2, 4 or 6.

The amount of the α-glucan phosphorylase contained in a solution at the start of the reaction is preferably about 0.01 U/ml or more, more preferably about 0.1 U/ml or more, particularly preferably about 0.5 U/ml or more, and most preferably about 1 U/ml or more. The amount of the α-glucan phosphorylase contained in a solution at the start of the reaction is preferably about 1, 000 U/ml or less, more preferably about 100 U/ml or less, particularly preferably about 50 U/ml or less, and most preferably about 20 U/ml or less. If the weight of α-glucan phosphorylase is too large, it may became easy to aggregate the enzyme denatured during the reaction. If the amount used is too small, reaction itself occurred, but the yield of glucan may be lowered. It is noted that unit amount of α-glucan phosphorylase is defined as follows:

Regarding one unit of α-glucan phosphorylase, an α-glucan phosphorylase activity which produces 1 μmol inorganic phosphate (Pi) per one minute shall be one unit (U or Unit) . This measurement of α-glucan phosphorylase activity quantitates free inorganic phosphate (Pi) produced from G-1-P. After 200 μl of a reaction solution (containing 12.5 mM G-1-P, 1% dextrin and an enzyme solution in a 100 mM acetate buffer (pH 6.0)) is incubated at 50°C for 15

minutes, 800 μl of a molybdenum regent (15 mM ammonium molybdate, 100 mM zinc acetate) is added, and this is stirred to stop the reaction. 200 μl of 568 mM ascorbic acid (pH 5.8) is added, followed by mixing. After incubation at 30°C for 15 minutes, an absorbance is measured at 850 nm using a spectrophotometer. An absorbance is measured similarly usingin organic phosphate having the known concentration, and a standard curve is produced. An absorbance value obtained for a sample is fitted to this standard curve, and the amount of inorganic phosphate in the sample is determined. Inorganic phosphate is quantitated as a phosphoric acid ion. The amount of glucose-1-phosphate is not quantitated.

(2.3 Production of α-glucan phosphorylase)

α- Glucan phosphorylase used in the present invention can be directly isolated from an organism producing α- glucan phosphorylase, such as the aforementioned organisms, present in the natural world. Alternatively, α- glucan phosphorylase used in the present invention may be isolated from a microorganism (for example, bacteria, fungi and the like) which has been genetically modified with a gene encoding α- glucan phosphorylase isolated from the aforementioned organism.

In a preferred embodiment, α-glucan phosphorylase derived from *Aquifex aeolicus* VF5 is produced by chemically synthesizing a gene fragment of SEQ ID NO: 1, constructing an expression vector containing this gene fragment, introducing this expression vector into a microorganism to make a recombinant microorganism, culturing this recombinant microorganism to produce α-glucan phosphorylase, and collecting produced α-glucan phosphorylase. α-glucan phosphorylase derived from other organism can also be produced as well. An enzymatic production method by gene recombination is well-known to those skilled in the art. A host microorganism used in the present invention includes a prokaryote and a eukaryote, and a mesophile is preferable. Examples of a particularly preferred microorganism include, but not limited to, *Escherichia coli.*

α- glucan phosphorylase having the amino acid sequence of positions 1- 692 of SEQ ID NO: 2, positions 15-930 of SEQ ID NO: 4, or positions 1- 717 of SEQ ID NO: 6, or an amino acid sequence having homology thereto and having glucosamine transferring activity, and a polynucleotide encoding the α- glucan phosphorylase which are used in the present invention can be produced using conventional genetic engineering techniques.

(2.4) Production of amino sugar-containing glucan

The amino sugar- containing glucan of the present invention can be produced by a method including a step of allowing a reaction of a solution containing glucosamine- 1- phosphate, a glucan, and α- glucan phosphorylase (for example, α- glucan phosphorylase derived from *Aquifex aeolicus* VF5, α- glucan phosphorylase derived from potato, or α- glucan phosphorylase derived from *Thermococcus zilligii* AN1) which can catalyze a reaction of transferring a glucosamine residue to a non- reducing end of a glucan. By using a glucan modified product in place of a glucan in this method, a modified product of the amino sugar- containing glucan can be produced. As an example, a method using a glucan will be explained below.

First, a reaction solution is prepared. The reaction solution can be prepared, for example, by adding an amino sugar- 1- phosphate (such as glucosamine- 1- phosphate) , a glucan, and α- glucan phosphorylase to a suitable solvent. If necessary, any buffer and inorganic salts for the purpose of adjusting the pH, as far as an enzymatic reaction is not inhibited, may be added to this reaction solution. If necessary, glucose- 1- phosphate which is an original substrate of α- glucan phosphorylase may be added to this reaction solution. In the case of a reaction where an amino sugar- 1- phosphate and glucose- 1- phosphate co- exist, a reaction of binding a glucose residue to a non- reducing end of a receptor glucan and a reaction of binding an amino monosaccharide residue are simultaneously performed. Even when an amino monosaccharide residue is bound to a non- reducing end of the glucan, α- glucan phosphorylase can further transfer a molecule to the non- reducing end of the amino monosaccharide residue, however, the efficiency of the transfer is much lower than the efficiency of transferring to a glucose residue. When a glucose residue is bound to a non- reducing end of a glucan, α- glucan phosphorylase can further transfer a glucose residue or an amino monosaccharide residue to a non- reducing end of a resulting molecule. For this reason, when glucose- 1- phosphate co- exists, the chain length of the glucan can be extended efficiently. Therefore, the structure of the finally obtained amino sugar- containing glucan is controlled by a ratio between added amino sugar- 1- phosphate and added glucose- 1- phosphate. If necessary, an enzyme selected from the group consisting of a debranching enzyme, a branching enzyme, 4- α- glucanotransferase and a glycogen debranching enzyme may be added to this reaction solution.

By changing the ratio, in a reaction solution, of the amount of amino sugar-1-phosphate and the number as a population of non-reducing ends of a glucan, frequency of introduction of amino monosaccharide residue can be controlled. That is, as increasing (number of molecule of glucan) x (amount of amino sugar-1-phosphate relative to number of non-reducing ends in the molecule), the frequency of introduction of amino monosaccharide residue can be increased. Also by regulating the amount of enzyme added or enzyme reaction time, the frequency of introduction of amino monosaccharide residue can be regulated.

Then, the reaction solution is reacted, if necessary, by heating by a method known in the art. The reaction temperature can be any temperature, as far as the effect of the present invention is obtained. The reaction temperature can be representatively a temperature of about 30°C to about 90°C. It is preferable that the temperature of a solution in this reaction step is such a temperature that, after a predetermined reaction time, about 50% or more, more preferably about 80% or more activity of the activity of the α- glucan phosphorylase contained in this solution before the reaction remains. α- Glucan phosphorylase derived from *Aquifex aeolicus* VF5 is a thermostable enzyme, and its optimal reaction temperature is about 80°C to 90°C. From the viewpoint of the reaction speed, it is preferable that the reaction temperature is high to some extent. On the other hand, from the viewpoint of the optimal reaction temperature of α- glucan phosphorylase derived from *Aquifex aeolicus* VF5, a reaction at about 80°C to 90°C is possible. However, from the viewpoint of stability of the resulting product, stability of amino sugar- 1- phosphate and glucose- 1- phosphate, and the like, it is preferable that the reaction temperature is slightly lower than the optimal reaction temperature of the α- glucan phosphorylase derived from *Aquifex aeolicus* VF5. The reaction temperature is preferably about 30°C or higher, more preferably about 35°C or higher, further preferably about 40°C or higher. In a particular embodiment, the reaction temperature may be about 45°C or higher or about 50°C or higher. The reaction temperature is preferably about 90°C or lower, more preferably about 80°C or lower, further preferably about 70°C or lower. In a particular embodiment, the reaction temperature may be about 65°C or lower or about 60°C or lower. In the case where an enzyme for which an optimum reaction temperature is lower such as potato- derived α- glucan phosphorylase is used, it is preferable that the reaction temperature is set lower than the case where *Aquifex aeolicus* VF5- derived α- glucan phosphorylase is used.

The reaction time can be set in any time period, in view of the reaction temperature and remaining activity of an enzyme. The reaction time is representatively about 1 hour to about 100 hours, more preferably about 1 hour to about 72 hours, further more preferably about 2 hours to about 36 hours, and most preferably about 2 hours to about 24 hours. In a particular embodiment, the reaction time may be, for example, about 1 hour or longer, about 2 hours or longer, about 5 hours or longer, about 10 hours or longer, about 12 hours or longer, or about 24 hours or longer. In a particular embodiment, the reaction time may be, for example, about 100 hours or shorter, about 72 hours or shorter, about 60 hours or shorter, about 48 hours or shorter, about 36 hours or shorter, or about 24 hours or shorter.

Heating may be performed using any means, but it is preferable that heating is performed with stirring so as to homogeneously transmit the heat to the whole solution. The solution is stirred by placing it into, for example, a reaction tank made of stainless steel, provided with a warm water jacket and a stirring device.

Furthermore, in the method of the present invention, at least one of amino sugar- 1- phosphate, a glucan, and α- glucan phosphorylase may be further added to a reaction solution at a stage where the reaction has proceeded to some extent.

In this way, a solution containing an amino sugar-containing glucan is produced.

After completion of the reaction, in the reaction solution, if necessary, an enzyme in the reaction solution can be inactivated by, for example, heating at 100°C for 10 minutes. Alternatively, a post step maybe performed without performing treatment of inactivating an enzyme. The reaction solution may be stored as it is, or may be treated in order to isolate the produced amino sugar-containing glucan.

After completion of the reaction, after the amino sugar-containing glucan is purified, or before the amino sugar-containing glucan is purified, a hydroxyl group-modified product of the amino sugar-containing glucan maybe produced by modifying at least one of alcoholic hydroxyl groups of a glucan moiety of the resulting amino sugar-containing glucan. It is preferable that modification is performed after purification of the amino sugar-containing glucan. Modification can be performed according to a method known in the art. Examples of modification include hydroxyalkylation, alkylation, acylation, carboxymethylation, sulfation, and phosphorylation. Acylation is preferable, and acetylation is more preferable. By modifying a reducing end of the glucan after producing the amino sugar-containing glucan or a hydroxyl group-modified product of the amino sugar-containing glucan, a reducing end-modified product of the amino sugar-containing glucan or a hydroxyl group-modified product of the amino sugar-containing glucan may be produced. Further, a non-reducing end of these glucan moiety to which no amino sugar is bound, may be modified. Binding of an amino monosaccharide residue to the glucan, modification of a hydroxyl group, modification of a reducing end, and modification of some of non-reducing ends with a modifying group other than an amino monosaccharide residue may be performed in any order.

(2.5 Purification of amino sugar-containing glucan) <Purification method>

The produced amino sugar- containing glucan (oramodified product thereof) can be purified as necessary. Examples of the impurities removed by purification include inorganic phosphate, amino sugar- 1- phosphate, inorganic salts and the like. Examples of a method of purifying a glucan include a method using an organic solvent (T. J. Schoch et al., J. American Chemical Society, 64, 2957 (1942) ) and a method not using an organic solvent.

Examples of the organic solvent which can be used in purification using the organic solvent include acetone,

n- amyl alcohol, pentazole, n- propyl alcohol, n- hexyl alcohol, 2- ethyl- 1- butanol, 2- ethyl- 1- hexanol, lauryl alcohol, cyclohexanol, n- butyl alcohol, 3- pentanol, 4- methyl- 2- pentanol, d, 1- borneol, α- terpineol, isobutyl alcohol, sec- butyl alcohol, 2- methyl- 1- butanol, isoamyl alcohol, tert- amyl alcohol, menthol, methanol, ethanol and ether.

As an example of the purification method not using an organic solvent, there is a method of removing inorganic phosphate, amino sugar- 1- phosphate, and inorganic salts by subjecting an amino sugar- containing glucan to membrane fractionation using an ultrafiltration membrane or chromatography, without precipitating the amino sugar- containing glucan dissolved in water, after the amino sugar- containing glucan production reaction.

Examples of the ultrafiltration membrane which can be used in purification include an ultrafiltration membrane of a molecular weight cut off of about $1 \times 10^3$ to about $1 \times 10^4$, preferably about $5 \times 10^3$ to about $5 \times 10^4$, more preferably about $1 \times 10^4$ to about $3 \times 10^4$ (UF membrane unit manufactured by DAICEL).

Examples of a support which can be used in chromatography include a support for gel filtration chromatography, a support for ligand exchange chromatography, a support for ion-exchange chromatography and a support for hydrophobic chromatography.

In a method for production of the amino sugar-containing glucan of the present invention, since a glucan having high molecular weight is used, the method has an advantage that separation after production is easy. In detail, since the producing reaction of an amino sugar-containing glucan reaches equilibrium when the concentration of the reaction byproduct inorganic phosphate increases, a large amount of amino sugar-1-phosphate remains in the reaction solution after completion of the reaction. When a glucan having low molecular weight is used, the resulting amino sugar-containing glucan and amino sugar-1-phosphate are both cationic. Since they have almost the same molecular weights and charges, separation is very difficult. For this reason, an amino sugar-containing glucan which does not comprise amino sugar-1-phosphate as an impurity cannot be produced. However, when a glucan having high molecular weight was used, by applying a method for production utilizing the difference of molecular weight, an amino sugar-containing glucan which does not comprise amino sugar-1-phosphate as an impurity could be produced. Glucan and the amino sugar-containing glucan can be separated easily by ion chromatography or the like, utilizing the difference in charge.

(2.6) Amino sugar-containing glucan and a modified product thereof and a conjugate

The amino sugar- containing glucan of the present invention is a glucan wherein at least one amino monosaccharide residue is bound via an α- 1, 4- bond to each of at least non- reducing end of glucan. To the end of amino monosaccharide bound to the non- reducing end of the glucan, if necessary, a sugar or the like may further be bound. When two or more amino monosaccharide residues are bound to a non- reducing end of glucan in the amino sugar-containing glucan of the present invention, the structure is that wherein these amino monosaccharides are bound and bind to one end. For example, the structure is that wherein one amino monosaccharide is bound to one end of glucan, and to the end of the amino monosaccharide, the next amino monosaccharide is further bound. In one embodiment, only one amino monosaccharide is bound to each end of glucan.

In the amino sugar-containing glucan of the present invention, amino monosaccharide (for example, glucosamine) may be bound to all of the non-reducing ends of glucan, or amino monosaccharide may be bound to only a part of the non-reducing ends. That is, some of the non-reducing ends can remain unbound by an amino monosaccharide.

Conversion ratio based on the number of non-reducing end(s), that is, the ratio of the number of bond (s) between amino monosaccharide(s) and non-reducing end(s) relative to the number of non-reducing end(s) present before amino monosaccharide(s) is/are bound, can be selected as appropriate depending on the application wherein the amino sugar-containing glucan is used or the like, from the ratio close proximity of 0% (for example, about 1%) to 100%. For example, when it is desired that a small amount of amino group (s) be present, a low conversion ratio can be selected, and when it is desired that a large amount of amino group (s) be present, a high conversion ratio can be selected.

By appropriately adjusting the condition of the enzymatic reaction in producing an amino sugar-containing glucan, for example, by adjusting the reaction time, the concentration of the substrate or the like of the enzymatic reaction, an amino sugar-containing glucan having a selected conversion ratio can easily be obtained.

Further, the ratio of the total number of amino monosaccharide (s) bound to non- reducing end (s) relative to the number of non- reducing end (s) present in a glucan before amino monosaccharide (s) is/are bound can be used as an indicator for the amount of amino group (s) . In the present specification, this ratio is described as binding ratio. In general, in analyzing an amino sugar- containing glucan, it is easier to measure the binding ratio than to measure the above- mentioned conversion ratio, therefore, it is advantageous from the practical viewpoint to design an amino sugar-containing glucan based on binding ratio.

It is noted that depending on the condition of enzymatic reaction in synthesizing an amino sugar-containing glucan, to an end of an amino monosaccharide bound to one non-reducing end, an amino monosaccharide may be further bound in some cases. That is, in some cases, the structure may be that wherein two amino monosaccharides are serially bound to one non-reducing end (that is, a structure wherein a disaccharide is bound to a non-reducing end).

A compound having such a structure can also be used as the amino sugar-containing glucan of the present invention. The above-mentioned binding ratio has an advantage that the amount of amino group (s) can be evaluated even if the amount of amino group(s) is increased by binding a plurality of amino monosaccharides to one non-reducing end as described above. For this reason, in contrast to the above-mentioned conversion ratio, the binding ratio may have a value greater than 100% in some cases. The binding ratio can be selected as appropriate depending on the application wherein the amino sugar-containing glucan is used or the like. For example, when it is desired that a small amount of amino group(s) be present, a low binding ratio (for example, about 1% to about 2%) can be selected, and when it is desired that a large amount of amino group(s) be present, a high binding ratio (for example, bout 150% to about 200%) can be selected.

When the application of the amino sugar-containing branched glucan is a nanoparticulate carrier for a DDS, the binding ratio of the amino monosaccharide residue is preferably about 10% or more, more preferably about 20% or more, further preferably about 30% or more, and most preferably about 40% or more; the binding ratio of the amino monosaccharide residue is preferably about 500% or less, more preferably about 400% or less, further preferably about 300% or less, and most preferably about 200% or less.

When the application of the amino sugar-containing branched glucan is a complex carrier, the binding ratio of the amino monosaccharide residue is preferably about 1% or more, more preferably about 5% or more, further preferably about 10% or more, and most preferably about 20% or more; the binding ratio of the amino monosaccharide residue is preferably about 500% or less, more preferably about 400% or less, further preferably about 300% or less, and most preferably about 200% or less.

When the application of the amino sugar-containing branched glucan is a nanoparticulate carrier for a DDS, the number of bond of amino monosaccharide residue per glucan molecule is preferably about 2 or more, more preferably about 5 or more, further preferably about 10 or more, and most preferably about 20 or more; the number of bond of amino monosaccharide residue per glucan molecule is preferably about 5000 or less, more preferably about 4000 or less, further preferably about 3000 or less, and most preferably about 2000 or less.

When the application of the amino sugar-containing branched glucan is a complex carrier, the number of bond of amino monosaccharide residue per glucan molecule is preferably about 10 or more, more preferably about 50 or more, further preferably about 100 or more, and most preferably about 200 or more; the number of bond of amino monosaccharide residue per glucan molecule is preferably about 5000 or less, more preferably about 4000 or less, further preferably about 3000 or less, and most preferably about 2000 or less.

When an amino sugar-containing branched glucan forms a complex with a nucleic acid, the binding ratio in the complex of the amino sugar-containing glucan of the present invention and the nucleic acid is preferably about 50% or more, more preferably about 60% or more, and further preferably about 70% or more in 0.1 M NaCl aqueous solution at 37°C; the binding ratio is preferably about 40% or less, further preferably about 30% or less, and most preferably about 20% or less in 1 M NaCl aqueous solution at 37°C. The binding ratio can be measured by the method described in Example 10.

In the amino sugar-containing glucan of the present invention, a sugar other than amino sugar may be bound to the non-reducing end to which no amino monosaccharide is bound. That is, some of non-reducing ends may bind with an amino monosaccharide, and the remaining non-reducing end (s) may bind with a sugar other than amino sugar.

Alternatively, it is also possible to make a structure wherein some of non- reducing ends bind to amino monosaccharide (s) , some bind to a sugar other than amino sugar, and the remaining non- reducing end (s) bind to nothing. In this regard, it is possible that among a plurality of non- reducing ends, the number of end (s) binding to amino monosaccharide (s) , the number of end (s) binding to a sugar other than amino monosaccharide, and the number (s) of end not binding to a sugar be selected freely.

The amino sugar-containing glucan and a modified product thereof of the present invention can be further bound at its amino group on the amino sugar residue to a medically effective ingredient to thereby obtain a conjugate. An amino sugar containing glucan conjugated with a medically effective ingredient is referred to as an "amino sugar-containing glucan-medically effective ingredient conjugate", and a modified product of an amino sugar-containing glucan conjugated with a medically effective ingredient is referred to as a "modified product of an amino sugar-containing glucan-medically effective ingredient conjugate". Similarly, in the case where the amino sugar is glucosamine, glucosamine-containing glucan conjugated with a medically effective ingredient is referred to as a "glucosamine-containing glucan-medically effective ingredient substance conjugate", and a modified product of glucosamine-containing glucan conjugated with a medically effective ingredient is referred to as a "modification product of glucosamine-containing glucan-medically effective ingredient conjugate".

A modification product of an amino sugar-containing glucan can be a hydroxyl group-modified product, a non-reducing end-modified productor a reducing end-modified product.

The hydroxyl group-modified product is as described above.

The non- reducing end- modified product will be explained. When a glucan moiety of the amino sugar- containing glucan or a modified product thereof is a branched $\alpha$- 1, 4- glucan, and there is a non- reducing end to which no amino

sugar residue is bound, other substances can be bound to a non- reducing end to which no amino sugar residue is bound. In a preferred embodiment, a targeting molecule is bound to a non- reducing end to which no amino sugar residue is bound. In the present specification, the term "targeting molecule" refers to a molecule having tissue targeting function. Examples of a targeting molecule include mannose, galactose, xylose, fucose, galactosamine, an antibody, an antibody fragment, a receptor, a receptor fragment and a receptor ligand. Particularly, since galactose is recognized by an asialoglycoprotein receptor present on a surface of a hepatic parenchymal cell, it is effective. In addition, since mannose is recognized by a mannose receptor expressed on a variety of macrophages including a Kupffer cell and a sinusoid vascular endothelial cell of liver, it is effective. Mannose and galactose can be bound to a non- reducing end of the amino sugar- containing glucan or a modified product thereof, for example, by allowing an $\alpha$- glucan phosphorylase to act to mannose- 1- phosphate or galactose- 1- phosphate as a substrate. When bound by an enzymatic reaction, mannose and galactose are bound at the position 4 of a non- reducing terminal glucose residue to which no amino sugar residue is bound. It is also possible to combine mannose and galactose at any proportion and bind to a plurality of non- reducing ends of the branched glucan. In one embodiment, mannose or galactose can bind to an amino sugar residue bound to the non- reducing end.

The reducing end-modified product will be explained. The "reducing end-modified product of the amino sugar-containing glucan" refers to a substance in which another substance is bound to a reducing end present in the amino sugar-containing glucan of the present invention. In a preferred embodiment, as a method of binding with a different substance at a reducing end, there are the following two methods. The first method is a method of binding a reducing end of a glucan having a degree of polymerization of $5 \times 10^3$ or more, more preferably a degree of polymerization of $1 \times 10^4$ or more, further preferably a degree of polymerization of $1 \times 10^5$ or more to another substance by a known enzymatic procedure or a known chemical procedure and, thereafter, binding an amino sugar to a non-reducing end of the glucan using the method of the present invention. The second method is a method of binding a reducing end of the amino sugar-containing glucan of the present invention to another substance by a known enzymatic procedure.

A method of enzymatically binding a glucan to a different substance in the first method is disclosed, for example, in Japanese Laid-Open Publication No. 5-276883, Japanese Laid-Open Publication No. 07-241181 and International Publication No. WO 01/073106. A glucan having high-molecular weight can be bound to a different substance similarly to these methods.

The method of chemically binding a glucan to a different substance in the first method can be used for substances having an amine group. For example, as a method of chemically binding maltopentaose and a substance having an amine group, there are the following three kinds of methods:

(A) A method of binding reducing terminal aldehyde of maltopentaose and a substance having an amine group by reductive amination;
(B) A method of oxidizing reducing terminal aldehyde of maltopentaose into maltotetraosyl gluconic acid and, thereafter, dehydration-condensing this with a substance having an amine group with a condensing agent; and
(C) A method of oxidizing reducing terminal aldehyde of maltopentaose into maltotetraosyl gluconic acid and, thereafter, dehydrating this to prepare maltotetraosyl gluconolactone, and heating this and a substance having an amine group under an anhydrous solvent condition to allow binding. The three kinds of methods (A), (B) and (C) are described in detail in Japanese Patent Application No. 2008-121693. A glucan having high-molecular weight can be bound to a different substance similarly to these methods.

An enzyme utilizable in the second method that is a method of binding a reducing end of the amino sugar-containing glucan of the present invention to a different substance by an enzymatic procedure can be applied only to carbohydrates and glycosides. As the enzyme, a so-called glucan chain transferring enzyme such as a branching enzyme, CGTase, a D enzyme, amylomaltase or the like is used. These enzymes cut an $\alpha$-1,4 bond in the amino sugar-containing glucan, and transfer a fragment on its non-reducing end side (amino sugar-containing fragment) to a receptor molecule (herein, a carbohydrate or a glycoside).

Examples of a substance to be bound to the reducing end include monosaccharides, non-reducing carbohydrates, biocompatible macromolecules, liposome constituent components, glycosides, and amine group-containing low-molecular weight substances.

Examples of monosaccharides include monosaccharides having a functional group, such as N-acetylglucosamine, gluconic acid and the like. Examples of non-reducing carbohydrates include sorbitol, sucrose, and trehalose. Examples of biocompatible macromolecules include amylose, dextrin, starches, cellulose, chitin, chitosan, dextran, proteins and peptides. Examples of liposome constituent components include phospholipids, fatty acids and surfactants. Examples of glycosides include ascorbic acid glucoside, hydroquinone glucoside, hesperidin glucoside, rutin glucoside, para-nitrophenyl maltopentaose, dodecylmaltose, flavonoid glycosides, terpene glycosides, phenol glycosides, chalcone glycosides and steroid glycosides. Examples of the amine group-containing low-molecular weight substances include various amino acids, dodecylamine, and the like.

An embodiment in which a different substance is bound to an amino group of an amino sugar residue of the amino sugar-containing glucan or a modified product thereof will be described in detail in the following "3".

(3. Utilization of amino sugar-containing glucan and modified product thereof)

Since in the amino sugar-containing glucan and a modified product thereof of the present invention, an amino sugar residue is bound to a non-reducing end of a glucan, they have an amino group on a non-reducing end and, as a result, can positively charge the glucan in an aqueous solution. For example, the amino sugar-containing glucan and a modified product thereof of the present invention in which a number of amino sugars are bound to non-reducing ends of the glucan, can generate a state wherein an amino group of amino sugar on a non-reducing end is $NH^{3+}$ and a state wherein the amino group remains as $NH_2$, by changing the pH of a solvent. It is thought that, in the state wherein an amino group of an amino sugar residue on a non-reducing end is dissociated, the amino sugar-containing glucan, a modified product thereof and a conjugate thereof come to have an extended outer structure by electrostatic repulsion and, in the state where the amino group is not dissociated, the amino sugar-containing glucan and a modified product thereof come into a shrunk state. Such a pH dependent change in conformation of a polysaccharide microgel can be utilized for medicament delivery.

The amino sugar-containing glucan and a modified product thereof of the present invention have an amino group, which is a reactive group, on a non-reducing end. For this reason, via this amino group, a glucan chain can be bound to a different substance (for example, a medically effective ingredient) directly or indirectly via a suitable spacer. As a result, physical properties of the substance can be altered, and a function can be imparted to the substance. A different substance referred to herein may be any of a low-molecular weight organic compound, a macromolecule organic compound, a nanoparticulate carrier for a DDS (a macromolecule micelle, virus particles, a liposome or the like), and an inorganic substance (for example, magnetic microparticles). The different substance is preferably a medically effective ingredient, and more preferably a medically effective ingredient which is negatively charged in an aqueous solution. For example, the amino sugar-containing glucan and a modified product thereof of the present invention can be easily bound to a substance having a carboxyl group, by reacting with the substance having a carboxyl group, in the presence of a suitable condensing agent such as carbodiimide. When the substance having a carboxyl group is a medically effective ingredient such as a peptide or a protein, the resulting compound is a conjugate in which the amino sugar-containing glucan or a modified product thereof and the medically effective ingredient are directly bound. Alternatively, the medically effective ingredient can be bound to the amino sugar-containing glucan or a modified product thereof via a spacer. In this case, a compound having a functional group which can be utilized in binding of an amine group and the medically effective ingredient can be bound to an amino group of the amino sugar-containing glucan or a modified product thereof. Binding of a different compound to an amino group for binding with the medically effective ingredient or the like is referred to as "modification of an amino group". As the effect of altering physical properties by impartation of a glucan chain, improvement in water solubility, impartation of bioaffinity due to formation of a hydration layer and the like can be expected.

For modifying an amino group, a modifying reagent having a carboxyl group and a different functional group canbeused. In the present specification, a substance having a carboxyl group which is used for modifying an amino group of the amino sugar- containing glucan and a modified product of the amino sugar- containing glucan is also referred to as an "amino group- modifying reagent". The amino group- modifying reagent has at least one carboxyl group and at least one different function group. Examples of this functional group include a cationic functional group, an anionic functional group, a hydrophobic group, a maleimide group, a thiol group and an aldehyde group. Examples of the cationic functional group include an amino group, a dimethylamino group, a diethylamino group, a trimethylamino group, an ammonium group, and a pyridinium group. Examples of the modifying reagent having a cationic functional group include 3- amino- 2, 5- dichlorobenzoic acid. Examples of the anionic functional group include a phosphoric acid group, a sulfonic acid group, a carboxyl group, and a sulfuric acid group. Examples of the modifying reagent having an anionic functional group include oxalic acid, malonic acid, succinic acid, glutalic acid, phthalic acid and citric acid. Examples of the hydrophobic group include alkyl groups such as a stearyl group, a palmityl group, a methyl group, a propyl group, and a butyl group, and aryl groups such as a phenyl group, a benzyl group, and a tolyl group. Examples of the modifying reagent having a hydrophobic group include stearic acid, benzoic acid, and cinnamic acid. Examples of the modifying reagent having a maleimide group include N- (4- maleimidebutyryloxy) succinimide. The thiol group is also called mercapto group. Examples of the modifying reagent having a thiol group include thioglycolic acid and mercaptobenzoic acid. Examples of the aldehyde group include a saturated acyclic aldehyde group, an unsaturated acyclic aldehyde group, a saturated alicyclic aldehyde group, and an aromatic aldehyde group. Examples of the modifying reagent having an aldehyde group include 2- carboxybenzaldehyde.

For example, in the case where a substance having a carboxyl group is a polysaccharide including an uronic acid such as arginic acid or hyaluronic acid, it is possible to graft (that is, bind) a large number of the amino sugar-containing glucans of the present invention to the main chain, to thereby it is possible to change largely the physical property of the uronic acid-containing polysaccharide. In this case, a conjugate of an amino sugar-containing glucan or

a modified product thereof and an uronic acid-containing polysaccharide is formed. In this conjugate, the amino sugar-containing glucan or the modified product thereof and the uronic acid-containing polysaccharide are bound directly. In a specific embodiment, no uronic acid residue is bound to the amino sugar-containing glucan of the present invention.

In addition, when the substance having a carboxyl group is a phospholipid, a phospholipid to which the amino sugar-containing glucan or a modified product thereof of the present invention is bound can be obtained. Such a phospholipid is also referred to as a conjugate of an amino sugar-containing glucan or a modified product thereof and a phospholipid. In this conjugate, the amino sugar-containing glucan or a modified product thereof and a phospholipid are directly bound. By producing a liposome using such a glucan-bound phospholipid, a glucan chain-bound liposome which can be utilized in delivery of medicaments can be easily obtained.

When the substance having a carboxyl group is a proteinaceous medically effective ingredient such as a protein or a peptide, the proteinaceous medically effective ingredient such as a protein or a peptide to which a glucan chain is bound can be obtained. Such a protein or peptide is also referred to as a conjugate of an amino sugar-containing glucan or a modif iedproduct thereof and a protein or a peptide. In this conjugate, the amino sugar-containing glucan or a modified product thereof and a protein or a peptide are directly bound. This technique can be utilized in improving the pharmacokinetics of a proteinaceous medically effective ingredient (medicament).

When the substance having a carboxyl group is a magnetic microparticle, a magnetic microparticle with a glucan chain bound thereto can be obtained, and this can be utilized as a contrast agent for clinical diagnosis. In this case, a conjugate of the amino sugar-containing glucan or a modified product thereof and the magnetic microparticle is formed. In this conjugate, the amino sugar-containing glucan or a modified product thereof and the magnetic microparticle are directly bound.

When the substance having a carboxyl group is a metal ligand (chelating agent), a metal ligand with a glucan chain bound thereto can be obtained, and this can be utilized as a contrast agent for clinical diagnosis by coordination with a radioactive metal element. In this case, a conjugate of the amino sugar-containing glucan or a modified product thereof and the metal ligand is formed. In this conjugate, the amino sugar-containing glucan or a modified product thereof and the metal ligand are directly bound.

The amino sugar-containing glucan of the present invention has an amino group, which is a reactive group, on a non-reducing end. The amino group is charged positively under a neutral condition in an aqueous solution. On the other hand, by chemically modifying this amino group, an anionic functional group or a hydrophobic group can be introduced into an end of the glucan. On the other hand, glucans having a variety of structures and molecular weights can be utilized in a glucan moiety of the present invention. When these techniques are combined, for example, a cationic functional group, an anionic functional group, a hydrophobic group and the like can be controllably introduced into a terminus of a branched glucan having such an extent of molecular weight that a protein can be enclosed. An end of such a modified glucan can be flexibly transferred, performs electrostatic interaction with a charged part present on a protein surface, and performs hydrophobic interaction with a hydrophobic region and, as a result, the glucan of the present invention forms a complex via a non-covalent bond with a protein. Therefore, by mixing the amino sugar-containing glucan or a modified product thereof of the present invention with a protein, a peptide or the like in a solution, a complex of the amino sugar-containing glucan or a modified product thereof of the present invention and a protein, a peptide, or the like can be formed. Similarly, the terminal structure of the glucan which can effectively form a non-covalently bound complex with a nucleic acid, a liposome, a virus particle, a macromolecule micelle, or a low-molecular weight compound can also be designed. As described above, the glucan and an terminal derivative thereof of the present invention can effectively form a complex with a protein, anucleicacid, a low-molecular weight compound, or a nanoparticulate carrier for a DDS (for example, a liposome, a macromolecule micelle, or virus particles), and can influence on stability, physical properties, absorbability, pharmacokinetics (for example, organ accumulating property, tissue targeting property, or blood retention property) and the like of them. As described above, the glucan of the present invention can be effectively utilized as a DDS carrier of the medically effective ingredient of medicaments or as an agent for modifying a nanoparticulate carrier for a DDS.

When the aminosugar- containing glucan of the present invention is utilized as the modifying material of the medically effective ingredient of medicaments, it is necessary to control degradation of the modifying material in a body. When the glucan is a linear $\alpha$- 1, 4- glucan, it undergoes rapid degradation with $\alpha$- amylase, and the time of existence in a body may be too short. In such a case, there is a possibility that an object as the modifying material cannot be attained. Then, by modifying some or all of hydroxyl groups of glucose constituting a glucan, the necessary time for degradation can be controlled. A hydroxyl group- modified product of such an amino sugar- containing glucan is preferable in the present invention. Modification is etherification or esterification. Etherification is preferably etherification with alkyl halide or an alcohol. The number of carbon atoms of alkyl halide or an alcohol used in etherification is preferably 1 to 10, more preferably 1 to 5, further preferably 1 to 3. The halogen group can be preferably fluoro, chloro, bromo or iodo. Esterification is preferably esterification with carboxylic acid or acyl halide. The number of carbon atoms of carboxylic acid or acyl halide used in esterification is preferably 1 to 10, more preferably 1 to 5. Modification is desirably esterification. Esterification is more preferably acylation, further preferably acetylation.

As described above, the amino sugar-containing glucan and a modified product thereof and a conjugate thereof of the present invention (preferably, an amino sugar-containing glucan and a modified product thereof and a conjugate thereof) can be freely designed for their structure of the glucan and the structure of its non-reducing end and, by utilizing this, the pharmacokinetics of the medically effective ingredient of medicaments can be arbitrary controlled. This is the modifying material of the medically effective ingredient of medicaments, which has a structure completely degradable in a body, and can be safely utilized without anxiety for toxicity due to accumulation.

Therefore, according to the present invention, a medicament containing the amino sugar- containing glucan or a modified product thereof of the present invention (preferably, an amino sugar- containing glucan and a modified product thereof) and the medically effective ingredient is provided. In the medicament of the present invention, the medically effective ingredient is preferably selected from the group consisting of a low- molecular weight organic compound, a protein, a peptide, an antibody, an antibody fragment, a receptor, a receptor fragment, a DNA, an RNA, a siRNA, an miRNA and an RNA aptamer, as well as the modified product thereof. Examples of the modification of a medically effective ingredient include, for example, addition of a functional group (phosphorylation, carboxylation, amidation and the like) . Example of the modification of a nucleic acid include amination, thiolation, phosphorothioation, glycerylation, and a chemical modification imparting cationic property (for example, introduction of cation group such as an amino group) .

According to a particular embodiment of the present invention, there is provided a conjugate of a medically effective ingredient and the amino sugar-containing glucan, a hydroxyl group-modified product thereof, a reducing end-modified product thereof, or an amino group-modified product thereof of the present invention, wherein the medically effective ingredient is covalently bound with at least one of amino groups of the amino sugar residue directly, or is bound with at least one of amino groups of the amino sugar residue via a spacer. The medically effective ingredient is preferably selected from the group consisting of a low-molecular weight organic compound, a protein, a peptide, an antibody, an antibody fragment, a receptor, a receptor fragment, a DNA, an RNA, a siRNA, an miRNA and an RNA aptamer, as well as the modified product thereof.

According to a particular embodiment, the present invention provides a composition for clinical diagnosis, containing the amino sugar-containing glucan, a hydroxyl group-modified product thereof, a reducing end-modified product thereof, an amino group-modified product thereof or a non-reducing end-modified product thereof (preferably, glucosamine-containing glucan, a hydroxyl group-modified product thereof, a reducing end-modified product thereof, an amino group-modified product thereof or a non-reducing end-modified product thereof) of the present invention.

According to a particular embodiment of the present invention, a nanoparticulate carrier for a DDS, containing the amino sugar-containing glucan, a hydroxyl group-modified product thereof, a reducing end-modified product thereof, or an amino group-modified product thereof of the present invention (preferably, an amino sugar-containing glucan, a hydroxyl group-modified product thereof, a reducing end-modified product thereof, or an amino group-modified product thereof) is provided. This nanoparticulate carrier for a DDS is preferably selected from the group consisting of a liposome, a virus particle, a macromolecule micelle and a nanogel composed of macromolecule bearing hydrophobic groups.

According to the present invention, the terminal structure of the glucan which can effectively form a non-covalently bound complex with a nucleic acid, a liposome, a virus particle, a macromolecule micelle, and a low-molecular weight compound can also be designed. As described above, the amino group-containing glucan, a hydroxyl group-modified product thereof, a reducing end-modified product thereof or an amino group-modified product thereof of the present invention can effectively form a complex with a protein, a nucleic acid, a low-molecular weight compound, or a nanoparticulate carrier for a DDS (for example, a liposome, a macromolecule micelle, and virus particles), and can influence on stability, physical properties, absorbability, pharmacokinetics (for example, organ accumulating property, tissue targeting property, or blood retention property) and the like of them. As described above, the amino group-containing glucan and a modified product thereof of the present invention can be effectively utilized as a DDS carrier of the medically effective ingredient of medicaments or as an agent for modifying a nanoparticulate carrier for a DDS.

Therefore, according to the present invention, a medicament containing the amino sugar- containing glucan or a modified product thereof of the present invention (preferably, glucosamine- containing glucan and a modified product thereof) and the medically effective ingredient is provided. In the medicament of the present invention, the medically effective ingredient is preferably selected from the group consisting of a low- molecular weight organic compound, a protein, a peptide, an antibody, an antibody fragment, a receptor, a receptor fragment, a DNA, an RNA, a siRNA, an miRNA and an RNA aptamer, as well as a modified product thereof.

(4. Amino sugar-containing glucan and modified product thereof and conjugate thereof of the present invention)

In the present specification, a glucan in which at least one amino monosaccharide residue is α- 1, 4- bound to each of at least one non- reducing end of a glucan, but no amino sugar residue is present at the positions other than the non- reducing end of the glucan is referred to as an "amino sugar- containing glucan". It is noted that when two or more amino monosaccharide are bound to one end of a glucan, then the structure is that wherein one amino monosaccharide is bound to one end of the glucan, and to the end of the amino monosaccharide, the next amino monosaccharide is bound. In the present specification, a glucan in which at least one glucosamine residue is α- 1, 4- bound to each of at least one non- reducing end of a glucan, but no glucosamine residue is present at the positions other than the non- reducing end is referred to as a "glucosamine- containing glucan".
In the present specification, even when a sugar chain consisting of two or more amino monosaccharide residues each bound via an α- 1, 4- bond is bound to a specific non- reducing end of a glucan and an amino sugar residue is not present at the position other than the non- reducing end of the glucan, it is considered that an amino sugar residue is bound only to a non- reducing end and an amino sugar residue is not present at the position other than the non- reducing end.

The amino sugar- containing glucan of the present invention is a amino sugar- containing glucan in which at least one amino monosaccharide residue is bound via an α- 1, 4- bond to each of at least one non- reducing end of a glucan, but no amino sugar residue is present at the positions other than the non- reducing end of the glucan, wherein the glucan is a branched α- 1, 4 glucan or a linear α- 1, 4 glucan.

The amino sugar- containing glucan of the present invention is preferably glucosamine- containing glucan, and more preferably a glucosamine- containing linear glucan or a glucosamine- containing branched glucan. When a glucan moiety of the amino sugar- containing glucan is a linear glucan, since the number of non- reducing end is one, at least one amino monosaccharide residue is bound via an α- 1, 4- bond to the non- reducing end. When a glucan moiety of the amino sugar- containing glucan is a branched glucan, since there are two or more non- reducing ends, at least one amino monosaccharide residue is bound via an α- 1, 4- bond to one or more non- reducing ends of them. In one embodiment, the glucosamine- containing branched glucan of the present invention is a glucosamine- containing branched glucan wherein the glucan has a plurality of non- reducing ends and at least one glucosamine residue is bound via an α- 1, 4- bond to one or more non- reducing ends of the branched glucan, but glucosamine residue is not present at the position other than the non- reducing end.

Absence of the glucosamine residue at the positions other than a non- reducing end in the amino sugar- containing glucan of the present invention can be confirmed, for example, by treating the amino sugar- containing glucan with α- amylase. α- Amylase acts on an α- 1, 4 glucan to generate maltose and glucose, however, it cannot degrade the glycosidic bond between the amino sugar and glucose in an amino- glycosilated α- 1, 4- glucan chain. In this reason, an amino sugar- containing glucan exhibits resistance to treatment with α- amylase. The glucan containing the amino sugar residue on a non- reducing end can be confirmed by the facts that it generates maltose and glucose by treatment with α- amylase, and that maltotriose containing an amino sugar is detected.

(4.1) Branched glucan in which amino sugar residue bound thereto, modified product thereof and conjugate thereof

When a glucan moiety contained in the amino sugar- containing glucan of the present invention is a branched α- 1, 4 glucan, in the amino sugar- containing glucan, at least one amino monosaccharide residue is α- 1, 4- bound to at least one of a plurality of non- reducing ends possessed by this branched α- 1, 4 glucan. According to the present invention, a modified product of the branched glucan with at least one amino monosaccharide residue α- 1, 4- bound to at least one non- reducing end is also provided. According to the present invention, a conjugate of the branched glucan with at least one amino monosaccharide residue α- 1, 4- bound to at least one non- reducing end or a modified product thereof is also provided. Modification of the modified product is as described above in detail. The substance to be bound in a conjugate is also as described above in detail.

A branched glucan moiety contained in the amino sugar-containing branched glucan, a modified product thereof and a conjugate thereof of the present invention is preferably selected from the group consisting of a starch, amylopectin, glycogen, dextrin, an enzymatically synthesized branched glucan and highly branched cyclic glucan. A preferable range of these branched glucan moieties is as explained in "(1.1) Glucans and modified products of glucan".

The molecular weight of the amino sugar-containing branched glucan of the present invention is preferably about $5 \times 10^3$ or more, more preferably about $1 \times 10^4$ or more, and further preferably about $5 \times 10^4$ or more . The molecular weight of the amino sugar-containing branched glucan of the present invention is preferably about $1 \times 10^9$ or less, more preferably about $3 \times 10^8$ or less, and further preferably about $1 \times 10^8$ or less.

The molecular weight of the modified product of the amino sugar-containing branched glucan of the present invention is preferably about $5 \times 10^3$ or more, more preferably about $1 \times 10^4$ or more, and further preferably about $5 \times 10^4$ or more. The molecular weight of the modified product of the amino sugar-containing branched glucan of the present invention is preferably about $1 \times 10^9$ or less, more preferably about $3 \times 10^8$ or less, and further preferably about $1 \times 10^8$ or less.

In the conjugate of the present invention, it is preferable that another substance (for example, targeting molecule, medically effective ingredient, or the like) is bound to the aforementioned amino sugar-containing branched glucan having a suitable molecular weight.

In the present invention, a modified product of the amino sugar-containing branched glucan is preferable. Modification is preferably acylation or etherification, and more preferably acetylation. The acylation degree is preferably

about 0.1 or more, further preferably about 0.2 or more, and particularly preferably about 0.3 or more. The acylation degree is calculated by quantification of acetic acid released by heating under an alkali condition. The etherification degree is preferably about 0.1 or more, further preferably about 0.2 or more, and particularly preferably about 0.3 or more. The etherification degree is calculated by NMR.

The number of amino monosaccharide residues bound to the amino sugar-containing branched glucan, a modified product thereof and a conjugate thereof of the present invention is preferably 1 or more, more preferably 2 or more, and further preferably 3 or more, per one molecule. The number of amino monosaccharide residues bound to the amino sugar-containing branched glucan or a modified product thereof of the present invention is not limited to them, and for example, may be 5 or more, 10 or more, 15 or more, 20 or more, 50 or more, or 100 or more, or the like, per one molecule. The number can be suitably adjusted according to the purpose. The upper limit of the number of amino monosaccharide residues bound to the amino sugar-containing branched glucan or a modified product thereof of the present invention is the number of non-reducing ends of the branched glucan moiety. The number of amino monosaccharide residues bound to the amino sugar-containing branched glucan or a modified product thereof of the present invention can be, for example, about 1,000 or less, about 800 or less, about 700 or less, about 600 or less, about 500 or less, about 400 or less, about 300 or less, about 200 or less, about 100 or less, about 50 or less, or the like, per one molecule.

In a particular embodiment, the number of amino monosaccharide residues bound to the amino sugar-containing branched glucan, a modified product thereof and a conjugate thereof of the present invention is preferably about 10% or more, more preferably about 20% or more, particularly preferably about 30% or more and, for example, can be about 40% or more, about 50% or more or about 60% or more of the number of non-reducing ends possessed by the branched glucan moiety. In a particular embodiment, the number of amino monosaccharide residues bound to the amino sugar-containing branched glucan, a modified product thereof and a conjugate thereof of the present invention is preferably about 100% or less, more preferably about 90% or less, particularly preferably about 80% or less and, for example, can be about 70% or less, about 60% or less, or about 50% or less of the number of non-reducing ends possessed by the branched glucan moiety.

(4.2) Linear glucan in which amino sugar residue bound thereto, modified product thereof and conjugate thereof

In the amino sugar- containing linear glucan of the present invention, at least one amino monosaccharide residue is α- 1, 4- bound to a non- reducing end of the linear glucan moiety. Since the linear glucan has only one non-reducing end, if the amount of amino monosaccharide added to the reaction solution at the reaction of amino monosaccharide and glucan is small, the amino sugar- containing linear glucan of the present invention contains only one amino monosaccharide residue. If the amount of amino monosaccharide added to the reaction solution at the reaction of amino monosaccharide and glucan is large, two or more amino monosaccharide bound via an α- 1, 4- bond can bind to the non- reducing end of the linear glucan. According to the preset invention, there is also provided a modified product of the linear glucan with at least one amino monosaccharide residue α- 1, 4- bound thereto. According to the present invention, there is also provided a conjugate of the linear glucan with at least one amino monosaccharide residue α- 1, 4- bound thereto or a modified product thereof. Modification of the modified product is as described above in detail. The substance bound to the amino sugar- containing glucan or a modified product thereof in a conjugate is as described above in detail.

The linear glucan moiety contained in the amino sugar-containing linear glucan or a modified product thereof of the present invention is preferably selected from the group consisting of amylose (for example, natural amylose or enzymatically synthesized amylose) and a derivative thereof. A preferable range of these linear glucan moieties is as explained in "(1.1) Glucans and modified products of glucan".

The molecular weight of the amino sugar-containing linear glucan of the present invention is for example about $1 \times 10^3$ or more, preferably about $1.5 \times 10^3$ or more, more preferably about $2 \times 10^3$ or more, and further preferably about $3 \times 10^3$ or more. The molecular weight of the amino sugar-containing linear glucan of the present invention is for example about $1 \times 10^7$ or less, preferably about $2 \times 10^5$ or less, more preferably about $1.5 \times 10^5$ or less, and further preferably about $1 \times 10^5$ or less.

The molecular weight of the modified product of the amino sugar-containing linear glucan of the present invention is for example about $1 \times 10^3$ or more, preferably about $1.5 \times 10^3$ or more, more preferably about $2 \times 10^3$ or more, and further preferably about $3 \times 10^3$ or more . The molecular weight of the modified product of the amino sugar-containing linear glucan of the present invention is for example about $1 \times 10^7$ or less, preferably about $2 \times 10^5$ or less, more preferably about $1.5 \times 10^5$ or less, and further preferably about $1 \times 10^5$ or less.

In the conjugate of the present invention, it is preferable that another substance (for example, targeting molecule, medically effective ingredient, or the like) is bound to the amino sugar-containing linear glucan or a modified product thereof having such a suitable molecular weight as mentioned before.

In the present invention, a modified product of the amino sugar-containing linear glucan is preferable. Modification is preferably acylation or etherification, more preferably acetylation. The acylation degree is preferably about 0.1 or more, further preferably about 0.2 or more, and particularly preferably about 0.3 or more. The acylation degree is calculated by quantification of acetic acid released by heating under an alkali condition. The etherification degree is preferably about 0.1 or more, further preferably about 0.2 or more, and particularly preferably about 0.3 or more. The etherification degree is calculated by NMR.

Examples of the glucosamine-containing linear glucan of the present invention can be represented, for example, by the structure of the following formula 1:

[Chemical formula 1]

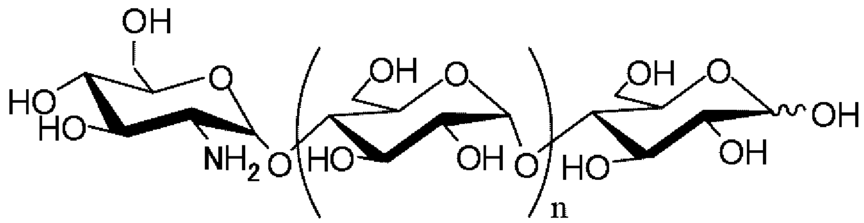

Formula 1

wherein n is an integer of 1 or more. n is preferably about 8 or more, more preferably about 9 or more, and further preferably about 10 or more. n is preferably about 1200 or less, more preferably about 900 or less, and further preferably about 600 or less.

Examples of the glucosamine-containing linear glucan and the reducing end-modified product thereof of the present invention can be represented, for example, by the following formula 2:

[Chemical formula 2]

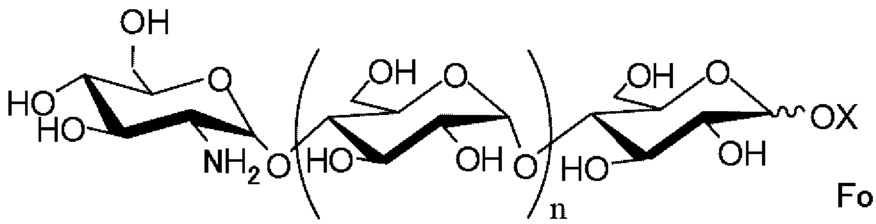

Formula 2

wherein n is an integer of 1 or more. n is preferably about 8 or more, more preferably about 9 or more, and further preferably about 10 or more. n is preferably about 1200 or less, more preferably about 900 or less, and further preferably about 600 or less. X is selected from the group consisting of hydrogen, monosaccharides, non-reducing carbohydrates, biocompatible macromolecules, liposome constituent components, glycosides, and amine group-containing low-molecular weight substances. X is preferably selected from the group consisting of hydrogen, glucosamine, N-acetylglucosamine, gluconic acid, sorbitol, sucrose, trehalose, dextrin, amylose, starches, cellulose, chitin, chitosan, dextran, proteins, peptides, phospholipids, fatty acids, surfactants, ascorbic acid glucosides, hydroquinone glucosides, hesperidin glucosides, rutin glucosides, para-nitrophenyl maltopentaose, dodecylmaltose, flavonoid glycosides, terpene glycosides, phenol glycosides, chalcone glycosides, steroid glycosides, amino acids and dodecylamine. When X is hydrogen, this molecule is a glucosamine-containing linear glucan; when X is a substance other than hydrogen, this molecule is a reducing end-modified product of a glucosamine-containing linear glucan.

Examples of the glucosamine-containing linear glucan, hydroxyl group-modified product of a glucosamine-containing linear glucan, and the reducing end-modified product thereof of the present invention can be represented, for example, by the following formula 3:

[Chemical formula 3]

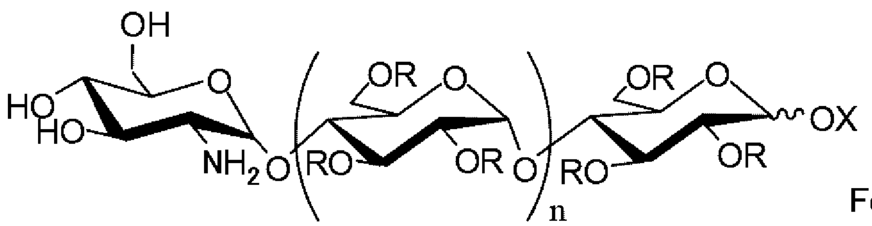

Formula 3

wherein n is an integer of 1 or more. n is preferably about 8 or more, more preferably about 9 or more, and further preferably about 10 or more. n is preferably about 1200 or less, more preferably about 900 or less, and further preferably about 600 or less. X is preferably selected from the group consisting of hydrogen, monosaccharides, non-reducing carbohydrates, biocompatible macromolecules, liposome constituent components, glycosides, and amine group-containing low-molecular weight substances. X is preferably selected from the group consisting of hydrogen, glucosamine, N-acetylglucosamine, gluconic acid, sorbitol, sucrose, trehalose, dextrin, amylose, cellulose, chitin, chitosan, dextran, proteins, peptides, phospholipids, fatty acids, surfactants, ascorbic acid glucosides, hydroquinone glucosides, hesperidin glucosides, rutin glucosides, para-nitrophenyl maltopentaose, dodecylmaltose, flavonoid glycosides, terpene glycosides, phenol glycosides, chalcone glycosides, steroid glycosides, amino acids and dodecylamine. Wherein R is preferably independently selected from the group consisting of hydrogen, hydroxyalkyl group, an alkyl group, an acetyl group, a carboxymethyl group, a sulfuric acid group and a phosphoric acid group. When X and all of R are hydrogen, this molecule is a glucosamine-containing linear glucan; when X is hydrogen, and each R is independently hydrogen or another group, provided that at least one of R is a group other than hydrogen, this molecule is a hydroxyl group-modified product of a glucosamine-containing linear glucan; when X is a substance other than hydrogen, and all of R is hydrogen, this molecule is a reducing end-modified product of a glucosamine-containing linear glucan; when X is a substance other than hydrogen, and each R is independently hydrogen or another group, provided that at least one of R is a group other than hydrogen, this molecule is a reducing end-modified product of a hydroxyl group-modified product of a glucosamine-containing linear glucan.

Examples of the glucosamine-containing linear glucan, hydroxyl group-modified product of a glucosamine-containing linear glucan, the reducing end-modified product thereof and the amino group-modified product thereof of the present invention can be represented, for example, by the following formula 4:

[Chemical formula 4]

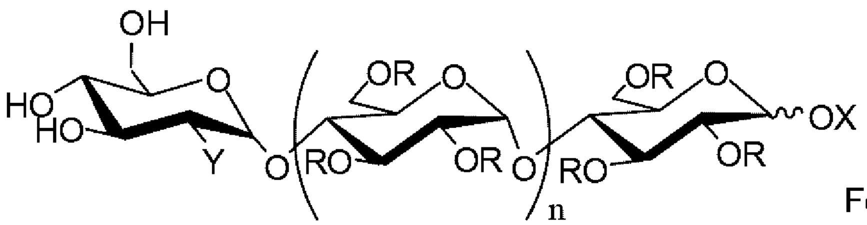

Formula 4

Wherein n is an integer of 1 or more. n is preferably about 8 or more, more preferably about 9 or more, and further preferably about 10 or more. n is preferably about 1200 or less, more preferably about 900 or less, and further preferably about 600 or less. X is selected from the group consisting of hydrogen, monosaccharides, non-reducing carbohydrates, biocompatible macromolecules, liposome constituent components, glycosides, and amine group-containing low-molecular weight substances. X is preferably selected from the group consisting of hydrogen, glucosamine, N-acetylglucosamine, gluconic acid, sorbitol, sucrose, trehalose, dextrin, amylose, starches, cellulose, chitin, chitosan, dextran, proteins, peptides, phospholipids, fatty acids, surfactants, ascorbic acid glucosides, hydroquinone glucosides, hesperidin glucosides, rutin glucosides, para-nitrophenyl maltopentaose, dodecylmaltose, flavonoid glycosides, terpene glycosides, phenol glycosides, chalcone glycosides, steroid glycosides, amino acids and dodecylamine. Wherein R is preferably independently selected from the group consisting of hydrogen, hydroxyalkyl group, an alkyl group, an acetyl group, a carboxymethyl group, a sulfuric acid group and a phosphoric acid group. Wherein, Y is preferably selected from the group consisting of amino group, an anionic substituent, a hydrophobic substituent, a maleimide group-containing substituent and a succinimide group-containingsubstituent. When X and all of R are hydrogen and Y is amino group, this molecule is a glucosamine-containing linear glucan; when X and all of R are hydrogen and Y is a group other than amino group (that is, an anionic substituent, a hydrophobic substituent, a maleimide group-containing substituent or a succinimide group-containing substituent), this molecule is an amino group-modified product of glucosamine-containing linear glucan; when X is hydrogen and each R is independently hydrogen or another group, provided that at least one of R is a group other than hydrogen and Y is amino group, this molecule is a hydroxyl group-modified product of a glucosamine-containing linear glucan; when X is hydrogen and each R is independently hydrogen or another group, provided that at least one of R is a group other than hydrogen and Y is a group other than amino group (that is, a cationic substituent, a hydrophobic substituent, a maleimide group-containing substituent or a succinimide group-containing substituent), this molecule is an amino group-modified product of a hydroxyl group-modified product of a glucosamine-containing linear glucan; when X is a substance other than hydrogen, all of R is hydrogen, and Y is amino group, this molecule is a reducing end-modified product of a glucosamine-containing linear glucan; when X is a substance other than hydrogen, all of R is hydrogen, and Y is group other than amino group (that is, an anionic substituent, a hydrophobic substituent, or a maleimide group-containing substituent), this molecule is a reducing end-modified product of an amino

group-modified product of a glucosamine-containing linear glucan; when X is a substance other than hydrogen, each R is independently hydrogen or another group, provided that at least one of R is a group other than hydrogen and Y is amino group, this molecule is a reducing end-modified product of a hydroxyl group-modified product of a glucosamine-containing linear glucan; and when X is a substance other than hydrogen, each R is independently hydrogen or another group, provided that at least one of R is a group other than hydrogen and Y is a group other than amino group (that is, an anionic substituent, a hydrophobic substituent, or a maleimide group-containing substituent), this molecule is an amino group-modified product of hydroxyl group-modified product a glucosamine-containing linear glucan.

Examples of the glucosamine-containing glucan of the present invention can be represented, for example, by the following formula 5:

[Chemical formula 5]

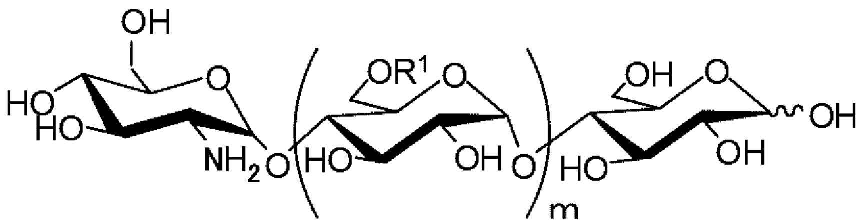

Formula 5

wherein m is an integer of 1 or more. m is preferably 8 or more, more preferably 9 or more, and further preferably 10 or more, and m is preferably about 1 x $10^4$ or less, more preferably about 1000 or less, and further preferably about 700 or less. $R^1$ is independently H, a glucan chain having the structure of formula A or a glucan chain having the structure of formula B. When all of $R^1$ is H, the glucan shown by formula 5 is a linear glucan. When at least one of $R^1$ has the structure of formula A or formula B, the glucan shown by formula 5 is a branched glucan.

[Chemical formula 6]

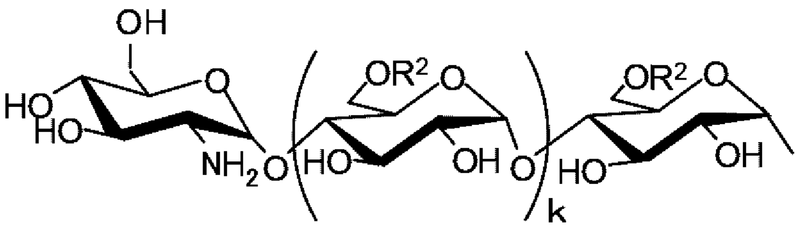

Formula A

in formula A, k is an integer of 1 or more. k is preferably 8 or more, more preferably 9 or more, and further preferably 10 or more, and k is preferably about 1 x $10^4$ or less, more preferably about 1000 or less, and further preferably about 700 or less. In formula A, each $R^2$ is independently H, a glucan chain having the structure of formula A or a glucan chain having the structure of formula B.

[Chemical formula 7]

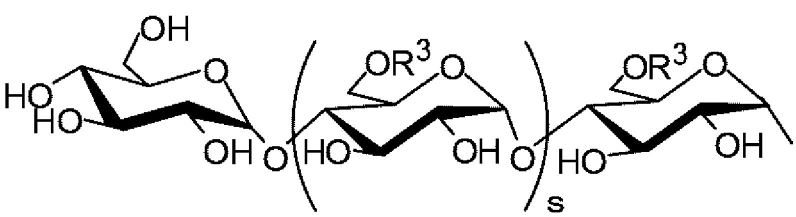

Formula B

In formula B, s is an integer of 1 or more. s is preferably 8 or more, more preferably 9 or more, and further preferably 10 or more, and s is preferably about 1 x $10^4$ or less, more preferably about 1000 or less, and further preferably about 700 or less. In formula B, $R^3$ is independently H, a glucan chain having the structure of formula A or a glucan chain having the structure of formula B.

As explained above, in formula 5, $R^1$ can have a structure in which the position of $R^2$ of a group having the structure of formula A or $R^3$ of a group having the structure of formula B is substituted with a group having the structure of formula A or a group having the structure of formula B several times. The total of times of substitutions with formula A and formula B is equal to the number of unit chains of the branched glucan molecule represented by formula 5. The

number of unit chain of branched glucan molecule is preferably 1 or more, more preferably 2 or more, and further more preferably 3 or more. The number of unit chain of branched glucan molecule is preferably about 5 x $10^3$ or less, more preferably about 2 x $10^3$ or less, and further more preferably about 1 x $10^3$ or less.

Examples of the glucosamine-containing glucan or the hydroxyl group-modified product of a glucosamine-containing glucan of the present invention can be represented, for example, by the following formula 6:

[Chemical formula 8]

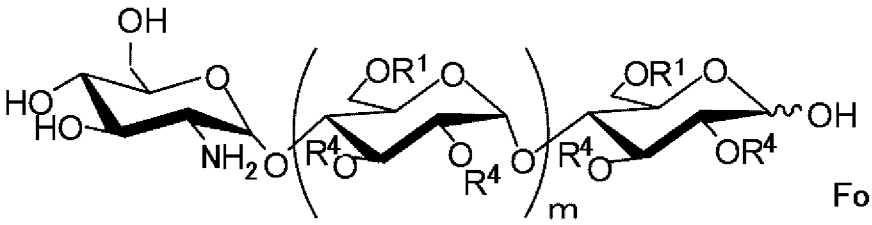

Formula 6

wherein m is an integer of 1 or more. m is preferably 8 or more, more preferably 9 or more, and further preferably 10 or more, and m is preferably about 1 x $10^4$ or less, more preferably about 1000 or less, and further preferably about 700 or less. $R^1$ is independently H, a hydroxyalkyl group, an alkyl group, an acetyl group, a carboxymethyl group, a sulfuric acid group, a phosphoric acid group, a glucan chain having the structure of formula 6A or a glucan chain having the structure of formula 6B.

[Chemical formula 9]

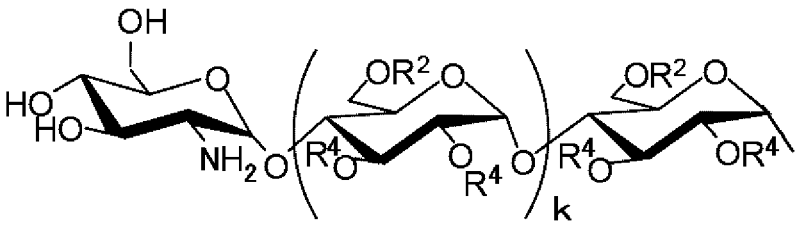

Formula 6A

in formula 6A, k is an integer of 1 or more. k is preferably 8 or more, more preferably 9 or more, and further preferably 10 or more, and k is preferably about 1 x $10^4$ or less, more preferably about 1000 or less, and further preferably about 700 or less. In formula 6A, each $R^2$ is independently H, a hydroxyalkyl group, an alkyl group, an acetyl group, a carboxymethyl group, a sulfuric acid group, a phosphoric acid group, a glucan chain having the structure of formula 6A or a glucan chain having the structure of formula 6B.

[Chemical formula 10]

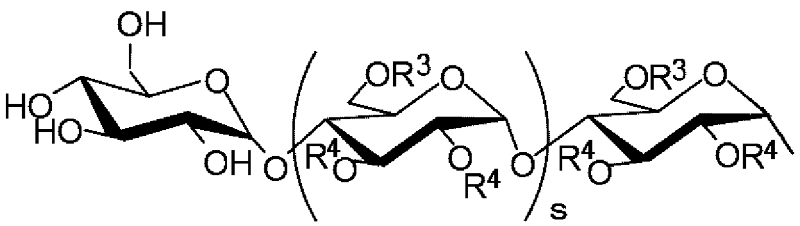

Formula 6B

in formula 6B, s is an integer of 1 or more. s is preferably 8 or more, more preferably 9 or more, and further preferably 10 or more, and s is preferably about 1 x $10^4$ or less, more preferably about 1000 or less, and further preferably about 700 or less. In formula 6B, each $R^3$ is independently H, a hydroxyalkyl group, an alkyl group, an acetyl group, a carboxymethyl group, a sulfuric acid group and a phosphoric acid group, a glucan chain having the structure of formula 6A or a glucan chain having the structure of formula 6B.

In formula 6, formula 6A and formula 6B, each $R^4$ is independently selected from the group consisting of H, a hydroxyalkyl group, an alkyl group, an acetyl group, a carboxymethyl group, a sulfuric acid group and a phosphoric acid group.

Examples of the glucosamine-containing glucan, a hydroxyl group-modified product of a glucosamine-containing glucan and a reducing end-modified product thereof of the present invention can be represented, for example, by

the following formula 7:

[Chemical formula 11]

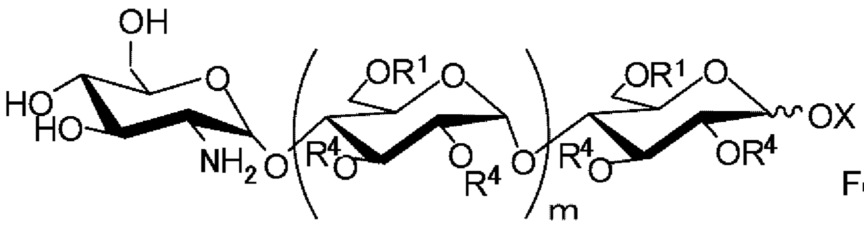

Formula 7

wherein m is an integer of 1 or more. m is preferably 8 or more, more preferably 9 or more, and further preferably 10 or more, and m is preferably about 1 x $10^4$ or less, more preferably about 1000 or less, and further preferably about 700 or less. $R^1$ is independently H, a glucan chain having the structure of formula 6A or a glucan chain having the structure of formula 6B. Formula 6A and formula 6B are same with the definition for the aforementioned formula 6.

In formula 7, formula 6A and formula 6B, $R^4$ is independently selected from the group consisting of hydrogen, a hydroxyalkyl group, an alkyl group, an acetyl group, a carboxymethyl group, a sulfuric acid group and a phosphoric acid group,
in formula 7, X is preferably independently selected from the group consisting of monosaccharides, non-reducing carbohydrates, biocompatible macromolecules, liposome constituent components, glycosides, and amine group-containing low-molecular weight substances. X is more preferably selected from the group consisting of hydrogen, glucosamine, N-acetylglucosamine, gluconic acid, sorbitol, sucrose, trehalose, dextrin, amylose, starches, cellulose, chitin, chitosan, dextran, proteins, peptides, phospholipids, fatty acids, surfactants, ascorbic acid glucosides, hydroquinone glucosides, hesperidin glucosides, rutin glucosides, para-nitrophenyl maltopentaose, dodecylmaltose, flavonoid glycosides, terpene glycosides, phenol glycosides, chalcone glycosides, steroid glycosides, amino acids and dodecylamine.

Examples of the glucosamine-containing glucan, a hydroxyl group-modified product of a glucosamine-containing glucan, a reducing end-modified product thereof, or an amino group-modified product thereof of the present invention can be represented, for example, by the following formula 8:

[Chemical formula 12]

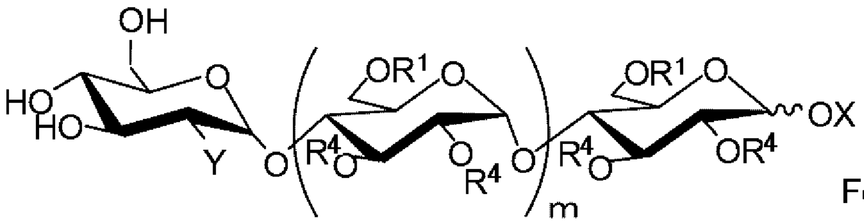

Formula 8

wherein m is an integer of 1 or more. m is preferably 8 or more, more preferably 9 or more, and further preferably 10 or more, and m is preferably about 1 x $10^4$ or less, more preferably about 1000 or less, and further preferably about 700 or less. $R^1$ is independently H, a hydroxyalkyl group, an alkyl group, an acetyl group, a carboxymethyl group, a sulfuric acid group, a phosphoric acid group, a glucan chain having the structure of formula 6A, a glucan chain having the structure of formula 8A or a glucan chain having the structure of formula 6B.

[Chemical formula 13]

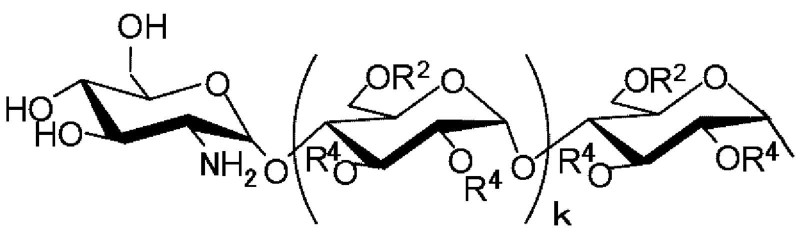

Formula 6A

in formula 6A, k is an integer of 1 or more. k is preferably 8 or more, more preferably 9 or more, and further preferably 10 or more, and k is preferably about 1 x $10^4$ or less, more preferably about 1000 or less, and further preferably

about 700 or less. In formula 6A, each $R^2$ is independently H, a hydroxyalkyl group, an alkyl group, an acetyl group, a carboxymethyl group, a sulfuric acid group, a phosphoric acid group, a glucan chain having the structure of formula 6A, a glucan chain having the structure of formula 8A or a glucan chain having the structure of formula 6B.

[Chemical formula 14]

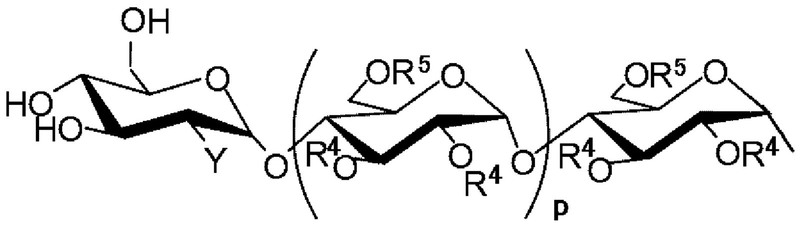

Formula 8A

in formula 8A, p is an integer of 1 or more. p is preferably 8 or more, more preferably 9 or more, and further preferably 10 or more, and p is preferably about $1 \times 10^4$ or less, more preferably about 1000 or less, and further preferably about 700 or less. In formula 8A, $R^5$ is independently H, a hydroxyalkyl group, an alkyl group, an acetyl group, a carboxymethyl group, a sulfuric acid group, a phosphoric acid group, a glucan chain having the structure of formula 6A, a glucan chain having the structure of formula 8A, or a glucan chain having the structure of formula 6B:

[Chemical formula 15]

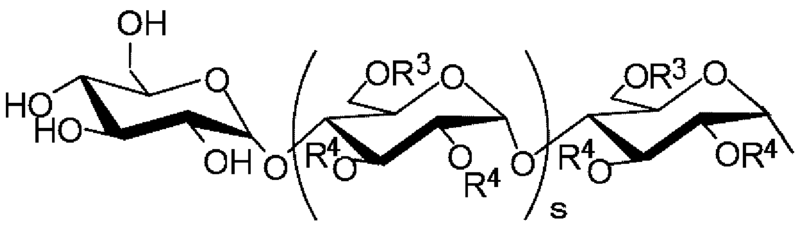

Formula 6B

in formula 6B, s is an integer of 1 or more. s is preferably 8 or more, more preferably 9 or more, and further preferably 10 or more, and s is preferably about $1 \times 10^4$ or less, more preferably about 1000 or less, and further preferably about 700 or less. In formula 6B, each $R^3$ is independently H, a hydroxyalkyl group, an alkyl group, an acetyl group, a carboxymethyl group, a sulfuric acid group, a phosphoric acid group, a glucan chain having the structure of formula 6A, a glucan chain having the structure of formula 8A, or a glucan chain having the structure of formula 6B.

In formula 8, formula 6A, formula 8A and formula 6B, $R^4$ is independently selected from the group consisting of hydrogen, a hydroxyalkyl group, an alkyl group, an acetyl group, a carboxymethyl group, a sulfuric acid group and a phosphoric acid group,

in formula 8, X is independently selected from the group consisting of monosaccharides, non-reducing carbohydrates, biocompatible macromolecules, liposome constituent components, glycosides, and amine group-containing low-molecular weight substances,

in formula 8, Y is a substituent introduced for binding with an amino group or a medically effective ingredient, preferably, selected from the group consisting of an amino group, an anionic substituent, a hydrophobic substituent, a maleimide group-containing substituent and a succinimide group-containing substituent, and in formula 8A, Y is a substituent introduced for binding with a medically effective ingredient, preferably, selected from the group consisting of an anionic substituent, a hydrophobic substituent, a maleimide group-containing substituent and a succinimide group-containing substituent, Y is obtained by a reaction with an amino group-modifying reagent, and the amino group-modifying reagent has at least one carboxyl group and at least one other functional group.

As explained above, in formula 8, $R^1$ can have a structure in which the position of $R^2$ of a group having the structure of formula 6A, or $R^3$ of a group having the structure of formula 6B, or $R^5$ of a group having the structure of formula 8A is substituted with a group having the structure of formula 6A, or a group having the structure of formula 6B, or a group having the structure of formula 8A several times. The total of times of substitutions with a group of formula 6A, a group of formula 6B or a group of formula 8A is equal to the number of unit chains of the branched glucan molecule represented by formula 8. The number of unit chain of branched glucan molecule is preferably about 2 or more, more preferably about 5 or more, and further more preferably about 10 or more. The number of unit chain of branched glucan molecule is preferably about 5, 000 or less, more preferably about 3, 000 or less, and further more preferably about 2,000 or less.

The glucosamine-containing glucan, a hydroxyl group-modified product of a glucosamine-containing glucan,

a reducing end-modified product thereof, or an amino group-modified product thereof of the present invention in a specific embodiment can have, for example, the structure of the following formula 9:

[Chemical formula 16]

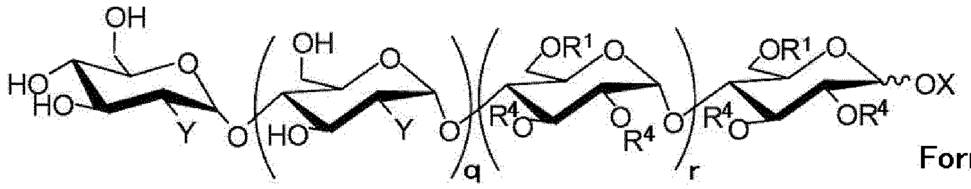

Formula 9

wherein q is 0 or an integer of 1 or more. In one embodiment, q can be, for example, 0, 1, 2, 3, 4, or 5. q is preferably 0, 1 or 2, and more preferably 0 or 1. r is an integer of 1 or more. r is preferably 8 or more, more preferably 9 or more, and further preferably 10 or more, and r is preferably about 1 x $10^4$ or less, more preferably about 1000 or less, further preferably about 700 or less. $R^1$ is independently H, a hydroxyalkyl group, an alkyl group, an acetyl group, a carboxymethyl group, a sulfuric acid group, a phosphoric acid group, a glucan chain having the structure of formula 9A, a glucan chain having the structure of formula 8A, or a glucan chain having the structure of formula 6B.

[Chemical formula 17]

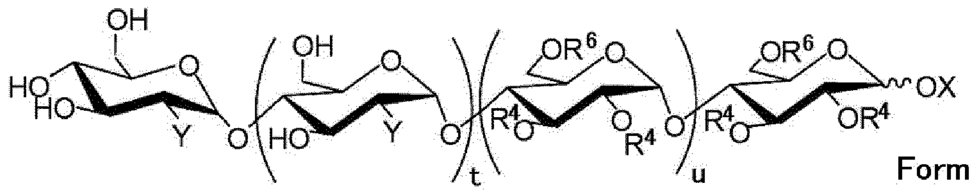

Formula 9A

In formula 9A, t is 0 or an integer of 1 or more. In one embodiment, t can be, for example, 0, 1, 2, 3, 4, or 5. t is preferably 0, 1 or 2, and more preferably 0 or 1. u is an integer of 1 or more. u is preferably 8 or more, more preferably 9 or more, and further preferably 10 or more, and u is preferably about 1 x $10^4$ or less, more preferably about 1000 or less, and further preferably about 700 or less. In formula 9A, $R^6$ is each independently, H, a hydroxyalkyl group, an alkyl group, an acetyl group, a carboxymethyl group, a sulfuric acid group, a phosphoric acid group, or a glucan chain having the structure of formula 9A.

In formula 9, X is independently selected from the group consisting of monosaccharides, non-reducing carbohydrates, biocompatible macromolecules, liposome constituent components, glycosides, and amine group-containing low-molecular weight substances.

In formula 9 and formula 9A, $R^4$ is independently selected from the group consisting of hydrogen, a hydroxyalkyl group, an alkyl group, an acetyl group, a carboxymethyl group, a sulfuric acid group and a phosphoric acid group,

In formula 9 and formula 9A, Y is a substituent introduced for binding with OH, $NH_2$ or a medically effective ingredient, provided that at least one of Y is a substituent introduced for binding with $NH_2$ or a medically effective ingredient. Y is preferably selected from the group consisting of amino group, an anionic substituent, a hydrophobic substituent, a maleimide group-containing substituent and a succinimide group-containing substituent. Y is obtained by a reaction with an amino group-modifying reagent, and the amino group-modifying reagent has at least one carboxyl group and at least one other functional group.

As explained above, in formula 9, $R^1$ can have a structure in which the position of $R^6$ of a group having the structure of formula 9A is substituted with a group having the structure of formula 9A several times. The total of times of substitutions with a group of formula 9A is equal to the number of unit chains of the branched glucan molecule represented by formula 9. The number of unit chain of branched glucan molecule is preferably about 2 or more, more preferably about 5 or more, and further more preferably about 10 or more. The number of unit chain of branched glucan molecule is preferably about 5, 000 or less, more preferably about 3, 000 or less, and further more preferably about 2, 000 or less.

Examples of modification of the glucosamine-containing glucan of the present invention and the purpose of the modification are summarized in the following Table 1. The structure in the table is merely an example.

[Table 1] Table 1: Examples of modification and application of glucosamine-containing glucan

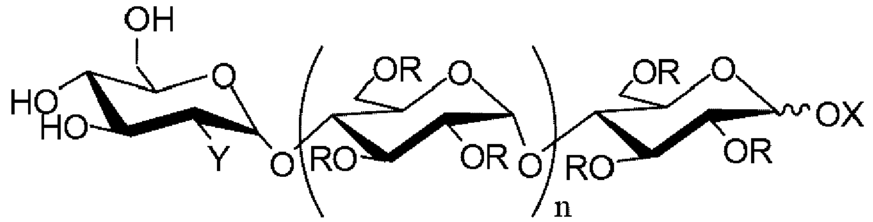 Formula 4

| Modification site | Specific example and structure | Application |
|---|---|---|
| Unmodified | (Y=$NH_2$, R=H, X=H)<br>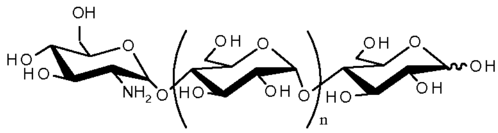 | Can be charged positively and form a complex with an anionic substance such as a nucleic acid. |
| Hydroxyl group ( R ) | Substitution on acetyl group (Y=$NH_2$, R=Ac, X=H)<br>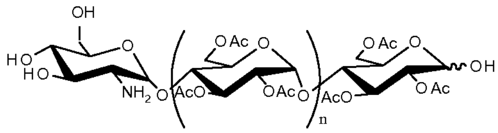 | Suppressing degradation with α-amylase, and improving blood retention property. |
| Amino group ( Y ) | Substitution on carboxyl group (Y=$NHCOCH_2COOH$, R=H, X=H)<br>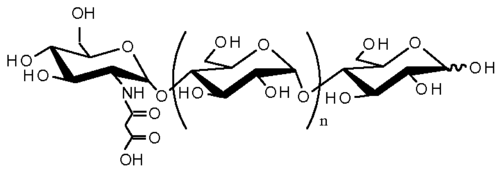 | Can impart a negative charge and form a complex with a cationic substance. |
| Amino group ( Y ) | Substitution on phenyl group (Y=$NHCOC_6H_5$, R=H, X=H)<br>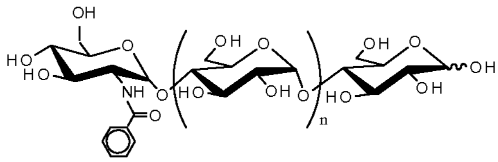 | Can impart a hydrophobic group and increase hydrophobicity. |
| Amino group ( Y ) | Substitution on maleimide group (Y= 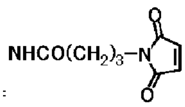 , R=H, X=H)<br>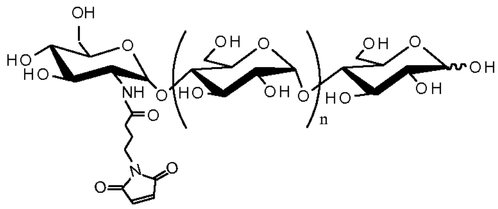 | Can synthesize a cross-linking reaction product of a maleimide group and a thiol group. |

In Table 1, the cases in which one glucosamine residue is bound to the non-reducing end are exemplified. As explained in other part in the present specification, an oligoglucosamine residue wherein two or more glucosamine residues are 1,4-bound may be bound to the non-reducing end. Further, a glucose residue, a mannose residue, a galactose residue, or an oligosaccharide residue constructed there from or the like may be bound to the 4-position of the glucosamine residue.

In one embodiment, a composition for binding with medicament comprising glucosamine-containing glucan, a hydroxyl group-modified product of glucosamine-containing glucan, a reducing end-modified product thereof, an amino group-modified product thereof, or a non-reducing end-modified product thereof of the present invention is provided. In the composition for binding with medicament of the present invention, the purity of these substances are very high, and the substances used in synthesizing these substances are hardly comprised. For example, the content of glucosamine-1-phosphate in the composition for binding with medicament of the present invention is about 10% by weight or less, more preferably about 1% by weight or less, further preferably about 0.1% by weight or less. Similarly for other synthetic materials, it is preferable that each content is preferably about 10% by weight or less, more preferably about 1% by weight or less, and most preferably about 0.1% by weight or less.

(Production of medicament)

When the glucosamine-containing branched glucan, the hydroxyl group-modified product, the reducing end-modified product, the amino group-modified product or the non-reducing end-modified product of the present invention does not comprise a drug, they can preferably be utilized as a carrier for a DDS. For example, by mixing the glucosamine-containing branched glucan, the hydroxyl group-modified product, the reducing end-modified product and the amino group-modified product of the present invention (they are collectively referred to as the carrier of the present invention) and a negatively-charged drug in an aqueous solution, the carrier of the present invention and the negatively-charged drug are electrostatically bound to form a complex. The complex can be utilized as a medicament whose molecular weight is suitably adjusted. Since the complex is constructed from two components, a carrier and a negatively-charged drug, the complex can also be referred to as a "two-component complex". As used in the present specification, the term "two-component complex" refers to, in a broad sense, a complex of the first substance or agent and the second substance or agent which specifically interacts with the first substance or agent.

When the substance bound in the conjugate of the present invention is a drug, the conjugate of the present invention can preferably used as a medicine.

(A complex of an amino sugar-containing glucan, a modified product thereof or a conjugate thereof and a nucleic acid molecule)

The amino sugar-containing glucan of the present invention forms a complex with a nucleic acid molecule (polynucleotide) in a solution. The bond between an amino sugar-containing glucan and a nucleic acid molecule in the complex is appropriately strong, and the strength of the bond can be adjusted by controlling the proportion of introduction of amino sugar, therefore, the complex can suitably be used for introducing a nucleic acid molecule into a cell. Preferably, the complex of the present invention is a complex formed with a nucleic acid molecule comprising a gene which can be expressed in the objective cell and the amino sugar-containing branched glucan of the present invention. More preferably, the complex of the present invention is a complex formed with a nucleic acid molecule comprising a gene which can be expressed in the objective cell and the glucosamine-containing branched glucan of the present invention. The gene can be a gene intended to be introduced and expressed at an appropriate place (for example, in a nucleus, in a mitochondrion, in cytoplasm or the like) in the objective cell. Preferably, the nucleic acidmolecule can be selected from the group consisting of a DNA, an RNA, a siRNA, an miRNA, and an RNA aptamer.

To the complex of the amino sugar- containing glucan of the present invention and a nucleic acid molecule, a cationic polymer or a cationic lipid can be further bound non- covalently to form a further complex.

Therefore, in a specific embodiment, the complex of the present invention is a complex formed with a complex carrier of the amino sugar- containing glucan of the present invention and a nucleic acid molecule, and a cationic polymer or a cationic lipid. The cationic polymer can comprise at least one kind of cationic polymer selected from the group consisting of polyethyleneimine, polylysine, polyarginine, polyamidoamine dendrimer, poly (aminostyrene) , chitosan, a cationic glucan and DEAE- dextran. Since the complex is constructed from three components, a carrier, a nucleic acid molecule and a cationic polymer or a cationic lipid, the complex can also be referred to as "three- component complex".

As used in the present specification, the term "three- component complex" refers to, in a broad sense, a complex of the first substance or agent, the second substance or agent which specifically interacts with the first substance or agent, and the third substance or agent which specifically interacts with either the first substance or agent or the second substance or agent. Such a three- component complex include, but not limited to, a complex of the carrier of the present invention, a negatively- charged drug (for example, a nucleic acid molecule) , and a positively- charged substance (for example, polycation polymer) .

(Method for delivering a gene)

The complex of the present invention can be used usefully for delivering a gene. The method for delivering a nucleic acid molecule of the present invention is a method for delivering a nucleic acid molecule into a cell comprising contacting a complex of the above-mentioned amino sugar-containing glucan of the present invention and a nucleic acid molecule, or a complex formed with a complex carrier of the amino sugar-containing glucan and the nucleic acid molecule, and a cationic polymer or a cationic lipid, with the cell. The complex and the cell are contacted *in vitro* or *in vivo,* and preferably contacted *in vitro.*

"Contact (ing)" as used in the present specification means that a cell is, either directly or indirectly, brought physically adjacent to the complex of the present invention. The complex can be present in a buffer, a salt, a solution or the like. Contacting *in vitro* can be conducted by placing the cell in, for example, a beaker, a microtiter plate, a cell-culturing flask or a microarray (for example, gene chip), containing the complex.

A "complex" in the present specification refers to a molecule made by non-covalently binding a plurality kinds of molecules such as a sugar, a polypeptide, a polynucleotide, a lipid, or a small molecule. Examples of such a complex include, but are not limited to, for example, a complex of an amino sugar-containing glucan and a polynucleotide; a complex of an amino sugar-containing glucan and a protein; a complex of an amino sugar-containing glucan, a nucleic acid molecule, and a cationic polymer or a cationic lipid; and the like.

*In vitro* and *in vivo* deliveries (transfection) of a polynucleotide to inside of a cell can be conducted by contacting a three-component complex with the cell to be transfected. Preferably, the cell is a mammalian cell. Contacting of a three-component complex and a cell *in vitro* can be conducted with various methods known to those skilled in the art, for example, as explained in the non-limiting Examples described below. Contacting of a three-component complex and a cell *in vivo* can be conducted by, preferably, introducing the complex into a body of an animal (preferably a mammal) by, for example, systemic administration or local administration. Preferably, administration is conducted by administrating to a mammal (for example, a human) having a cell requiring transfection with a specific polynucleotide, a three-component complex comprising the polynucleotide in an effective amount to deliver the desired amount of polynucleotide to the cell. The amount of the three-component complex effective to deliver such a desired polynucleotide can be determined by a routine *in vitro* experiment, and/or other methods known to those skilled in the art such as clinical trial.

(Method of therapy)

The complex of the present invention can be used usefully in gene therapy, drug therapy or the like. In a specific embodiment of gene therapy, method of therapy of the present invention is a method comprising administering a complex of the amino sugar-containing glucan of the present invention and a nucleic acid molecule, or a complex formed with a complex carrier of the amino sugar-containing glucan and a nucleic acid molecule, and a cationic polymer or a cationic lipid to a subject, wherein the nucleic acid molecule comprises a gene for therapy. In a specific embodiment of drug therapy, method of therapy of the present invention comprises administering a complex of the amino sugar-containing glucan of the present invention and a medically effective ingredient to a subject.

A nucleic acid molecule comprising a gene encoding a protein for therapy suitable for the therapy of the objective disease can also be produced using conventional genetic engineering techniques. The nucleic acid molecule may be an expression vector or a plasmid. For an expression vector, a plasmid and elements included therein, refer to the general explanation.

Examples

The present invention will be explained below based on examples, but the present invention is not limited to the examples. α-Glucan phosphorylase derived from *Aquifex aeolicus* VF5 used in the examples is *Aquifex aeolicus* VF5-derived α-glucan phosphorylase prepared by the following Production Example 1.

(Production Example 1: Preparation of *Aquifex aeolicus* VF5-derived α-glucan phosphorylase)

*Aquifex aeolicus* VF5-derived α-glucan phosphorylase was recombination-produced by the following method.

(A) Making of *Aquifex aeolicus* VF5-derived α-glucan phosphorylase gene

A nucleic acid (also referred to as an "α-glucan phosphorylase gene") having a base sequence (base sequence of 491380$^{th}$ to 493458$^{th}$ of ACCESSION No. AE000657 of GenBank base sequence database) encoding the amino acid sequence for *Aquifex aeolicus* VF5-derived α-glucan phosphorylase gene (the amino acid sequence described in SEQ ID NO : 2 of Sequence Listing; the amino acid sequence obtained by translating the base sequence of 491380$^{th}$ to 493458$^{th}$ of ACCESSION No. AE000657 of GenBank base sequence database of National Center for Biotechnology Information (NCBI) in the USA) was chemically synthesized by a method well-known to those skilled in the art. An NdeI site was created upstream of a translation initiation codon of this α-glucan phosphorylase gene. In addition, a BamHI site was created downstream of a translation stop codon, and this synthetic gene was cut with NdeI and BamHI, and inserted into plasmid pET11c (manufactured by Novagen) which had been previously cut with NdeI and BamHI to make a plasmid pET-AqGP having an *Aquifex aeolicus* VF5-derived α-glucan phosphorylase gene.

(B) Expression of *Aquifex aeolicus* VF5-derived α-glucan phosphorylase gene in *Escherichia coli*

*Escherichia coli* BL21 (DE3) was transformed with this plasmid pET-AqGP according to a conventional method to obtain a transformant. A liquid containing the transformant was diluted and applied on an ampicillin-containing LB agar medium (100 μg/ml ampicillin, 1% tryptone manufactured by Difco, 0.5% yeast extract manufactured by Difco, 0.5% NaCl, 1.5% agar, pH 7.3) so that independent colonies were obtained, and this was cultured at 37°C overnight. *Escherichia coli* grown on this ampicillin-containing LB agar medium is a transformant which harbors an introduced plasmid, and can express the introduced plasmid. In such a way, *Escherichia coli* expressing an α-glucan phosphorylase gene was successfully made.

(C) Preparation of *Aquifex aeolicus* VF5-derived α-glucan phosphorylase enzyme

*Escherichia coli* expressing the *Aquifex aeolicus* VF5- derived α- glucan phosphorylase gene, made in the aforementioned (B) , was inoculated with a LB medium (50 μg/ml ampicillin, 1% tryptone manufactured by Difco, 0.5% yeast extract manufacture by Difco, 0.5% NaCl, pH 7.3) , and cultured at 37°C for 5 hours. Then, IPTG (isopropyl- β- D- thiogalactopyranoside) and pyridoxine hydrochloride were added to this culture so that the final concentration became 0.1 mM IPTG and 1 mM pyridoxine hydrochloride and, further, this was cultured at 37°C for 24 hours. Then, bacterial cells were collected by centrifugation of the culture, and washed with 20 mM citrate buffer (pH 6.7) to remove medium components. The bacterial cells after washing were suspended in 20 mM citrate buffer (pH 6.7) , crushed with a sonicator, and centrifuged, and the supernatant was used as a bacterial cell extract. The resulting bacterial cell extract was heated at 60°C for 30 minutes. Then, this bacterial cell extract was loaded on a Q- Sepharose FF column which had been previously equilibrated, and this was eluted at a concentration gradient of from 0.1 M to 0.3 M NaCl, in 20 mM citrate buffer (pH 6.7) , to collect a GP- purified enzyme- containing active fraction.

Using about 1 μg of the resulting purified enzyme-containing active fraction, native PAGE (Native polyacrylamide gel electrophoresis) was performed. As a result, in a fraction obtained from *Escherichia coli* expressing α-glucan phosphorylase, a single band was recognized at the position of the molecular weight of about 150 kDa and, at other places, no band was found. Since the molecular weight of the α-glucan phosphorylase derived from *Aquifex aeolicus* VF5 is predicted to be about 75 kDa as calculated from their amino acid sequence, as a result of this native PAGE, it is thought that this α-glucan phosphorylase takes a dimer structure. In this manner, it was demonstrated that the α-glucan phosphorylase derived from *Aquifex aeolicus* VF5 was homogeneously purified.

(Production Example2:Productionofbranched glucan (B))

50 g of a waxy corn starch (manufactured by SANWA CORNSTARCH CO., LTD) was suspended into 1,000 ml of 10 mM sodium phosphate buffer (pH 7.0), and the suspension was heated to about 100°C to gelatinize the waxy corn starch. 200,000 units of a highly thermostable branching enzyme prepared according to the method described in Example 1 of Japanese Laid-Open Publication No. 2000-316581 was added to the starch paste which had been cooled to about 70°C to prepare a reaction solution, and then which was allowed to react at 70°C for 16 hours. After the reaction solution was heated at 100°C for 20 minutes, the supernatant after centrifugation at 6,500 rpm for 10 minutes was filtered with a membrane having a pore diameter of 0.8 μm. Then, the filtrate was desalted using a gel filtration chromatography (AKTA purifier) system (column: HiPrep™ 26/10 Desalting manufactured by GE Health care) to remove low-molecular weight polysaccharides. 1, 000 ml of the filtrate was divided into 7.5 ml of aliquots, and applied to a gel filtration chromatography system, and elution fractions from 2.7 minutes to 3.7 minutes at a flow rate of 10 ml/min were fractionated, respectively. The elution fractions obtained from 1,000 ml of the filtrate were combined, the combined elution fractions was filtered with a membrane having a pore diameter of 0.2μ m, and then lyophilized to obtain about 35 g of a powder of a branched glucan (B). The weight averagemolecularweight of the branched glucan was investigated using a high performance liquid chromatography (HPLC) system (column: OHPAK SB-806MHQ, manufactured by SHOWA DENKO K.K.) equipped with a multiangle laser light scattering detector (DAWN DSP, manufactured by Wyatt Technology Corporation) and a differential refractometer (Shodex RI-71, manufactured by SHOWA DENKO K.K.). 20 mg of a powder of the branched glucan was dissolved in 10 ml of a 100 mM aqueous sodium nitrate solution, and the solution was filtered with a membrane having a pore diameter of 0.45 μm to obtain a filtrate. 100 μl of the resulting filtrate was injected into the aforementioned HPLC system. It was shown that the weight average molecular weight of the branched glucan (B) is about 140 K.

When isoamylase was allowed to act on the aforementioned branched glucan, and the reducing power was investigated by the modified Park-Johnson method (Hizukuri et al., Starch, Vol., 35, pp. 348-350, (1983)), it was shown that the unit chain length of branching was about 15 on average, and the number of branching was about 60 on average.

(Production Example 3: Production of branched glucan (P))

An aqueous sucrose solution was prepared by dissolving 150 g of sucrose in 1, 000 ml of distilled water, and filtering the solution with a membrane having a pore diameter of 0.2 μm. The aqueous sucrose solution (800 ml) , 20 ml of a 5% aqueous branched glucan (B) solution (prepared by filtering a 5% aqueous solution of the branched glucan (B) produced by the aforementioned Production Example 2 of branched glucan, with a membrane having a pore diameter of 0.2 μm) , 4 ml of a 1 M sodium phosphate buffer (pH 7.0) , 1, 800 U of recombinant *Streptococcus mutans* sucrose phosphorylase prepared by the method as described in Example 2.5 of International Publication WO 02/097107 pamphlet, 1, 200 U of α- glucan phosphorylase produced in Production Example 1 of the present application, and 600, 000 U of the highly thermostable branching enzyme prepared according to the method described in Example 1 of Japanese Laid-Open Publication No. 2000- 316581 used in Production Example 2 were mixed, and the liquid volume was adjusted to 1, 000 ml with distilled water, followed by allowing reaction at 55°C for 24 hours. After the reaction solution was heated at 100°C for 20 minutes and centrifuged at 6, 500 rpm for 20 minutes, the resulting supernatant was filtered with a membrane having a pore diameter of 0.8 μm to obtain filtrate. Further, the resulting filtrate was desalted using a gel filtration chromatography (AKTA purifier) system (column: HiPrep™ 26/10 Desalting, manufactured by GE Health care) to remove low- molecular weight polysaccharides. 1, 000 ml of the filtrate was divided into 7.5 ml of aliquots, and applied to a gel filtration chromatography system, and elution fractions of from 2.7 minutes to 3.7 minutes at a flow rate of 10 ml/min were fractionated, respectively. The elution fractions obtained from 1, 000 ml of the filtrate were combined and filtered with a membrane having a pore diameter of 0.2 μm, and then lyophilized to obtain about 40 g of the branched glucan (P) . When the weight average molecular weight of the glucan was similarly investigated as the branched glucan in Production Example 2, it was shown that the weight average molecular weight of the branched glucan (P) was about 4, 000 K (about 25, 000 in terms of the degree of polymerization) . In addition, as a result of measuring the reducing power similarly to Production Example 2, it was shown that the unit chain length of the branching of the obtained branched glucan (P) was about 15 on average, and the number of branching was about 1, 600 on average. Further, when the average particle size of the branched glucan (P) was measured with a concentrated system particle size analyzer (FPAR-1000, manufactured by Otsuka Electronics Co., Ltd.) , the average particle size was about 37 nm.

(Production Example 4: Production of low-molecular weight amylopectin)

100ml of 1% suspension of waxy corn starch in water was treated on a sonicator (Ultrasonic Homogenizer, UH-600S) for 10 minutes, then centrifuged at 10,000 rpm and collected the supernatant soluble fraction. The obtained soluble fraction was partially purified with a desalting column (HiPrep Desalting Column, GE Company) to obtain starch fractions, and lyophilized the starch fractions to obtain powders. When the obtained powders were analyzed on a size exclusion chromatography equipped with an angle light scattering detector, it was confirmed that low-molecular weight amylopectin having the weight average molecular weight of about 6,100 kDa was obtained. In addition, as a result of measuring the reducing power similarly to Production Example 2, it was shown that the unit chain length of the branching of the obtained low-molecular weight amylopectin was about 22 on average, and the number of branching was about 1,600 on average.

(Production Example 5: Production of acetylated branched glucan)

The branched glucan (B) obtained in Production Example 2 was dissolved in dimethyl sulfoxide to 1.8% (w/v), 0.2% sodium carbonate and 0.055 M vinyl acetate were added, and stirring was performed at 25°C for 1 hour. The resulting solution was diluted 2-fold with ultrapure water, and glucan fractions were collected on a gel filtration column (PD-10) to obtain acetylated branched glucan. For confirming a degree of acetylation, 200 μl of a 5 N aqueous sodium hydroxide solution was added to 300 μl of the collected fractions, this was heated at 55°C for 30 minutes to perform a deacetylation reaction. 300 μl of a 1 N Tris buffer (pH 7.0) was added to this reaction solution, and 200 μl of 5 N hydrochloric acid was further added to perform neutralization. Using the solution after neutralization, glucose was quantified by a phenol sulfuric acid method. In addition, free acetic acid in the solution after neutralization was quantified with a free acetic acid quantification kit. As a result of them, it was confirmed that acetylated branched glucans having a degree of acetylation of 0.43 was obtained.

(Production Example 6: Recombination production of recombinant potato α-glucan phosphorylase)

Type L potato α-glucan phosphorylase was recombination-produced by the following method shown in Japanese National Phase PCT Laid-Open Publication No. 2004-526463.

BamHI site was created at the upstream and the downstream of a potato α- glucan phosphorylase gene (Nakano et al., Journal of Biochemistry 106: 691- 695 (1989) ; SEQ ID NO: 3 which is a nucleic acid sequence encoding the

amino acid sequence of SEQ ID NO: 4) , this gene was cut with BamHI, and was incorporated into pET3d (manufactured by STRATAGENE) that had been previously cut with BamHI to obtain the plasmid pET- PGP113. In this plasmid, an α- glucan phosphorylase gene was operably linked under control of an isopropyl- β- D- thiogalactopyranoside (IPTG) inducible promoter. This plasmid was introduced into *Escherichia coli* TG- 1 (manufactured by STRATAGENE) by the competent cell method. This *Escherichia coli* was plated on a plate containing LB medium (1% tryptone (manufactured by Difco) , 0.5% yeast extract (manufactured by Difco) , 1% sodium chloride, and 1.5% agar) containing the antibiotic ampicillin, and this was cultured at 37°C overnight. *Escherichia coli* grown on this plate was selected to obtain *Escherichia coli* in which a potato- derived α- glucan phosphorylase gene had been introduced. It was confirmed by analyzing the sequence of the introduced gene that the resulting *Escherichia coli* contains the α- glucan phosphorylase gene. In addition, it was confirmed by activity measurement that the resulting *Escherichia coli* expresses the α- glucan phosphorylase.

This *Escherichia coli* was inoculated in 1 liter of LB medium (1% tryptone (manufactured by Difco), 0.5% yeast extract (manufactured by Difco) and 1% sodium chloride) containing the antibiotic ampicillin, and this was cultured at 37°C for 3 hours with shaking at 120 rpm. Thereafter, IPTG was added to this medium to 0.1mM, and pyridoxine was added to this medium to 1mM, and this was cultured with shaking at 22°C for a further 20 hours. Then, this culture was centrifuged at 5,000 rpm for 5 minutes to collect the *Escherichia coli* cells. The resulting cells were suspended in 50 ml of 20 mM Tris-HCl buffer (pH 7.0) containing 0.05% Triton X-100 and, then, this was crushed by sonication to obtain 50 ml of a crushed cell liquid. This crushed liquid contained 4.7 U/mg α-glucan phosphorylase.

This crushed cell liquid is heated at 55°C for 30 minutes. After heating, this is centrifuged at 8, 500rpm for 20 minutes to remove insoluble proteins and the like, to obtain the supernatant. The resulting supernatant was applied to an anion exchange resin Q-Sepharose which had been pre-equilibrated, allowing an α-glucan phosphorylase to be adsorbed onto the resin. The resin was washed with a buffer containing 200 mM sodium chloride to remove impurities. Subsequently, the protein was eluted with a buffer containing 300 mM sodium chloride, to obtain the recombinant potato α-glucan phosphorylase enzyme solution.

(Production Example 7: Preparation of *Thermococcus zilligii* AN1-derived α-glucan phosphorylase)

Using a nucleic acid having a base sequence (base sequence of 1$^{st}$ to 2151$^{th}$ described in ACCESSION No. AJ318499 of GenBank base sequence database; SEQ ID NO: 5) encoding the amino acid sequence of *Thermococcus zilligii* AN1-derived α-glucan phosphorylase (SEQ ID NO: 6) in place of an *Aquifex aeolicus* VF5-derived α-glucan phosphorylase gene, α-glucan phosphorylase liquid was obtained in the same manner with the Production Example 1.

(Example 1: Production of glucosamine-containing branched glucan)

A reaction solution containing 50 mM glucosamine- 1- phosphate, 100 mM citrate buffer, *Aquifex aeolicus* VF5- derived α- glucan phosphorylase obtained in Production Example 1 (18 Unit/ml) and the branched glucan (B) obtained in Production Example 2 was incubated at 55°C for 6 hours and allowed to perform an enzymatic reaction to thereby obtained an enzymatically reacted product. In order to determine the structure of the enzymatically reacted product, the result of analysis of an enzymatically treated product of the branched glucan obtained in Production Example 2 and an enzymatically treated product of enzymatically reacted product obtained in the present Example 1 with a HPAEC- PAD apparatus manufactured by DIONEX is shown in Fig. 2. Fig. 2A is the result obtained by analyzing with a HPAEC- PAD apparatus the degradation product obtained after degrading the branched portions of the branched glucan with the sufficient amount (that is, excess amount) of isoamylase to cleave all the branches, and shows the distribution of the length of glucan linear- chain moiety of a branched glucan. Regarding numbers above the peaks in Fig. 2A, **6** indicates maltohexaose, and **7** indicates maltoheptaose. The peaks shown with black solid circles thereabove indicate, from left to right, maltooligosaccharides having the degree of polymerization of glucose of from 8 to 14, respectively. Fig. 2B shows the result obtained by analyzing the degradation product obtained after degrading the branched moiety of a branched glucan with the sufficient amount (that is, excess amount) of isoamylase to cleave all the branches, and then further degrading it with an excess amount of microorganism- derived α- glucosidase (2000Unit/ml) . Since α- glucosidase is an enzyme which degrades glucan in glucose units starting from the non- reducing end, the branched glucan was completely degraded and the peak of glucose (shown by "Glucose" in Fig. 2B) was detected. Fig. 2C shows the result obtained by analyzing the degradation product obtained after degrading the glucosamine- containing branched glucan obtained in the present Example 1 with an excess amount of isoamylase. While the peaks shown with black solid circles in Fig. 2C correspond to the peaks detected at the same elusion time as the peaks shown with black solid circles at the corresponding positions in Fig. 2A, the peaks shown with "x" having different intervals therefromwere detected. Fig. 2D shows the result obtained by analyzing the degradation product obtained after degrading the glucosamine- containing branched glucan obtained in the present Example 1 with an excess amount of isoamylase and further degrading with an excess amount of microorganism- derived α- glucosidase. The group of peaks shown with black solid circles disappeared due to the degradation, and as a result, the peak of glucose (shown by "Glucose" in Fig. 2D) appeared, however, the peaks shown with "x" did not disappeared. That is, the partially degraded product indicated by the peaks shown with "x" exhibited resistance to degradation with α- glucosidase. Therefore, it was confirmed that a branched glucan in which glucosamine residue is bound to the non- reducing end was obtained in this Example 1. Fig. 2E shows the result obtained by analyzing the degradation product obtained after degrading the glucosamine- containing branched glucan obtained in the present Example 1 with an excess amount of isoamylase, and further degrading with an excess amount of α- glucosidase and α- amylase simultaneously. As a result, in addition to the peak of glucose, the peaks shown with asterisks were detected. When a branched glucan is degraded with α- amylase and α- glucosidase, α- amylase hydrolyses α- 1, 4- bonds randomly to degrade the branched glucan into glucose and maltose, and α- glucosidase degrades maltose into glucose. Therefore, when α- glucosidase and α- glucosidase are allowed to react with a branched glucan simultaneously, the branched glucan is completely degraded into glucose, however, in the case of glucosamine- containing branched glucan, since α- amylase and α- glucosidase cannot breake the bond between glucosamine and glucose, the peaks shown with asterisks were detected. In this manner, it was confirmed that the glucosamine- containing branched glucan of the present invention having a structure wherein at least one glucosamine residue is bound to each of a large number of non- reducing ends (an exemplified structure wherein the number of glucosamine residues bound to one non-reducing end is 1 is shown in Fig. 1) could be produced.

The binding ratio of glucosamine to the branched glucan was calculated by quantifying the amount of free inorganic phosphate in the reaction solution, and using the following equation:

binding ratio of glucosamine =(the number of inorganic phosphate generated in the reaction solution/the number of non-reducing end) x 100(%)

(Note: the number of non-reducing end = 1 + the number of branching in branched glucan)

The amount of inorganic phosphate was measured as follows. First, a molybdenum reagent (15 mM ammonium molybdate, and 100 mM zinc acetate) (800 μl) was mixed with a sample (an aqueous solution containing inorganic phosphate) (200 μl), subsequently, 200 μl of a 568 mM aqueous ascorbic acid solution (pH 5.0) was added, and the mixture was stirred to obtain a reaction system. After this reaction system was retained at 30°C for 20 minutes, absorbance at 850 nm was measured using a spectroscopic photometer. Using inorganic phosphate having a known concentration, absorbance was measured similarly, and a standard curve was produced. Absorbance obtained in a sample was fitted on this standard curve to obtain inorganic phosphate in the sample. As a result, it was confirmed that 58% of glucosamine per non-reducing end could be bound to the branched glucan (B) using Aquifex aeolicus VF5-derived α-glucan phosphorylase.

(Example 2: Preparation of glucosamine-containing glucan using a branched glucan as raw material)

Using the potato-derived α-glucan phosphorylase obtained in Production Example 6 (18 Unit/ml) or the *Thermococcus zilligii* AN1-derived α-glucan phosphorylase obtained in Production Example 7 (18 Unit/ml) in place of the Aquifex aeolicus VF5-derived α-glucan phosphorylase obtained in Production Example 1 (18 Unit/ml), glucosamine-containing glucans could be produced by enzymatically reacting similarly to Example 1. By quantifying the amount of free inorganic phosphate in the reaction solution similarly to Example 1, the amount of glucosamine bound to the non-reducing end of the glucosamine-containing glucan was obtained. As a result, it was confirmed that 37% of glucosamine residues per non-reducing end could be bound to the branched glucan (B) using potato-derived α-glucan phosphorylase.

(Example 3: Production of various glucosamine-containing branched glucans (BNs) having different glucosamine binding ratios)

*Aquifex aeolicus* VF5- derived α- glucan phosphorylase (18 Units/ml) was allowed to act on a reaction solution containing the branched glucan produced in Production Example 2, glucosamine- 1- phosphate, and 50 mM sodium acetate buffer (pH 5.5) at 55°C for 4 hours or 18 hours to produce seven kinds of glucosamine- containing branched glucans (BN1 to BN7) having different glucosamine binding ratios. The reaction conditions of the various glucosamine-containing branched glucans (branched glucan concentration, glucosamine- 1- phosphate concentration, and the reaction time) , and the glucosamine binding ratio of the resulting glucosamine- containing branched glucan are shown in Table 2. By quantifying the amount of free inorganic phosphate in the reaction solution similarly to Example 1, the amount of glucosamine bound to the non- reducing end of the glucosamine- containing glucan was obtained.

As shown in Table 2, by increasing the concentration of glucosamine- 1- phosphate added, the glucosamine binding ratio was increased. Further, by elongating the reaction time, the glucosamine binding ratio was increased. In this way, by changing the proportion of glucosamine- 1- phosphate added relative to a branched glucan and changing the reaction time, the introduction amount glucan to the non- reducing end of a branched could be adjusted. Regarding BN6 and BN7, the binding ratio exceeds 100%. This shows that two or more glucosamine residues are bound to one non- reducing end, and as a result, an oligoglucosamine residue having the degree of polymerization of 2 or more is bound to one non- reducing end. That is, assuming that glucosamine residues were bound equally to the non- reducing ends, it is shown that in BN7 glucosamine- containing branched glucan, an oligoglucosamine residue having the degree of polymerization of 2 is bound to 71% of the non- reducing ends, and a glucosamine residue is bound to the remaining 29%. In this way, it was confirmed that it is possible to bind one glucosamine residue with an α- 1, 4- bond to the non-reducing end, and thereafter, another glucosamine residue can further be bound via an α- 1, 4- bond.

[Table 2] Table 2: Reaction conditions for producing various glucosamine-containing branched glucans (BNs) and glucosamine binding ratio.

| | BN1 | BN2 | BN3 | BN4 | BN5 | BN6 | BN7 |
|---|---|---|---|---|---|---|---|
| Branched glucan concentration (mM) | 10 | 10 | 10 | 10 | 10 | 10 | 4 |
| Glucosamine-1-phosphate concentration (mM) | 0.04 | 0.2 | 1 | 4 | 15 | 15 | 10 |
| Reaction time (hrs) | 4 | 4 | 4 | 4 | 4 | 18 | 18 |
| Glucosamine binding ratio at non-reducing end of branched glucan (%) | 1.6 | 5.0 | 20 | 42 | 58 | 131 | 171 |

(Example 4: Production of various glucosamine-containing branched glucan having different glucosamine binding ratios)

To the branched glucan (B) produced in Production Example 2, the branched glucan (P) produced in Production Example 3, the low-molecular weight amylopectin produced in Production Example 4, or the acetylated branched glucan produced in Production Example 5, glucosamine-1-phosphate was added in the concentrations shown in Table 3, then added 100 mM sodium acetate buffer (pH 5.5) and *Aquifex aeolicus* VF5-derived α-glucan phosphorylase (18 Unit/ml), and allowed to react at 55°C. The reaction time is as shown in Table 3. As a result, glucosamine-containing branched glucans (BN1 to BN7, PN1 to PN6, AN1, and AcBN8) could be produced. By quantifying the amount of free inorganic phosphate in the reaction solution similarly to Example 3, the amount of glucosamine bound to the non-reducing end of the branched glucan was obtained.

As shown in Table 3, by changing the proportion of glucosamine-1-phosphate added relative to branched glucan and changing the reaction time, introduction amount of glucosamine into the non-reducing end of branched glucan could be adjusted.

[Table 3] Table 3: Reaction conditions for producing various glucosamine-containing branched glucans and glucosamine binding ratio.

| Branched glucan used as raw material | Branched glucan concentration (mM) | Glucosamine-1-phosphate concentration (mM) | Reaction time (hrs) | Binding ratio[1] (%) | Abbreviation[2] |
|---|---|---|---|---|---|
| Production Example 2 | 10 | 0.04 | 4 | 1.6 | BN1 |
| | 10 | 0.2 | 4 | 5 | BN2 |
| | 10 | 1 | 4 | 20 | BN3 |
| | 10 | 4 | 4 | 42 | BN4 |
| | 10 | 15 | 4 | 58 | BN5 |
| | 10 | 15 | 18 | 13<br>1 | BN6 |
| | 4 | 10 | 18 | 17<br>1 | BN7 |
| Production Example 3 | 20 | 10 | 18 | 36 | PN1 |
| | 15 | 10 | 18 | 53 | PN2 |
| | 10 | 10 | 18 | 57 | PN3 |
| | 5 | 10 | 18 | 64 | PN4 |
| | 2 | 10 | 18 | 69 | PN5 |
| | 5 | 2.5 | 42 | 87 | PN6 |
| Production Example 4 | 2 | 5 | 42 | 159 | AN1 |
| Production Example 5 | 2 | 5 | 42 | 63 | AcBN8 |

Binding ratio[1] (%) : Glucosamine binding ratio to a non-reducing end of a branched glucan (%).
Abbreviation[2] : Abbreviations for glucosamine-containing glucans.

(Example 5: Production of various branched glucans to which glucosamine and mannose are bound)

To each of three kinds of glucosamine- containing branched glucans BN7, PN5 and AN1 produced in Example 4 (5 mM) , 5 mM of mannose- 1- phosphate, 100 mM sodium acetate buffer (pH 5.5) , and *Aquifex aeolicus* VF5- derived α- glucan phosphorylase (18 Unit/ml) were added and allowed to react at 55°C for 42 hours. As a result, a glucosamine/ mannose- containing branched glucan having mannose bound to a glucosamine- containing branched glucan could be produced. By quantifying the amount of free inorganic phosphate in the reaction solution similarly to Example 3, the amount of mannose bound to the non- reducing end of the glucosamine- containing branched glucan was obtained. Relative to the number of the non- reducing ends, 13%, 9%, and 18% of mannose could be introduced to BN7, PN5, and AN1, respectively. As a result, the branched glucans having glucosamine residue and mannose residue bound to non- reducing ends could be produced.

(Summary of Examples 1 to 5)

The glucans used in Examples 1 to 5 as raw material and the glucosamine-containing branched glucansobtainedin each Example are shown below.

[Table 3A] Glucans used as raw material in Examples.

| | | Abbreviation | Weight average molecular weight of product (Da) | Unit chain length of product | Number of branching in product |
|---|---|---|---|---|---|
| Production Example 2 | Branched glucan | B | about 140K | about 15 | about 60 |
| Production Example 3 | Branched glucan | P | about 4000K | about 15 | about 1600 |
| Production Example 4 | Low molecular weight amylopectin | A | about 6100K | about 22 | about 1500 |
| Production Example 5 | Acetylated branched glucan | AcB | about 140K | about 15 | about 60 |

[Table 3B] Glucosamine-containing branched glucans.

| Example | Raw material | Abbreviation | Mw[1] (Da) | Average unit chain length[2] | Number of branching[3] | GlcN Binding ratio[4] (%) | GlcN Binding number[5] | Man Binding ratio[6] (%) | Man Binding number[7] |
|---|---|---|---|---|---|---|---|---|---|
| 1 | B | | about 140K | about 15 | about 60 | 58 | about 35 | - | - |
| 2 | B | | about 140K | about 15 | about 60 | 37 | about 22 | - | - |
| 3/4 | B | BN1 | about 140K | about 15 | about 60 | 1.6 | about 1 | - | - |
| | | BN2 | about 141K | about 15 | about 60 | 5.0 | about 3 | - | - |
| | | BN3 | about 142K | about 15 | about 60 | 20 | about 12 | - | - |
| | | BN4 | about 145K | about 15 | about 60 | 42 | about 25 | - | - |
| | | BN5 | about 146K | about 16 | about 60 | 58 | about 35 | - | - |
| | | BN6 | about 154K | about 16 | about 60 | 131 | about 77 | - | - |
| | | BN7 | about 159K | about 17 | about 60 | 171 | about 103 | - | - |
| 4 | P | PN1 | about 4100K | about 15 | about 1600 | 36 | about 576 | - | - |
| | | PN2 | about 4150K | about 16 | about 1600 | 53 | about 848 | - | - |
| | | PN3 | about 4160K | about 16 | about 1600 | 57 | about 912 | - | - |
| | | PN4 | about 4180K | about 16 | about 1600 | 64 | about 1024 | - | - |
| | | PN5 | about 4200K | about 16 | about 1600 | 69 | about 1104 | - | - |
| | | PN6 | about 4250K | about 16 | about 1600 | 87 | about 1392 | - | - |
| | A | AN1 | about 6500K | about 24 | about 1500 | 159 | about 2385 | - | - |
| | AcB | AcBN 8 | about 147K | about 16 | about 60 | 63 | about 38 | - | - |

(continued)

| Example | Raw material | Abbreviation | Mw[1] (Da) | Average unit chain length[2] | Number of branching[3] | GlcN Binding ratio[4] (%) | GlcN Binding number[5] | Man Binding ratio[6] (%) | Man Binding number[7] |
|---|---|---|---|---|---|---|---|---|---|
| 5 | BN7 | | about 160K | about 17 | about 60 | 171 | about 103 | 13 | about 8 |
| | PN5 | | about 4220K | about 16 | about 1600 | 69 | about 1104 | 9 | about 144 |
| | AN1 | | about 6500K | about 24 | about 1500 | 159 | about 2385 | 18 | about 270 |

Mw[1] = Weight average molecular weight of product.
Average unit chain length[2] = Average unit chain length of product.
Number of branching[3] = Average number of branching in product. GlcN Binding ratio[4] = Average binding ratio of glucosamine to a non-reducing end of product (weight average).
GlcN Binding number[5] = Average number of glucosamine residue bound to one molecule of product (weight average).
Man Binding ratio[6] = Average binding ratio of mannose to a non-reducing end of product (weight average).
Man Binding number[7] = Average number of mannose residue bound to one molecule of product (weight average).

(Example 6: Making protein to be a macromolecule) 0.2 mg of albumin (derived from bovine serum) and 2 mg of the glucosamine-containing branched glucan produced in Example 1 were dissolved in 700 μl of a 0.1 M 2- [N-morpholino] ethane sulfonic acid (MES) buffer (pH 5.0).

After 1 mg of 1- ethyl- 3- (3- dimethylaminopropyl) carbodiimide (EDC) was added and allowed to react at room temperature for 2 hours, the reaction solution was diluted to 1/4 with water, and desalted using a PD- 10 column (manufactured by GE) . The resulting solution was analyzed by FPLC analysis using a Superose 6 10/300 GL column (a column for size fractionation, manufactured by GE) . Using an eluent (a 50 mM phosphate buffer (pH 7.0) , 150 mM sodium chloride) , elusion was performed at 0.5 ml/min, and the product was detected with UV (280 nm) . The product of a sample obtained by a condensation reaction of the glucosamine- containing glucan and albumin had an early elution time and, it was shown that the molecular size is great. Therefore, it was shown that the glucosamine- containing glucan and a protein can be dehydration- condensed to prepare a macromolecule.

(Example 7 and Comparative Example 1: Complex formation of glucosamine-containing branched glucan and a nucleic acid)

0.5 μg of lambda DNA-Hind III fragment, each sample was added respectively and allowed to stand at room temperature for 5 minutes in 10 μl, and it was subjected to 1% agarose gel electrophoresis. Each sample used was as follows: Sample 1 was ion-exchanged water (Comparative Example 1-1) ; Sample 2 was 0.1 mg of branched glucan obtained in Production Example 2 (Comparative Example 1-2); Sample 3 was 0.5 mg of branched glucan obtained in Production Example 2 (Comparative Example 1-3) ; Sample 4 was 0.1 mg of glucosamine-containing branched glucan obtained in Example 1 (Example 7-1); Sample 5 was 0.5 mg of glucosamine-containing branched glucan obtained in Example 1 (Example 7-2); Sample 6 was the same molar concentration of glucosamine as that of glucosamine unit contained in Sample 4 (Comparative Example 1-4); and Sample 7 was the same molar concentration of glucosamine as that of glucosamine unit contained in Sample 5 (Comparative Example 1-5). The results of agarose gel electrophoresis are shown in Fig. 3. In Fig. 3, Lane 1 is the result of Sample 1, Lane 2 is the result of Sample 2, Lane 3 is the result of Sample 3, Lane 4 is the result of Sample 4, Lane 5 is the result of Sample 5, Lane 6 is the result of Sample 6, and Lane 7 is the result of Sample 7. While bands (a) were detected in Samples 1, 2 and 3, DNA did not migrate in agarose gel in Samples 4 and 5 to which glucosamine-containing branched glucan was added (b). Therefore, it was found that a cationized glucosamine-containing glucan can form a complex with a DNA. This result is clear from Lanes 4 and 5 in Fig. 3. On the other hand, when glucosamine was used, change in mobility of DNA in agarose gel electrophoresis did not observed, and it was confirmed that glucosamine cannot change the charge and the molecular weight of a DNA. This result is clear from Lanes 6 and 7 in Fig. 3.

(Reference Example 8: Production of glucosamine-containing glucan using linear glucan as raw material)

Using a linear glucan (weight average molecular weight about 5000) in place of the branched glucan obtained in Production Example 2, glucosamine-containing glucan was produced by enzymatically reacting similarly to Example 1. By analyzing similarly to Example 1, it was confirmed that one glucosamine residue is bound to the non-reducing end of the resulting glucosamine-containing glucan.

(Reference Example 9: Stabilization of nucleic acid and complex formation by glucosamine-containing linear glucan)

The same treatments as the method described in Example 7 were performed except for using the glucosamine-containing linear glucan obtained in Reference Example 8 (weight average molecular weight about 5000) in place of the glucosamine-containing branched glucan obtained in Example 1, and agarose gel electrophoresis was performed. Change in mobility of DNA was observed, and the effect of increasing molecular weight could be confirmed. However, the effect of increasing molecular weight was slight as compared to that of the glucosamine-containing branched glucan obtained in Example 1.

(Example 10, Comparative Example 2 and Comparative Example 3: Evaluation of binding force between glucosamine-containing branched glucan and a nucleic acid)

50 mg of oligo (dT)- cellulose (Wako Pure Chemical Industries, Ltd.) was previously washed with 2MNaCl solution, and thereafter, it was equilibrated with ultrapure water. The equilibrated oligo (dT)- cellulose was suspended in 1 ml solutions of 0.1 M, 0.2 M, 0.3 M, 0.5 M, 1 M, and 2 M of NaCl, added thereto 0.2 mg/ 20 μL of the glucosamine-containing branched glucan obtained in Example 3 or Example 4 (BN5, BN6 or PN6) , the branched glucan (B) obtained in Production Example 2, the glucosamine- containing linear glucan obtained in Reference Example 8, or polylysine,

mixed, and allowed the mixture to stand at room temperature for 30 minute, and thereafter, centrifuged at 3, 000 rpm for 5 minutes. The amount of glucosamine- containing branched glucan (or branched glucan or linear glucan) , which were not bound to oligo (dT)- cellulose, contained in the supernatant fraction was obtained by a phenol sulfuric acid method, and the proportion (%) of the amount of the bound cationized glucan (total sugar amount) relative to the amount of glucosamine- containing glucan (or branched glucan or linear glucan) added (total sugar amount) . The result is shown in Fig. 4. It was found that BN6 having greater amount of bound glucosamine of the cationized glucan than those of BN5 retains the bond under higher NaCl concentration. Further, it was found that PN6 having higher molecular weight and more branching than those of BN6 retains the bond stably even in higher NaCl concentration. From these results, it was found that the binding force with a nucleic acid can be controlled by the binding amount of glucosamine residue.

As described above, the binding force with oligo (dT) -cellulose was evaluated using the branched glucan obtained in Production Example 2. As shown in Fig. 4, it was found that the branched glucan has little binding force to a nucleic acid.

As described above, the binding force with oligo (dT)- cellulose was evaluated using polylysine. However, quantity of polylysine was measured using Protein Quantification Kit (manufactured by Dojindo) and according to the instructions of the manufacture. As shown in Fig. 4, the binding force between polylysine and oligo (dT)- cellulose is very strong, and their binding cannot be separated even by increasing the concentration of NaCl to 2 M.

Since polylysine is a polymerization product of cationic amino acid, the charge is very strong, and when complexed with a nucleic acid, they bind too strongly to be separated. Therefore, when a complex of polylysine and a nucleic acid is utilized as a medically effective ingredient of a medicament, it is expected that the toxicity of the medically effective ingredient is very high, and this is a problem.

In case of glucosamine-containing branched glucan, it binds with a nucleic acid stably at 0.15 M NaCl which is the level of a physiological saline, and it can be separated from the nucleic acid at 1 M NaCl.

On the other hand, while the glucosamine-containing linear glucan used herein is an isoamylase-degradation product of BN5, this linear glucan is separated from a nucleic acid under the condition of low concentration of NaCl. Specifically, this linear glucan is separated from a nucleic acid at the level of a physiological saline. That is, the binding force of the linear glucan to a nucleic acid is too weak to be used in a body. At the same time, the result of the linear glucan shows that when glucosamine-containing branched glucan is administered in a living body, the branched glucan is degraded by a glucan degrading enzyme in the living body, and thereby the binding force between the product and a nucleic acid becomes lower, and as a result, the nucleic acid can be released progressively.

The concentration of NaCl at which a nucleic acid and glucosamine-containing glucan are bound stably is preferably about 0.1 M or more and about 2 M or less, more preferably about 0.15 M or more and about 1 M or less.

(Example 11: Transfection experiment of macrophage cell line using glucosamine-containing branched glucan)

pGL4.51 [luc2P/CMV/NEO] Vector comprising LUC gene encoding firefly-derived luciferase (manufactured by Promega) was mixed with the glucosamine-containing branched glucan (PN6) obtained in Example 4, polyethyleneimine or polylysine in an aqueous solution and allowed to stand at room temperature for 15 minutes, thereby formed a complex of the vector and the glucosamine-containing glucan. The ratio of mixing was based on N/P ratio (the molar ratio of phosphate group of DNA and glucosamine-containing branched glucan), N/P ratios of 2, 10, 50 or 200 were used for PN6, and N/P ratio of 200 was used for polyethyleneimine and polylysine. The solution containing the complex was added without purification to macrophage cell line RAW264.7 (purchased from DS Pharma Biomedical Co., Ltd.) culture (seeded at the cell number per well of 4 x $10^4$/0.1mL/well and cultured in Dulbecco's Modified Medium (D-MEM) for 24 hours). The culture was incubated at 37°C, under 5% $CO_2$ atmosphere for 24 hours, and thereafter, the medium was removed, and Reporter Lysis Buffer (manufactured by Promega) was added to lyse the cells. All according to the instructions of the manufacturer, photogenesis reaction was performed on a 96-well plate, and determined the luciferase activity in the supernatant of cell lysate using a luminometer (manufactured by Berthold, Centro LB 960).

As shown in Fig. 5, it was found that PN6 exhibit transfection function, and especially, the highest transfection efficiency was exhibited when N/P ratio was 200. Polyethyleneimine had remarkably low efficiency at N/P ratio of 200. Polylysine had remarkably low efficiency at N/P ratio of 200.

(Example 12: Evaluation of cytotoxicity in transfection of macrophage cell line using glucosamine-containing branched glucan)

Concentration of protein in the supernatant of cell lysate obtained in Example 11 was measured using Protein Quantification Kit (manufactured by Dojindo) according to the instructions of the manufacturer to thereby investigate the change in amount of cell by transfection. The lower concentration of the protein shows that the cytotoxicity is stronger. As shown in Fig. 6, while polyethyleneimine and polylysine generated high toxicity at N/P ratio of 200, PN6 did not exhibit remarkable toxicity even at N/P ratio of 50 or more. As described above, it was shown that while polyethyleneimine and

polylysine cannot be used since their transfection efficiency is low and toxicity is high at N/P ratio of 200, the glucosamine-containing branched glucan of the present invention is very useful as a carrier for transfection since its toxicity is low and transfection efficiency is high even at a high N/P ratio.

(Example 13: Synergistic effect in transfection of macrophage cell line when using glucosamine-containing branched glucan and polyethyleneimine in combination)

pGL4.51 [luc2P/CMV/NEO] Vector comprising LUC gene encoding firefly-derived luciferase (manufactured by Promega) was mixed with the glucosamine-containing branched glucan (BN7) obtained in Example 3 in an aqueous solution and allowed to stand at room temperature for 15 minutes, thereby formed a complex of the vector and the glucosamine-containing glucan. The ratio of mixing was based on N/P ratio (the molar ratio of phosphate group of DNA and glucosamine-containing branched glucan), and N/P ratios of 2, 10, 50 or 200 were used. Then, polyetyleneimine was added to the mixture to make N/P ratio 10, and thereafter allowed to stand at room temperature for 15 minutes, thereby further complexed polyethyleneimine to the complex of the vector and the glucosamine-containing glucan. The resulting complex was added to macrophage cell line RAW264.7 (purchased from DS Pharma Biomedical Co., Ltd.) culture (seeded at the cell number per well of 4 x $10^4$/0.1mL/well and cultured in Dulbecco's Modified Medium (D-MEM) for 24 hours). The culture was incubated at 37°C, under 5% $CO_2$ atmosphere for 24 hours, and thereafter, the medium was removed, and Reporter Lysis Buffer (manufactured by Promega) was added to lyse the cells. All according to the instructions of the manufacturer, photogenesis reaction was performed on a 96-well plate, and determined the luciferase activity in the supernatant of cell lysate using a luminometer (manufactured by Berthold, Centro LB960). The result is shown in Table 4. It was found that compared to polyethyleneimine alone or glucosamine-containing branched glucan alone, when both of polyethyleneimine and glucosamine-containing branched glucan were added, the transfection efficiency was remarkably high.

[Table 4] Table 4: Transfection efficiency of glucosamine-containing branched glucan,polyethyleneimine, and mixture thereof (in amount of luminescence (Relative Light Unit; RLU))

| | | Amount of added glucosamine-containing branched glucan (BN7) | |
|---|---|---|---|
| | | (N/P 2) | (N/P 200) |
| Amount of added polyethyleneimine | Not added | 3.6 x $10^3$ | 101 x $10^3$ |
| | (N/P 2) | 95 x $10^3$ | 769 x $10^3$ |
| *The numbers in boxes of thick lines represent transfection efficiency. | | | |

(Example 14 : α-amylase digestibility)

1% solution of each of the glucosamine-containing branched glucans obtained in Example 4 was made in a reaction solutioncontaining0.2Macetatebuffer (pH5.5), 1 mM calcium chloride, and porcine pancreas-derived α-amylase (26 unit/mL), and reacted at 37°C for 4 hours. After 4 hours, a portion of the reaction solution was taken and glucose was quantified using Glucose CII Test Wako and according to the instructions of the manufacturer. The total sugar amount was obtained by a phenol sulfuric acid method, and digestibility (%) = {glucose content (g) / total sugar amount (g)} x 100 was calculated. α-amylase digestion of glucosamine-containing branched glucan was 31.6%, and α-amylase digestibility of glucosamine-containing acetylated branched glucan was 9.3%. Therefore, α-amylase digestibility could be suppressed by acetylating glucan.

(Example 15: Particle formation by glucosamine-containing branched glucan and anionic glucan)

The glucosamine-containing glucan (BN5) obtained in Example 4 and an anionic glucan were mixed and the particle size was investigated. For an anionic glucan, 15 mM of glucuronic acid-1-phosphate was added relative to 10 mM of branched glucan produced in Production Example 2, added 100 mM sodium acetate buffer (pH 5.5) and *Aquifex aeolicus* VF5-derived α-glucan phosphorylase (18 Unit/ml) and allowed to react at 55°C for 18 hours, thereby glucuronic acid-containing glucan (an anionic glucan) was produced. The binding ratio of glucuronic acid to the branched glucan was obtained by quantifying the amount of free inorganic phosphate in the reaction solution similarly to Example 3, and as a result, it was 50%. 1% solution for each of BN5 and an anionic glucan was prepared and mixed at various ratio, and allowed to stand at room temperature for 20 minutes, and thereafter, measured using a particle size measuring

apparatus (zeta sizer nano manufactured by Malvern Instrument).
The particle size of BN5 was 20 nm, and the particle size of the anionic glucan was 23 nm. The mixture of BN5 and anionic glucan formed a complex particle. The particle size of 1:1 mixture of BN5 and anionic glucan was 200 μm, and the particle size of 10:1 mixture was 53 μm, and the particle size of 100:1 mixture was 774 nm.

(Example 16: Fluorescent labeling of glucosamine-containing branched glucan)

Each glucosamine-containing branched glucan BN5 or AcBN8 obtained in Example 4 was prepared into a 50 mg/ml dimethyl sulfoxide solution. 2 ml of each glucan/dimethyl sulfoxide solution was placed into a test tube, and the solution was stirred at 90°C. 56 μl or 112 μl of 50 mg/ml of fluorescein isothiocyanate (FITC) solution in dimethyl sulfoxide was added to these respectively, one droplet of pyridine and one droplet of di-n-butyltin dilaurate were added to obtain reaction solutions. These reaction solutions were stirred at 90°C for 2 hours. After completion of the reaction, 20 ml of ethanol was added to these reaction solutions, these were centrifuged at 3,500 rpm for 5 minutes, and the precipitates were collected. Ethanol was further added to the precipitates, washing was performed, the washed precipitates were dissolved in 1 ml of ultrapure water, 1 ml of the solutions were placed on a gel filtration column (PD-10), 1.5 ml of ultrapure water was passed therethrough and, thereafter, 2 ml was collected with ultrapure water to perform purification. The resulting samples were lyophilized. Regarding a part of the resulting sample, UV absorption at 490 nm was measured. When each FITC introduction amount for BN5 and AcBN8 was obtained using uranine as a standard substance, it was confirmed that each introduction ratio of FITC was 0.86% and 0.82%.

(Introduction ratio of FITC (W/W %) = ((fluorescein concentration of FITC-labeled branched glucan) / (total sugar amount of FITC-labeled branched glucan)) x 100)

(Example 17: Production of reducing end-modified product of glucosamine-containing glucan)

To a solution wherein 6 g of glucose- 1- phosphate and 0.5 mg of maltosyl trehalose were dissolved in 100 ml of 0.2 M maleate buffer (pH 6.0) , 150 U of *Aquifex aeolicus* VF5- derived α- glucan phosphorylase produced in Production Example 1 of the present invention and 2, 000 U of highly thermostable branching enzyme prepared according to the method described in Example 1 of Japanese Laid- Open Publication No. 2000- 316581 were added to prepare a reaction solution, and thereafter, the reaction was performed for 16 hours with stirring at 50°C. The enzyme was inactivated by heating the reaction solution, and the inactivated enzyme was removed by filtering through a glass filter. Twice the amount of ethanol was added to the filtrate to precipitate the glucan reaction product and then centrifuged. The precipitate was washed twice with 300 milliliters of 1: 1 water: ethanol solution to remove the coexisting glucose- 1- P, and the precipitate was further washed twice with ethanol, and thereafter lyophilized. The reducing end- modified branched glucan having molecular weight of 110 kDa was obtained in yield of 1.8 g.
On a reaction solution containing 1.6 mg/mL of the above mentioned reducing end- modified branched glucan, 10 mM glucosamine- 1- phosphate, and 50 mM sodium acetate buffer (pH 5.5) , *Aquifex aeolicus* VF5- derived α- glucan phosphorylase produced in Production Example 1 of the present invention (18 Unit/ml) was allowed to act at 55°C for 18 hours, and thereby glucosamine- containing glucan could be produced. The binding ratio of glucosamine was obtained in a similar method as Example 3, and it was 74%. In this way, the glucosamine- containing branched glucan having a modified reducing end was obtained.

(Example 18: Production of glucosamine-containing glucan having gluconic acid residue further bound at reducing end)

10 ml of 0.2 M $I_2$/ MeOH solution was added to 10 ml of 5% maltopentaose (G5) , and 4 ml of 4% KOH/ MeOH solution was added dropwise slowly with stirring at 30°C to conduct iodate oxidation reaction. Until the color of iodine disappeared, 4% KOH/ MeOH solution was further added dropwise and reacted for 1 hour with stirring. Methanol in three times amount of the volume of the resulting reaction solution was added to precipitate G5 oxide, and the precipitate (about 0.4 g) was collected by centrifugation. The precipitate was maltohexaosyl gluconic acid wherein reducing terminus glucose residue of maltopentaose was converted to gluconic acid residue. To a solution wherein 6 g of glucose- 1- phosphate and 0.6 mg of maltohexaosyl gluconic acid were dissolved in 100 ml of 0.2 M maleate buffer (pH 6.0) , 150 U of *Aquifex aeolicus* VF5- derived α- glucan phosphorylase produced in Production Example 1 of the present invention and 2, 000 U of highly thermostable branching enzyme prepared according to the method described in Example 1 of Japanese Laid- Open Publication No. 2000- 316581 were added to prepare a reaction solution, and thereafter, the reaction was performed for 16 hours with stirring at 50°C. The enzyme was inactivated by heating the solution, and the

inactivated enzyme wasremoved byfilteringthrough a glassfilter. Twice amount of ethanol was added to the filtrate to precipitate the glucan reaction product and centrifuged. The precipitate was washed twice with 300 milliliters of 1: 1 water: ethanol solution to remove the coexisting glucose- 1- P, and further washed twice with ethanol, and thereafter lyophilized. The branched glucan which has gluconic acid bound to the reducing end- modified having molecular weight of 120 kDa was obtained in yield of 1.7 g.

To a reaction solution containing 1.6 mg/mL of the above mentioned reducing end- modified branched glucan, 10 mM glucosamine- 1- phosphate, and a 50 mM sodium acetate buffer (pH 5.5) , *Aquifex aeolicus* VF 5- derived α-glucan phosphorylase (18 Unit/ml) was added and allowed to act at 55°C for 18 hours, and thereby glucosamine residue was bound to the non- reducing end of a branched glucan having a gluconic acid residue bound to the reducing end. The binding ratio of glucosamine was obtained in a similar method as Example 3, and it was 70%. In this way, the glucosamine- containing branched glucan having a modified reducing end was obtained.

As described above, the present invention has been exemplified using preferable embodiments of the present invention, but the present invention should not be construed so as to be limited to these embodiments. It is understood that the scope of the present invention should be construed only by claims. It is understood that those skilled in the art can carry out an equivalent scope based on the description of the present invention and common technical knowledge, from the description of specific preferable embodiments of the present invention. It is understood that the content of patents, patent applications and references cited in the present specification should be incorporated herein by reference, as if the content itself is specifically described in the present specification.

## Industrial Applicability

The novel amino sugar-containing glucan of the present invention has an amino sugar residue bound only to the non-reducing end. Since the present amino sugar-containing glucan has an amino sugar on a terminus, a glucan terminus comes to be positively charged, and a physicochemical nature of the glucan is changed. The strength of the charge can be changed in accordance with the frequency of introduction of the amino sugar. The present amino sugar-containing glucan binds non-covalently to a nucleic acid to increase the apparent molecular weight of the nucleic acid. The present amino sugar-containing glucan can be widely utilized in foods, cosmetics, medicaments and the like.

## Sequence Listing free text

SEQ ID NO: 1: Base sequence of the *Aquifex aeolicus* VF5-derived α-glucan phosphorylase.
SEQ ID NO: 2: Amino acid sequence of the *Aquifex aeolicus* VF5-derived α-glucan phosphorylase.
SEQ ID NO: 3: Base sequence of the potato-derived α-glucan phosphorylase.
SEQ ID NO: 4: Amino acid sequence of the potato-derived α-glucan phosphorylase.
SEQ ID NO: 5: Base sequence of the *Thermococcus zilligii* AN1-derived α-glucan phosphorylase.
SEQ ID NO: 6: Amino acid sequence of the *Thermococcus zilligii* AN1-derived α-glucan phosphorylase.

## Claims

1. A glucosamine-containing branched glucan, wherein the glucan has a plurality of non-reducing ends and at least one glucosamine residue is bound via an α-1,4-bond to each of two or more non-reducing ends of the branched α-1,4-glucan, but no glucosamine residue is present at the position other than the non-reducing ends of the branched α-1,4-glucan, and the degree of polymerization of the branched α-1,4-glucan is 15 or more and 4 x $10^5$ or less.

2. The glucosamine-containing branched glucan according to claim 1, wherein the branched α-1,4-glucan is selected from the group consisting of a branched maltooligosaccharide, a starch, amylopectin, glycogen, dextrin, enzymatically synthesized branched glucan and highly branched cyclic glucan.

3. A hydroxyl group-modified product of the glucosamine-containing branched glucan according to claim 1, wherein the modification on the hydroxyl group is a modification on some or all of alcoholic hydroxyl groups of the glucan, and the modification on the hydroxyl group is independently selected from the group consisting of hydroxyalkylation, alkylation, acetylation, carboxymethylation, sulfation and phosphorylation.

4. A reducing end-modified product of the glucosamine-containing branched glucan according to claim 1 or a hydroxyl group-modified product thereof.

5. An amino group-modified product of the glucosamine-containing branched glucan according to claim 1, a hydroxyl group-modified product thereof or a reducing end-modified product thereof, wherein the modification on the amino group is a modification on some or all of amino groups of the glucosamine residues, the modification on the amino group is attained by a reaction of the amino group and an amino group-modifying reagent, and the amino group-modifying reagent has at least one carboxyl group and at least one other functional group.

6. A non-reducing end-modified product of the glucosamine-containing branched glucan according to claim 1, a hydroxyl group-modified product thereof, a reducing end-modified product thereof or an amino group-modified product thereof, wherein a targeting molecule is bound to at least one of non-reducing ends to which the glucosamine residue of the branched glucan is not bound, wherein the targeting molecule is selected from the group consisting of mannose, galactose, glucuronic acid, N-acetylglucosamine, xylose, fucose, galactosamine, an antibody, an antibody fragment, a receptor, a receptor fragment and a receptor ligand.

7. A method for producing a glucosamine-containing branched glucan, **characterized by** allowing an α-glucan phosphorylase to act on an aqueous solution comprising a branched α-1,4-glucan and glucosamine-1-phosphate, wherein the degree of polymerization of the branched α-1,4-glucan is 15 or more and 4 x $10^5$ or less.

8. The method according to claim 7, wherein the α-glucan phosphorylase has 95% or more sequence identity with the amino acid sequence of α-glucan phosphorylase derived from *Aquifex aeolicus* VF5, and has activity of transferring glucosamine to a non-reducing end of a branched glucan to form an α-1,4-bond.

9. A medicament comprising the glucosamine-containing branched glucan according to claim 1, a hydroxyl group-modified product thereof, a reducing end-modified product thereof, an amino group-modified product thereof, or a non-reducing end-modified product thereof, and a medically effective ingredient.

10. The medicament according to claim 9, wherein the medically effective ingredient is selected from the group consisting of a low-molecular weight organic compound, a protein, a peptide, an antibody, an antibody fragment, a receptor, a receptor fragment, a DNA, an RNA, a siRNA, an miRNA and an RNA aptamer.

11. A composition for clinical diagnosis comprising the glucosamine-containing branched glucan according to claim 1, a hydroxyl group-modified product thereof, a reducing end-modified product thereof, an amino group-modified product thereof, or a non-reducing end-modified product thereof.

12. A nanoparticulate carrier for a DDS comprising the glucosamine-containing branched glucan according to claim 1, a hydroxyl group-modified product thereof, a reducing end-modified product thereof, an amino group-modified product thereof, or a non-reducing end-modified product thereof.

13. The carrier according to claim 12, wherein the nanoparticulate carrier for a DDS is selected from the group consisting of a liposome, a virus particle, a macromolecule micelle and a nanogel composed of macromolecule bearing hydrophobic groups.

**14.** A complex formed with a nucleic acid molecule and the glucosamine-containing branched glucan according to claim 1.

**15.** The complex according to claim 14, wherein the nucleic acid molecule is selected from the group consisting of a DNA, an RNA, a siRNA, an miRNA and an RNA aptamer.

**16.** A complex formed with the complex carrier according to claim 14, and a cationic polymer or a cationic lipid.

**17.** The complex according to claim 16, wherein the cationic polymer comprises at least one cationic polymer selected from the group consisting of polyethyleneimine, polylysine, polyarginine, polyamidoamine dendrimer, poly(aminostyrene), chitosan, a cationic glucan and DEAE-dextran.

**18.** A method for delivering a nucleic acid molecule into an isolated cell, comprising contacting the complex according to claim 14 with the cell.

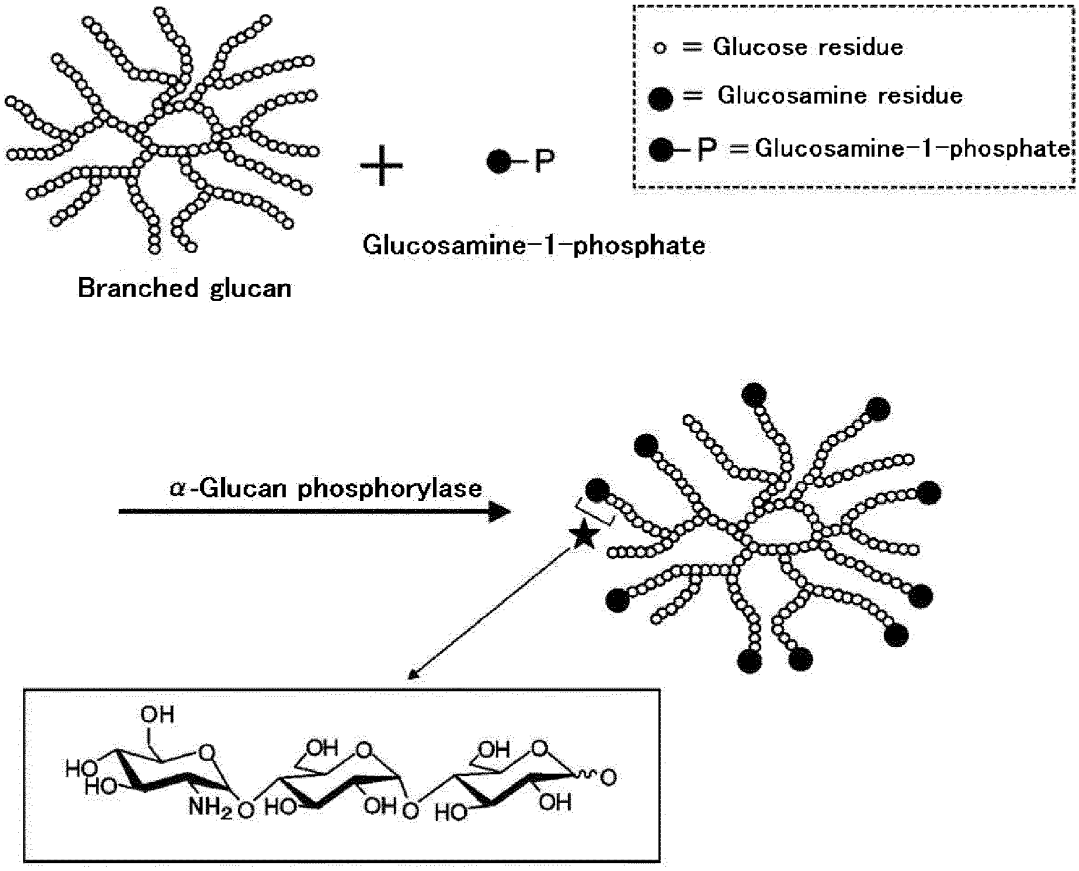
○ = Glucose residue
● = Glucosamine residue
●-P = Glucosamine-1-phosphate
Branched glucan
Glucosamine-1-phosphate
α-Glucan phosphorylase

*Fig.1*

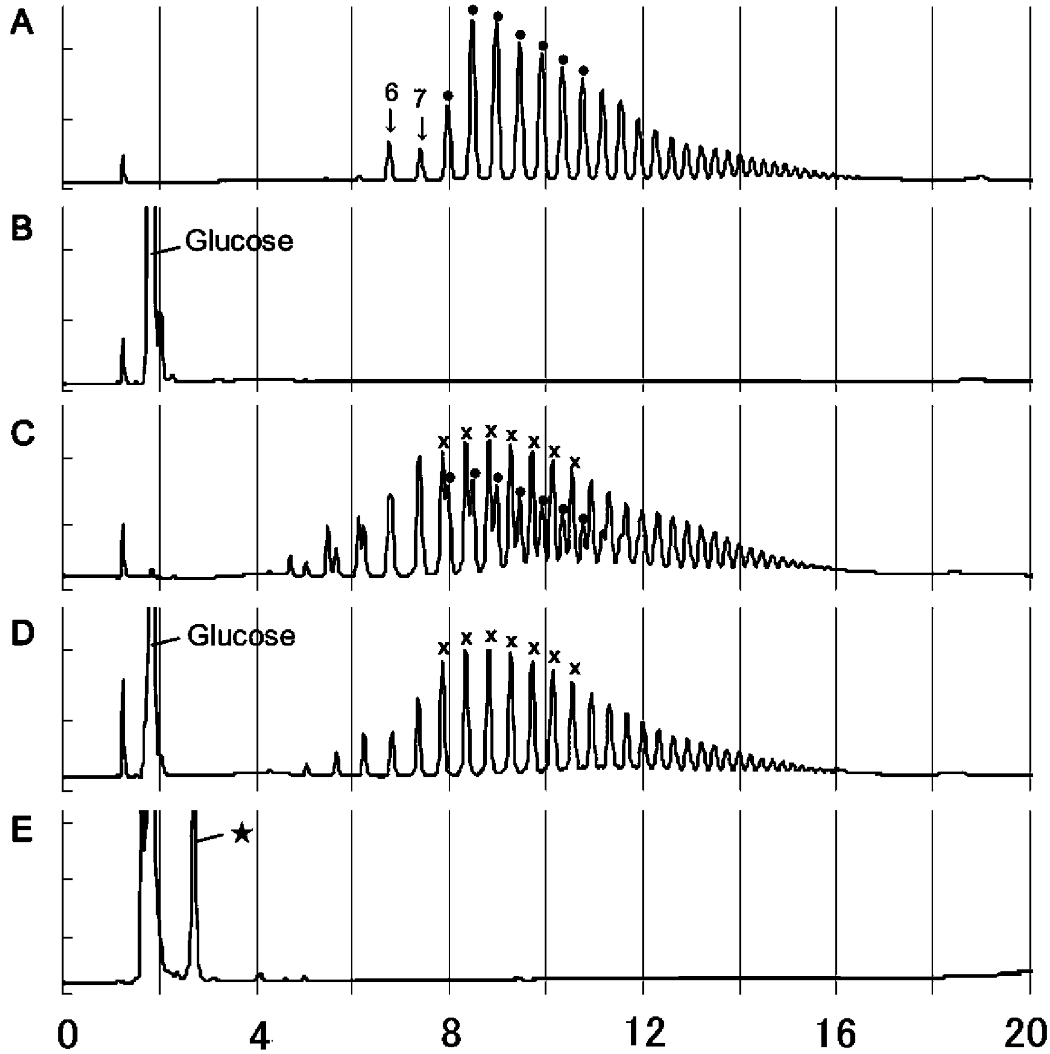


*Fig.2*

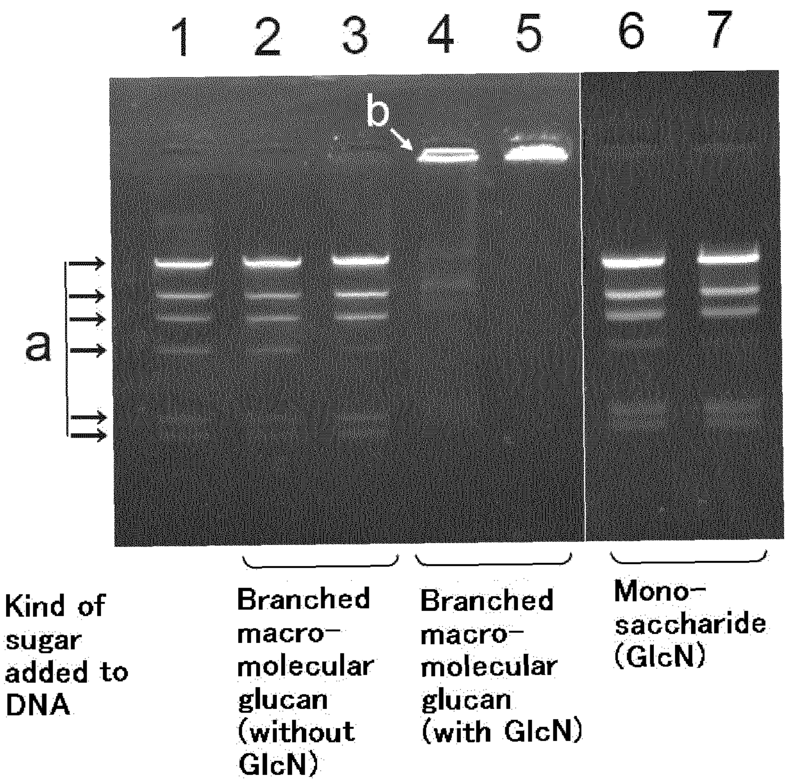
1
2
3
4
5
6
7
b
a
Kind of sugar added to DNA
Branched macro-molecular glucan (without GlcN)
Branched macro-molecular glucan (with GlcN)
Mono-saccharide (GlcN)

*Fig. 3*

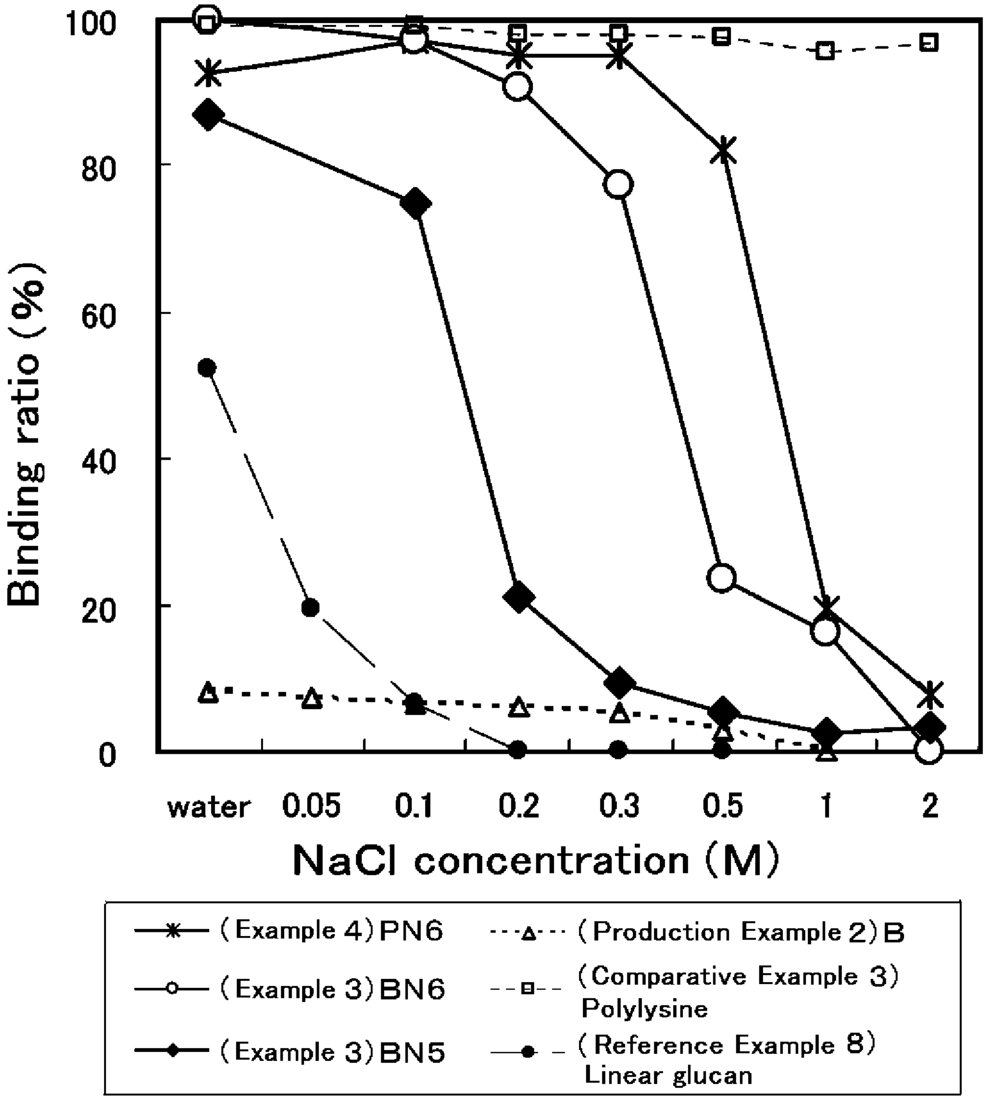
100
80
60
40
20
0
Binding ratio (%)
water
0.05
0.1
0.2
0.3
0.5
1
2
NaCl concentration (M)
(Example 4) PN6
(Example 3) BN6
(Example 3) BN5
(Production Example 2) B
(Comparative Example 3) Polylysine
(Reference Example 8) Linear glucan

*Fig. 4*

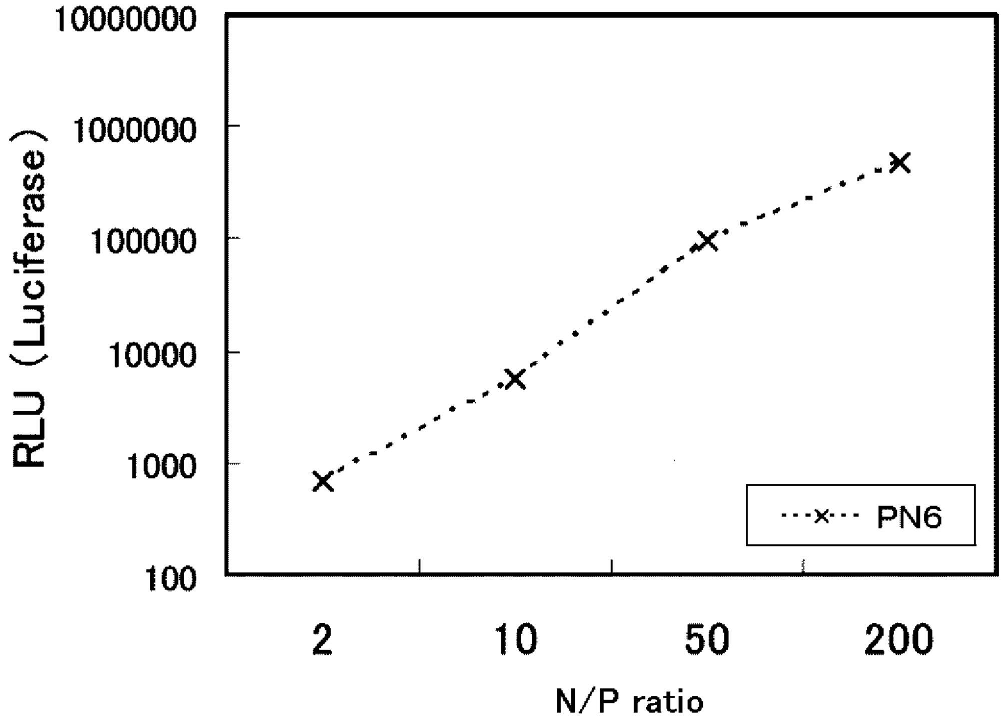
10000000
1000000
100000
10000
1000
100
RLU（Luciferase）
PN6
2
10
50
200
N/P ratio

*Fig.5*

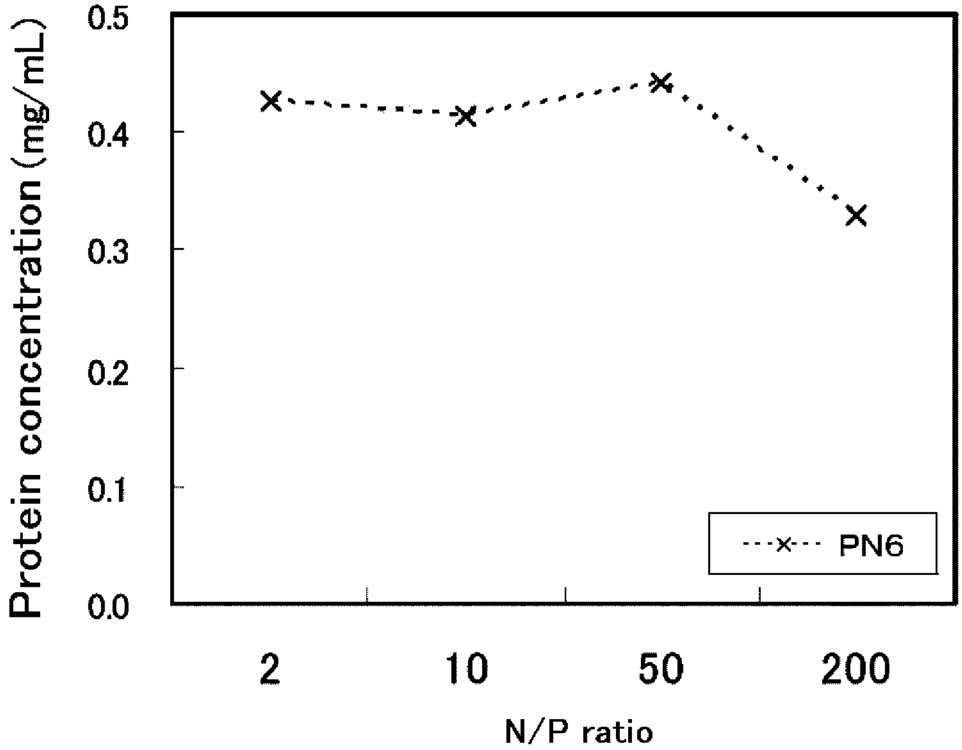
Protein concentration (mg/mL)
0.5
0.4
0.3
0.2
0.1
0.0
PN6
2
10
50
200
N/P ratio

*Fig. 6*

**INTERNATIONAL SEARCH REPORT**

International application No.
PCT/JP2011/006223

A. CLASSIFICATION OF SUBJECT MATTER
*C12P19/26*(2006.01)i, *A61K47/36*(2006.01)i, *C08B37/00*(2006.01)i

According to International Patent Classification (IPC) or to both national classification and IPC

B. FIELDS SEARCHED

Minimum documentation searched (classification system followed by classification symbols)
C12P19/26, A61K47/36, C08B37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/BIOSIS/WPIDS(STN), JSTPlus(JDreamII)

C. DOCUMENTS CONSIDERED TO BE RELEVANT

| Category* | Citation of document, with indication, where appropriate, of the relevant passages | Relevant to claim No. |
|---|---|---|
| Y | Yuta UMEGAYA et al., "Phosphorylase Shokubai ni yoru Mattan ni Glucuronic Acid Zanki o Yusuru Bunkigata Tato no Gosei", Polymer Preprints, Japan, 11 May 2010 (11.05.2010), vol.59, no.1, page 1814 | 1-18 |
| Y | Yuta UMEGAYA et al., "Phosphorylase ni yoru Bunkijo Tato eno Glucuronic Acid Zanki no Ten'i Hanno", The Cellulose Society of Japan Nenji Taikai Koen Yoshishu, 01 July 2010 (01.07.2010), vol.17th, page 44 | 1-18 |
| Y | Jun'ichi KADOKAWA et al., "Kosoteki Glucuronic Acid Zanki no Ten'i Hanno o Riyo shita Anion-sei Tabunki Tato no Gosei", Polymer Preprints, Japan, 01 September 2010 (01.09.2010), vol.59, no.2, page 4875 | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 January, 2012 (25.01.12) | 07 February, 2012 (07.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/006223

C (Continuation). DOCUMENTS CONSIDERED TO BE RELEVANT

| Category* | Citation of document, with indication, where appropriate, of the relevant passages | Relevant to claim No. |
|---|---|---|
| Y | NAWAJI, M et al., Enzymatic alpha-glucosaminylation of maltooligosaccharides catalyzed by phosphorylase, Carbohydr.Res., 2008, Vol.343,No.15, p.2692-2696 | 1-18 |
| A | KAWAZOE, S. et al., Phosphorylase-catalyzed N-formyl-alpha-glucosaminylation of maltooligosaccharides, Carbohydr.Res., 2010.03.30, Vol.345,No.5, p.631-636 | 1-18 |
| A | Satoshi KAWAZOE et al., "Tainetsusei Phosphorylase ni yoru N-formyl Glucosamine Zanki no Maltooligo eno Ten'i Hanno", CSJ: The Chemical Society of Japan Koen Yokoshu, 12 March 2010 (12.03.2010), vol.90th, no.4, page 1032 | 1-18 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4399216 A
- WO 02097107 A
- JP 8134104 A
- JP 3107358 B
- JP 5276883 A
- JP 7241181 A
- WO 01073106 A
- JP 2008121693 A
- JP 2000316581 A
- JP 2004526463 W


### Non-patent literature cited in the description


- Drug delivery using functional DDS carriers. **HIROAKI OKADA.** General Statement: Kinousei DDS carrier wo mochiita seizai sekkei ni yoru soyaku (Drug Discovery by Preparation Design Using Functional DDS Carrier. CMC Publishing Co., Ltd, 2008, 1-23
- **MASAYUKI YOKOYAMA.** Polymeric materials for drug carriers, Special Topic, DDS ni riyou sareru kobunshi kagaku (Polymer Chemistry Utilized in DDS. *Drug Delivery System,* 2009, vol. 23-6, 610-617
- **MARIA LAURA IMMORDINO et al.** *International Journal of Nanomedicine,* 2006, vol. 1 (3), 297-315
- **J. MILTON HARRIS ; ROBERT B. CHESS.** *NATURE REVIEWS, DRUG DISCOVERY,* March 2003, vol. 2, 214-221
- **NAWAJI et al.** *Carbohydr. Res.,* 2008, vol. 343, 2692-2696
- **SAMBROOK, J. et al.** Molecular Cloning: A Laboratory Manual. Cold Spring Harbor, 1989
- **AUSUBEL, F.M.** Current Protocols in Molecular Biology. Greene Pub. Associates and Wiley-Interscience, 1987
- **AUSUBEL, F.M.** Short Protocols in Molecular Biology: A Compendium of Methods from Current Protocols in Molecular Biology. Greene Pub. Associates and Wiley-Interscience, 1989
- **INNIS, M.A.** PCR Protocols: A Guide to Methods and Applications. Academic Press, 1990
- **AUSUBEL, F.M.** Short Protocols in Molecular Biology: A Compendium of Methods from Current Protocols in Molecular Biology. Greene Pub. Associates, 1992
- **AUSUBEL, F.M.** Short Protocols in Molecular Biology: A Compendium of Methods from Current Protocols in Molecular Biology. Greene Pub. Associates, 1995
- **INNIS, M.A. et al.** PCR Strategies. Academic Press, 1995
- **AUSUBEL, F.M.** Short Protocols in Molecular Biology: A Compendium of Methods from Current Protocols in Molecular Biology. Wiley, 1999
- **SNINSKY, J.J. et al.** PCR Applications: Protocols for Functional Genomics. Academic Press, 1999
- Idenshidounyu & Hatsugen kaiseki jikkenhou. **BESSATSU JIKKEN-IGAKU.** Experimental Medicine. Yodosha Co., Ltd, 1997
- **GAIT, M.J.** Oligonucleotide Synthesis: A Practical Approach. IRL Press, 1985
- **GAIT, M.J.** Oligonucleotide Synthesis: A Practical Approach. IRL Press, 1990
- **ECKSTEIN, F.** Oligonucleotides and Analogues: A Practical Approac. IRL Press, 1991
- **ADAMS, R.L. et al.** Biochemistry of the Nucleic Acids. Chapman & Hall, 1992
- **SHABAROVA, Z. et al.** *Advanced Organic Chemistry of Nucleic Acids,* 1994
- **BLACKBURN, G.M. et al.** Nucleic Acids in Chemistry and Biology. Oxford University Press, 1996
- **HERMANSON, G.T.** Bioconjugate Techniques. Academic Press, 1996
- **AUSUBEL F.A. et al.** Current Protocols in Molecular Biology. Wiley, 1988
- **SAMBROOK J et al.** Molecular Cloning: A Laboratory Manual. Cold Spring Harbor Laboratory Press, 1987
- **BESSATSU JIKKEN-IGAKU.** Idenshidounyu & Hatsugen kaiseki jikkenhou. Yodosha Co., Ltd, 1997
- *Methods. Enzymol.,* 1990, vol. 194, 182
- *Proc. Natl. Acad. Sci. USA,* 1978, vol. 84, 1929
- DNA Microarray to Saishin PCR Hou. Saibo Kogaku Bessatsu. December 2002
- *Nat Genet.,* vol. 32, 526-32
- Understand the Basics of Genomics Experiments - Yusuke Nakamura Laboratory Manual. **GENOMU KAISEKI JIKKENHOU.** Nakamura Yusuke lab manual. Yodosha Co., Ltd, 2002

- **TAKEISHI, K. ; GOTOH, O.** Sequence Relationships among Various 4.5 S RNA Species. *J. Biochem.,* 1984, vol. 92, 1173-1177
- **T. J. SCHOCH et al.** *J. American Chemical Society,* 1942, vol. 64, 2957
- **HIZUKURI et al.** *Starch,* 1983, vol. 35, 348-350
- **NAKANO et al.** *Biochemistry,* 1989, vol. 106, 691-695

SEQUENCE LISTING

<110> EZAKI GLICO CO., LTD.

<120> AMINO SUGAR-CONTAINING GLUCAN, METHOD FOR PRODUCING SAME, AND USE THEREOF

<130> EG037PCT

<150> JP 2010-249082
<151> 2010-11-05

<160> 6

<170> PatentIn version 3.4

<210> 1
<211> 2079
<212> DNA
<213> Aquifex aeolicus

<220>
<221> CDS
<222> (1)..(2079)

<400> 1
```
atg gaa gaa gaa aaa gta aaa gag gga ttg tgg gag tta gct tac aac        48
Met Glu Glu Glu Lys Val Lys Glu Gly Leu Trp Glu Leu Ala Tyr Asn
1               5                   10                  15

ctg tgg tgg acg tgg aat ccg ccg gct aag gaa tta ttc aga agc att        96
Leu Trp Trp Thr Trp Asn Pro Pro Ala Lys Glu Leu Phe Arg Ser Ile
            20                  25                  30

gac ccg ctt ttg tgg aag gaa act aag gaa aac ccc att gag tta ttg       144
Asp Pro Leu Leu Trp Lys Glu Thr Lys Glu Asn Pro Ile Glu Leu Leu
        35                  40                  45

agg aaa acc aaa ctc ctt gaa aac aag ctc aaa gac gaa gat ttt ata       192
Arg Lys Thr Lys Leu Leu Glu Asn Lys Leu Lys Asp Glu Asp Phe Ile
    50                  55                  60

tct cac ttc aag tac gtt tat tcc ctt tac aaa acc tac atg aac agg       240
Ser His Phe Lys Tyr Val Tyr Ser Leu Tyr Lys Thr Tyr Met Asn Arg
65                  70                  75                  80

cat tcg aaa tac gag gat acg tat aag aag cct ata gtt ttc ctg tct       288
His Ser Lys Tyr Glu Asp Thr Tyr Lys Lys Pro Ile Val Phe Leu Ser
                85                  90                  95

ccc gag tac gga ctt cac cac aca cta ctt ata tac gcg ggg gga ctg       336
Pro Glu Tyr Gly Leu His His Thr Leu Leu Ile Tyr Ala Gly Gly Leu
            100                 105                 110

ggc ttt tta gca gga gat ata ctc aag gag agc agt gac ttg gga ttt       384
Gly Phe Leu Ala Gly Asp Ile Leu Lys Glu Ser Ser Asp Leu Gly Phe
        115                 120                 125

ccg ctt ata ggt gtc ggg ttt atg tac cct cag ggc tac gta aag cag       432
Pro Leu Ile Gly Val Gly Phe Met Tyr Pro Gln Gly Tyr Val Lys Gln
    130                 135                 140

agg ata agg gtt gac gga tgg cag gaa gac ctt gac gca caa aat caa       480
```

```
Arg Ile Arg Val Asp Gly Trp Gln Glu Asp Leu Asp Ala Gln Asn Gln
145                 150                 155                 160

aag gaa tta atg ccc gtt aaa aaa gtt ctg gac aaa gaa gga aaa tgg      528
Lys Glu Leu Met Pro Val Lys Lys Val Leu Asp Lys Glu Gly Lys Trp
                165                 170                 175

ctc aag tgc tac gtt tac gta agg gat gaa aag gtt tac ttt gga gtc      576
Leu Lys Cys Tyr Val Tyr Val Arg Asp Glu Lys Val Tyr Phe Gly Val
            180                 185                 190

tgg gaa gtt aac gtg gga aag aca aag ctc tac ctt ctt gac acg aac      624
Trp Glu Val Asn Val Gly Lys Thr Lys Leu Tyr Leu Leu Asp Thr Asn
        195                 200                 205

gta gag gaa aat act ccc tgg aac agg gaa ata tcc tca aga ctc tac      672
Val Glu Glu Asn Thr Pro Trp Asn Arg Glu Ile Ser Ser Arg Leu Tyr
    210                 215                 220

gtt ccg gac aaa gac ctg agg tta aga caa cag ata gtt ctt ggt ttt      720
Val Pro Asp Lys Asp Leu Arg Leu Arg Gln Gln Ile Val Leu Gly Phe
225                 230                 235                 240

ggc acc gta ata ctc ctt gaa aag ctg ggc att gat gca gga ggt ttt      768
Gly Thr Val Ile Leu Leu Glu Lys Leu Gly Ile Asp Ala Gly Gly Phe
                245                 250                 255

cac ata aac gaa gat tat ccc tcg ttc gtg ttc ctt gca gaa ata ttt      816
His Ile Asn Glu Asp Tyr Pro Ser Phe Val Phe Leu Ala Glu Ile Phe
            260                 265                 270

aaa ctt cta aaa aaa ggt ctg acc tgg gat aag gcg ata gaa gaa gta      864
Lys Leu Leu Lys Lys Gly Leu Thr Trp Asp Lys Ala Ile Glu Glu Val
        275                 280                 285

aga aag att tct ctc ttt acc acg cac aca cca cta cgg gtt gcc gta      912
Arg Lys Ile Ser Leu Phe Thr Thr His Thr Pro Leu Arg Val Ala Val
    290                 295                 300

aat act tat ccc ttc cac atg ata gag gaa cag ttt cta ttc gtt aag      960
Asn Thr Tyr Pro Phe His Met Ile Glu Glu Gln Phe Leu Phe Val Lys
305                 310                 315                 320

gat gtt tac gga ata gac gta aag aaa gtt ctg gaa ctc gga acg aat     1008
Asp Val Tyr Gly Ile Asp Val Lys Lys Val Leu Glu Leu Gly Thr Asn
                325                 330                 335

cct gaa gac cct tcg gag ggt ttt aac agt acg att atg tcc ctc aga     1056
Pro Glu Asp Pro Ser Glu Gly Phe Asn Ser Thr Ile Met Ser Leu Arg
            340                 345                 350

ctc gca aag tac gta aac gca gtg agt aaa aga cat caa gaa gtt tca     1104
Leu Ala Lys Tyr Val Asn Ala Val Ser Lys Arg His Gln Glu Val Ser
        355                 360                 365

agc aag atg tgg agt ttt tta ttt aaa gaa aag gag aat cca ata gat     1152
Ser Lys Met Trp Ser Phe Leu Phe Lys Glu Lys Glu Asn Pro Ile Asp
    370                 375                 380

tac gta acg aac ggt gtt cac ttt ccc aca tgg att tgt tca gat ttg     1200
Tyr Val Thr Asn Gly Val His Phe Pro Thr Trp Ile Cys Ser Asp Leu
385                 390                 395                 400
```

```
aga aga ctg tac gag gag tat ttg gga gag aac ttt gtg gaa ctt cac      1248
Arg Arg Leu Tyr Glu Glu Tyr Leu Gly Glu Asn Phe Val Glu Leu His
                405                 410                 415

gac cac aag tct ctg tgg gaa tta ata aga gac ata ccc gac gaa gaa      1296
Asp His Lys Ser Leu Trp Glu Leu Ile Arg Asp Ile Pro Asp Glu Glu
            420                 425                 430

ctg tgg gaa tat cac ata aga aat aaa gaa aga ctt att gag cac ata      1344
Leu Trp Glu Tyr His Ile Arg Asn Lys Glu Arg Leu Ile Glu His Ile
        435                 440                 445

aaa gac agg gca agg gaa agg tgg gtc aag gaa aaa gcg gat cct tca      1392
Lys Asp Arg Ala Arg Glu Arg Trp Val Lys Glu Lys Ala Asp Pro Ser
    450                 455                 460

atc ctt atg gcc gaa ggt ctg ttc ctt gat tct gac gtt ctt acg gtc      1440
Ile Leu Met Ala Glu Gly Leu Phe Leu Asp Ser Asp Val Leu Thr Val
465                 470                 475                 480

ggt ttt gcg agg agg atg acc ggt tac aaa aga ccg gat ctt ata ttc      1488
Gly Phe Ala Arg Arg Met Thr Gly Tyr Lys Arg Pro Asp Leu Ile Phe
                485                 490                 495

acg gat gta gaa cgc tta aaa aag ata gtg aat gat tcg gaa aga cct      1536
Thr Asp Val Glu Arg Leu Lys Lys Ile Val Asn Asp Ser Glu Arg Pro
            500                 505                 510

gtt cag ata ata ttc gcg gga aag gct cat ccg gct gat atc gaa ggg      1584
Val Gln Ile Ile Phe Ala Gly Lys Ala His Pro Ala Asp Ile Glu Gly
        515                 520                 525

aaa aag ata atc cag aga ata ttt aac ttt gcg aaa gat ccg gaa ttt      1632
Lys Lys Ile Ile Gln Arg Ile Phe Asn Phe Ala Lys Asp Pro Glu Phe
    530                 535                 540

ggg gga aga ata gct ttc gtt gaa gat tac gac gaa ctc ctt gcc cat      1680
Gly Gly Arg Ile Ala Phe Val Glu Asp Tyr Asp Glu Leu Leu Ala His
545                 550                 555                 560

tac atg gtg agg ggt gtg gac gta tgg ttg aac aac cct ctt cct ccc      1728
Tyr Met Val Arg Gly Val Asp Val Trp Leu Asn Asn Pro Leu Pro Pro
                565                 570                 575

ctt gaa gcc tgc ggg aca agc ggt atg aaa gct tct atg aac gga gtg      1776
Leu Glu Ala Cys Gly Thr Ser Gly Met Lys Ala Ser Met Asn Gly Val
            580                 585                 590

ctt cac ctt tca ata ctt gac ggt tgg tgg att gag ggt tat aac gga      1824
Leu His Leu Ser Ile Leu Asp Gly Trp Trp Ile Glu Gly Tyr Asn Gly
        595                 600                 605

aag aac ggt tgg gct ttc gga gat tac gaa gtt gaa gga gac agg aac      1872
Lys Asn Gly Trp Ala Phe Gly Asp Tyr Glu Val Glu Gly Asp Arg Asn
    610                 615                 620

aga gcg gat gcg gag gcc att tac aac atc ctt gag aat gaa gta atc      1920
Arg Ala Asp Ala Glu Ala Ile Tyr Asn Ile Leu Glu Asn Glu Val Ile
625                 630                 635                 640

ccc ctt tat tac gaa agg gac gag agg gga gtg cca gtt aag tgg ata      1968
Pro Leu Tyr Tyr Glu Arg Asp Glu Arg Gly Val Pro Val Lys Trp Ile
                645                 650                 655
```

```
agt atg atg aag gaa gct ata aaa agc att acc cct aac ttt tgc tcc      2016
Ser Met Met Lys Glu Ala Ile Lys Ser Ile Thr Pro Asn Phe Cys Ser
            660                 665                 670

aga agg atg tta aaa gat tac ata aat aag ttc tat tca aaa att tta      2064
Arg Arg Met Leu Lys Asp Tyr Ile Asn Lys Phe Tyr Ser Lys Ile Leu
        675                 680                 685

aag gag gag gga tga                                                  2079
Lys Glu Glu Gly
    690
```

<210> 2
<211> 692
<212> PRT
<213> Aquifex aeolicus

<400> 2

```
Met Glu Glu Glu Lys Val Lys Glu Gly Leu Trp Glu Leu Ala Tyr Asn
1               5                   10                  15

Leu Trp Trp Thr Trp Asn Pro Pro Ala Lys Glu Leu Phe Arg Ser Ile
            20                  25                  30

Asp Pro Leu Leu Trp Lys Glu Thr Lys Glu Asn Pro Ile Glu Leu Leu
        35                  40                  45

Arg Lys Thr Lys Leu Leu Glu Asn Lys Leu Lys Asp Glu Asp Phe Ile
    50                  55                  60

Ser His Phe Lys Tyr Val Tyr Ser Leu Tyr Lys Thr Tyr Met Asn Arg
65                  70                  75                  80

His Ser Lys Tyr Glu Asp Thr Tyr Lys Lys Pro Ile Val Phe Leu Ser
                85                  90                  95

Pro Glu Tyr Gly Leu His His Thr Leu Leu Ile Tyr Ala Gly Gly Leu
            100                 105                 110

Gly Phe Leu Ala Gly Asp Ile Leu Lys Glu Ser Ser Asp Leu Gly Phe
        115                 120                 125

Pro Leu Ile Gly Val Gly Phe Met Tyr Pro Gln Gly Tyr Val Lys Gln
    130                 135                 140

Arg Ile Arg Val Asp Gly Trp Gln Glu Asp Leu Asp Ala Gln Asn Gln
145                 150                 155                 160

Lys Glu Leu Met Pro Val Lys Lys Val Leu Asp Lys Glu Gly Lys Trp
                165                 170                 175
```

```
Leu Lys Cys Tyr Val Tyr Val Arg Asp Glu Lys Val Tyr Phe Gly Val
            180                 185                 190

Trp Glu Val Asn Val Gly Lys Thr Lys Leu Tyr Leu Leu Asp Thr Asn
        195                 200                 205

Val Glu Glu Asn Thr Pro Trp Asn Arg Glu Ile Ser Ser Arg Leu Tyr
    210                 215                 220

Val Pro Asp Lys Asp Leu Arg Leu Arg Gln Gln Ile Val Leu Gly Phe
225                 230                 235                 240

Gly Thr Val Ile Leu Leu Glu Lys Leu Gly Ile Asp Ala Gly Gly Phe
                245                 250                 255

His Ile Asn Glu Asp Tyr Pro Ser Phe Val Phe Leu Ala Glu Ile Phe
            260                 265                 270

Lys Leu Leu Lys Lys Gly Leu Thr Trp Asp Lys Ala Ile Glu Glu Val
        275                 280                 285

Arg Lys Ile Ser Leu Phe Thr Thr His Thr Pro Leu Arg Val Ala Val
    290                 295                 300

Asn Thr Tyr Pro Phe His Met Ile Glu Glu Gln Phe Leu Phe Val Lys
305                 310                 315                 320

Asp Val Tyr Gly Ile Asp Val Lys Lys Val Leu Glu Leu Gly Thr Asn
                325                 330                 335

Pro Glu Asp Pro Ser Glu Gly Phe Asn Ser Thr Ile Met Ser Leu Arg
            340                 345                 350

Leu Ala Lys Tyr Val Asn Ala Val Ser Lys Arg His Gln Glu Val Ser
        355                 360                 365

Ser Lys Met Trp Ser Phe Leu Phe Lys Glu Lys Glu Asn Pro Ile Asp
    370                 375                 380

Tyr Val Thr Asn Gly Val His Phe Pro Thr Trp Ile Cys Ser Asp Leu
385                 390                 395                 400

Arg Arg Leu Tyr Glu Glu Tyr Leu Gly Glu Asn Phe Val Glu Leu His
                405                 410                 415

Asp His Lys Ser Leu Trp Glu Leu Ile Arg Asp Ile Pro Asp Glu Glu
```

```
            420                 425                 430

Leu Trp Glu Tyr His Ile Arg Asn Lys Glu Arg Leu Ile Glu His Ile
        435                 440                 445

Lys Asp Arg Ala Arg Glu Arg Trp Val Lys Glu Lys Ala Asp Pro Ser
    450                 455                 460

Ile Leu Met Ala Glu Gly Leu Phe Leu Asp Ser Asp Val Leu Thr Val
465                 470                 475                 480

Gly Phe Ala Arg Arg Met Thr Gly Tyr Lys Arg Pro Asp Leu Ile Phe
                485                 490                 495

Thr Asp Val Glu Arg Leu Lys Lys Ile Val Asn Asp Ser Glu Arg Pro
            500                 505                 510

Val Gln Ile Ile Phe Ala Gly Lys Ala His Pro Ala Asp Ile Glu Gly
        515                 520                 525

Lys Lys Ile Ile Gln Arg Ile Phe Asn Phe Ala Lys Asp Pro Glu Phe
    530                 535                 540

Gly Gly Arg Ile Ala Phe Val Glu Asp Tyr Asp Glu Leu Leu Ala His
545                 550                 555                 560

Tyr Met Val Arg Gly Val Asp Val Trp Leu Asn Asn Pro Leu Pro Pro
                565                 570                 575

Leu Glu Ala Cys Gly Thr Ser Gly Met Lys Ala Ser Met Asn Gly Val
            580                 585                 590

Leu His Leu Ser Ile Leu Asp Gly Trp Trp Ile Glu Gly Tyr Asn Gly
        595                 600                 605

Lys Asn Gly Trp Ala Phe Gly Asp Tyr Glu Val Glu Gly Asp Arg Asn
    610                 615                 620

Arg Ala Asp Ala Glu Ala Ile Tyr Asn Ile Leu Glu Asn Glu Val Ile
625                 630                 635                 640

Pro Leu Tyr Tyr Glu Arg Asp Glu Arg Gly Val Pro Val Lys Trp Ile
                645                 650                 655

Ser Met Met Lys Glu Ala Ile Lys Ser Ile Thr Pro Asn Phe Cys Ser
            660                 665                 670
```

```
Arg Arg Met Leu Lys Asp Tyr Ile Asn Lys Phe Tyr Ser Lys Ile Leu
        675                 680                 685


Lys Glu Glu Gly
    690


<210>  3
<211>  2793
<212>  DNA
<213>  Solanum tuberosum


<220>
<221>  CDS
<222>  (1)..(2790)

<220>
<221>  mat_peptide
<222>  (43)..(2790)

<400>  3
atg gct agc atg act ggt gga cag caa atg ggt cgg atc ctc acc ttg        48
Met Ala Ser Met Thr Gly Gly Gln Gln Met Gly Arg Ile Leu Thr Leu
                -10                 -5                  -1  1

agt gag aaa att cac cat ccc att act gaa caa ggt ggt gag agc gac        96
Ser Glu Lys Ile His His Pro Ile Thr Glu Gln Gly Gly Glu Ser Asp
        5                   10                  15

ctg agt tct ttt gct cct gat gcc gca tct att acc tca agt atc aaa       144
Leu Ser Ser Phe Ala Pro Asp Ala Ala Ser Ile Thr Ser Ser Ile Lys
    20                  25                  30

tac cat gca gaa ttc aca cct gta ttc tct cct gaa agg ttt gag ctc       192
Tyr His Ala Glu Phe Thr Pro Val Phe Ser Pro Glu Arg Phe Glu Leu
35                  40                  45                  50

cct aag gca ttc ttt gca aca gct caa agt gtt cgt gat tcg ctc ctt       240
Pro Lys Ala Phe Phe Ala Thr Ala Gln Ser Val Arg Asp Ser Leu Leu
                55                  60                  65

att aat tgg aat gct acg tat gat att tat gaa aag ctg aac atg aag       288
Ile Asn Trp Asn Ala Thr Tyr Asp Ile Tyr Glu Lys Leu Asn Met Lys
            70                  75                  80

caa gcg tac tat cta tcc atg gaa ttt ctg cag ggt aga gca ttg tta       336
Gln Ala Tyr Tyr Leu Ser Met Glu Phe Leu Gln Gly Arg Ala Leu Leu
        85                  90                  95

aat gca att ggt aat ctg gag ctt act ggt gca ttt gcg gaa gct ttg       384
Asn Ala Ile Gly Asn Leu Glu Leu Thr Gly Ala Phe Ala Glu Ala Leu
    100                 105                 110

aaa aac ctt ggc cac aat cta gaa aat gtg gct tct cag gaa cca gat       432
Lys Asn Leu Gly His Asn Leu Glu Asn Val Ala Ser Gln Glu Pro Asp
115                 120                 125                 130

gct gct ctt gga aat ggg ggt ttg gga cgg ctt gct tcc tgt ttt ctg       480
Ala Ala Leu Gly Asn Gly Gly Leu Gly Arg Leu Ala Ser Cys Phe Leu
                135                 140                 145
```

```
gac tct ttg gca aca cta aac tac cca gca tgg ggc tat gga ctt agg      528
Asp Ser Leu Ala Thr Leu Asn Tyr Pro Ala Trp Gly Tyr Gly Leu Arg
            150                 155                 160

tac aag tat ggt tta ttt aag caa cgg att aca aaa gat ggt cag gag      576
Tyr Lys Tyr Gly Leu Phe Lys Gln Arg Ile Thr Lys Asp Gly Gln Glu
        165                 170                 175

gag gtg gct gaa gat tgg ctt gaa att ggc agt cca tgg gaa gtt gtg      624
Glu Val Ala Glu Asp Trp Leu Glu Ile Gly Ser Pro Trp Glu Val Val
    180                 185                 190

agg aat gat gtt tca tat cct atc aaa ttc tat gga aaa gtc tct aca      672
Arg Asn Asp Val Ser Tyr Pro Ile Lys Phe Tyr Gly Lys Val Ser Thr
195                 200                 205                 210

gga tca gat gga aag agg tat tgg att ggt gga gag gat ata aag gca      720
Gly Ser Asp Gly Lys Arg Tyr Trp Ile Gly Gly Glu Asp Ile Lys Ala
                215                 220                 225

gtt gcg tat gat gtt ccc ata cca ggg tat aag acc aga acc aca atc      768
Val Ala Tyr Asp Val Pro Ile Pro Gly Tyr Lys Thr Arg Thr Thr Ile
            230                 235                 240

agc ctt cga ctg tgg tct aca cag gtt cca tca gcg gat ttt gat tta      816
Ser Leu Arg Leu Trp Ser Thr Gln Val Pro Ser Ala Asp Phe Asp Leu
        245                 250                 255

tct gct ttc aat gct gga gag cac acc aaa gca tgt gaa gcc caa gca      864
Ser Ala Phe Asn Ala Gly Glu His Thr Lys Ala Cys Glu Ala Gln Ala
    260                 265                 270

aac gct gag aag ata tgt tac ata ctc tac cct ggg gat gaa tca gag      912
Asn Ala Glu Lys Ile Cys Tyr Ile Leu Tyr Pro Gly Asp Glu Ser Glu
275                 280                 285                 290

gag gga aag atc ctt cgg ttg aag caa caa tat acc tta tgc tcg gct      960
Glu Gly Lys Ile Leu Arg Leu Lys Gln Gln Tyr Thr Leu Cys Ser Ala
                295                 300                 305

tct ctc caa gat att att tct cga ttt gag agg aga tca ggt gat cgt     1008
Ser Leu Gln Asp Ile Ile Ser Arg Phe Glu Arg Arg Ser Gly Asp Arg
            310                 315                 320

att aag tgg gaa gag ttt cct gaa aaa gtt gct gtg cag atg aat gac     1056
Ile Lys Trp Glu Glu Phe Pro Glu Lys Val Ala Val Gln Met Asn Asp
        325                 330                 335

act cac cct aca ctt tgt atc cct gag ctg atg aga ata ttg ata gat     1104
Thr His Pro Thr Leu Cys Ile Pro Glu Leu Met Arg Ile Leu Ile Asp
    340                 345                 350

ctg aag ggc ttg aat tgg aat gaa gct tgg aat att act caa aga act     1152
Leu Lys Gly Leu Asn Trp Asn Glu Ala Trp Asn Ile Thr Gln Arg Thr
355                 360                 365                 370

gtg gcc tac aca aac cat act gtt ttg cct gag gca ctg gag aaa tgg     1200
Val Ala Tyr Thr Asn His Thr Val Leu Pro Glu Ala Leu Glu Lys Trp
                375                 380                 385

agt tat gaa ttg atg cag aaa ctc ctt ccc aga cat gtc gaa atc att     1248
Ser Tyr Glu Leu Met Gln Lys Leu Leu Pro Arg His Val Glu Ile Ile
            390                 395                 400
```

```
gag gcg att gac gag gag ctg gta cat gaa att gta tta aaa tat ggt      1296
Glu Ala Ile Asp Glu Glu Leu Val His Glu Ile Val Leu Lys Tyr Gly
        405                 410                 415

tca atg gat ctg aac aaa ttg gag gaa aag ttg act aca atg aga atc      1344
Ser Met Asp Leu Asn Lys Leu Glu Glu Lys Leu Thr Thr Met Arg Ile
    420                 425                 430

tta gaa aat ttt gat ctt ccc agt tct gtt gct gaa tta ttt att aag      1392
Leu Glu Asn Phe Asp Leu Pro Ser Ser Val Ala Glu Leu Phe Ile Lys
435                 440                 445                 450

cct gaa atc tca gtt gat gat gat act gaa aca gta gaa gtc cat gac      1440
Pro Glu Ile Ser Val Asp Asp Asp Thr Glu Thr Val Glu Val His Asp
                455                 460                 465

aaa gtt gaa gct tcc gat aaa gtt gtg act aat gat gaa gat gac act      1488
Lys Val Glu Ala Ser Asp Lys Val Val Thr Asn Asp Glu Asp Asp Thr
            470                 475                 480

ggt aag aaa act agt gtg aag ata gaa gca gct gca gaa aaa gac att      1536
Gly Lys Lys Thr Ser Val Lys Ile Glu Ala Ala Ala Glu Lys Asp Ile
        485                 490                 495

gac aag aaa act ccc gtg agt ccg gaa cca gct gtt ata cca cct aag      1584
Asp Lys Lys Thr Pro Val Ser Pro Glu Pro Ala Val Ile Pro Pro Lys
    500                 505                 510

aag gta cgc atg gcc aac ttg tgt gtt gtg ggc ggc cat gct gtt aat      1632
Lys Val Arg Met Ala Asn Leu Cys Val Val Gly Gly His Ala Val Asn
515                 520                 525                 530

gga gtt gct gag atc cat agt gaa att gtg aag gag gag gtt ttc aat      1680
Gly Val Ala Glu Ile His Ser Glu Ile Val Lys Glu Glu Val Phe Asn
                535                 540                 545

gac ttc tat gag ctc tgg ccg gaa aag ttc caa aac aaa aca aat gga      1728
Asp Phe Tyr Glu Leu Trp Pro Glu Lys Phe Gln Asn Lys Thr Asn Gly
            550                 555                 560

gtg act cca aga aga tgg att cgt ttc tgc aat cct cct ctt agt gcc      1776
Val Thr Pro Arg Arg Trp Ile Arg Phe Cys Asn Pro Pro Leu Ser Ala
        565                 570                 575

atc ata act aag tgg act ggt aca gag gat tgg gtc ctg aaa act gaa      1824
Ile Ile Thr Lys Trp Thr Gly Thr Glu Asp Trp Val Leu Lys Thr Glu
    580                 585                 590

aag ttg gca gaa ttg cag aag ttt gct gat aat gaa gat ctt caa aat      1872
Lys Leu Ala Glu Leu Gln Lys Phe Ala Asp Asn Glu Asp Leu Gln Asn
595                 600                 605                 610

gag tgg agg gaa gca aaa agg agc aac aag att aaa gtt gtc tcc ttt      1920
Glu Trp Arg Glu Ala Lys Arg Ser Asn Lys Ile Lys Val Val Ser Phe
                615                 620                 625

ctc aaa gaa aag aca ggg tat tct gtt gtc cca gat gca atg ttt gat      1968
Leu Lys Glu Lys Thr Gly Tyr Ser Val Val Pro Asp Ala Met Phe Asp
            630                 635                 640

att cag gta aaa cgc att cat gag tac aag cga caa ctg tta aat atc      2016
Ile Gln Val Lys Arg Ile His Glu Tyr Lys Arg Gln Leu Leu Asn Ile
```

```
        645                 650                 655

ttc ggc atc gtt tat cgg tat aag aag atg aaa gaa atg aca gct gca      2064
Phe Gly Ile Val Tyr Arg Tyr Lys Lys Met Lys Glu Met Thr Ala Ala
    660                 665                 670

gaa aga aag act aac ttc gtt cct cga gta tgc ata ttt ggg gga aaa      2112
Glu Arg Lys Thr Asn Phe Val Pro Arg Val Cys Ile Phe Gly Gly Lys
675                 680                 685                 690

gct ttt gcc aca tat gtg caa gcc aag agg att gta aaa ttt atc aca      2160
Ala Phe Ala Thr Tyr Val Gln Ala Lys Arg Ile Val Lys Phe Ile Thr
                695                 700                 705

gat gtt ggt gct act ata aat cat gat cca gaa atc ggt gat ctg ttg      2208
Asp Val Gly Ala Thr Ile Asn His Asp Pro Glu Ile Gly Asp Leu Leu
            710                 715                 720

aag gta gtc ttt gtg cca gat tac aat gtc agt gtt gct gaa ttg cta      2256
Lys Val Val Phe Val Pro Asp Tyr Asn Val Ser Val Ala Glu Leu Leu
        725                 730                 735

att cct gct agc gat cta tca gaa cat atc agt acg gct gga atg gag      2304
Ile Pro Ala Ser Asp Leu Ser Glu His Ile Ser Thr Ala Gly Met Glu
    740                 745                 750

gcc agt gga acc agt aat atg aag ttt gca atg aat ggt tgt atc caa      2352
Ala Ser Gly Thr Ser Asn Met Lys Phe Ala Met Asn Gly Cys Ile Gln
755                 760                 765                 770

att ggt aca ttg gat ggc gct aat gtt gaa ata agg gaa gag gtt gga      2400
Ile Gly Thr Leu Asp Gly Ala Asn Val Glu Ile Arg Glu Glu Val Gly
                775                 780                 785

gaa gaa aac ttc ttt ctc ttt ggt gct caa gct cat gaa att gca ggg      2448
Glu Glu Asn Phe Phe Leu Phe Gly Ala Gln Ala His Glu Ile Ala Gly
            790                 795                 800

ctt aga aaa gaa aga gct gac gga aag ttt gta cct gat gaa cgt ttt      2496
Leu Arg Lys Glu Arg Ala Asp Gly Lys Phe Val Pro Asp Glu Arg Phe
        805                 810                 815

gaa gag gtg aag gaa ttt gtt aga agc ggt gct ttt ggc tct tat aac      2544
Glu Glu Val Lys Glu Phe Val Arg Ser Gly Ala Phe Gly Ser Tyr Asn
    820                 825                 830

tat gat gac cta att gga tcg ttg gaa gga aat gaa ggt ttt ggc cgt      2592
Tyr Asp Asp Leu Ile Gly Ser Leu Glu Gly Asn Glu Gly Phe Gly Arg
835                 840                 845                 850

gct gac tat ttc ctt gtg ggc aag gac ttc ccc agt tac ata gaa tgc      2640
Ala Asp Tyr Phe Leu Val Gly Lys Asp Phe Pro Ser Tyr Ile Glu Cys
                855                 860                 865

caa gag aaa gtt gat gag gca tat cgc gac cag aaa agg tgg aca acg      2688
Gln Glu Lys Val Asp Glu Ala Tyr Arg Asp Gln Lys Arg Trp Thr Thr
            870                 875                 880

atg tca atc ttg aat aca gcg gga tcg tac aag ttc agc agt gac aga      2736
Met Ser Ile Leu Asn Thr Ala Gly Ser Tyr Lys Phe Ser Ser Asp Arg
        885                 890                 895

aca atc cat gaa tat gcc aaa gac att tgg aac att gaa gct gtg gaa      2784
```

```
Thr Ile His Glu Tyr Ala Lys Asp Ile Trp Asn Ile Glu Ala Val Glu
    900                 905                 910

ata gca taa                                                         2793
Ile Ala
915


<210>  4
<211>  930
<212>  PRT
<213>  Solanum tuberosum

<400>  4

Met Ala Ser Met Thr Gly Gly Gln Gln Met Gly Arg Ile Leu Thr Leu
                -10                 -5                  -1  1

Ser Glu Lys Ile His His Pro Ile Thr Glu Gln Gly Gly Glu Ser Asp
        5                   10                  15

Leu Ser Ser Phe Ala Pro Asp Ala Ala Ser Ile Thr Ser Ser Ile Lys
    20                  25                  30

Tyr His Ala Glu Phe Thr Pro Val Phe Ser Pro Glu Arg Phe Glu Leu
35                  40                  45                  50

Pro Lys Ala Phe Phe Ala Thr Ala Gln Ser Val Arg Asp Ser Leu Leu
                55                  60                  65

Ile Asn Trp Asn Ala Thr Tyr Asp Ile Tyr Glu Lys Leu Asn Met Lys
            70                  75                  80

Gln Ala Tyr Tyr Leu Ser Met Glu Phe Leu Gln Gly Arg Ala Leu Leu
        85                  90                  95

Asn Ala Ile Gly Asn Leu Glu Leu Thr Gly Ala Phe Ala Glu Ala Leu
    100                 105                 110

Lys Asn Leu Gly His Asn Leu Glu Asn Val Ala Ser Gln Glu Pro Asp
115                 120                 125                 130

Ala Ala Leu Gly Asn Gly Gly Leu Gly Arg Leu Ala Ser Cys Phe Leu
                135                 140                 145

Asp Ser Leu Ala Thr Leu Asn Tyr Pro Ala Trp Gly Tyr Gly Leu Arg
            150                 155                 160

Tyr Lys Tyr Gly Leu Phe Lys Gln Arg Ile Thr Lys Asp Gly Gln Glu
        165                 170                 175
```

```
Glu Val Ala Glu Asp Trp Leu Glu Ile Gly Ser Pro Trp Glu Val Val
    180                 185                 190

Arg Asn Asp Val Ser Tyr Pro Ile Lys Phe Tyr Gly Lys Val Ser Thr
195                 200                 205                 210

Gly Ser Asp Gly Lys Arg Tyr Trp Ile Gly Gly Glu Asp Ile Lys Ala
                215                 220                 225

Val Ala Tyr Asp Val Pro Ile Pro Gly Tyr Lys Thr Arg Thr Thr Ile
            230                 235                 240

Ser Leu Arg Leu Trp Ser Thr Gln Val Pro Ser Ala Asp Phe Asp Leu
        245                 250                 255

Ser Ala Phe Asn Ala Gly Glu His Thr Lys Ala Cys Glu Ala Gln Ala
    260                 265                 270

Asn Ala Glu Lys Ile Cys Tyr Ile Leu Tyr Pro Gly Asp Glu Ser Glu
275                 280                 285                 290

Glu Gly Lys Ile Leu Arg Leu Lys Gln Gln Tyr Thr Leu Cys Ser Ala
                295                 300                 305

Ser Leu Gln Asp Ile Ile Ser Arg Phe Glu Arg Arg Ser Gly Asp Arg
            310                 315                 320

Ile Lys Trp Glu Glu Phe Pro Glu Lys Val Ala Val Gln Met Asn Asp
        325                 330                 335

Thr His Pro Thr Leu Cys Ile Pro Glu Leu Met Arg Ile Leu Ile Asp
    340                 345                 350

Leu Lys Gly Leu Asn Trp Asn Glu Ala Trp Asn Ile Thr Gln Arg Thr
355                 360                 365                 370

Val Ala Tyr Thr Asn His Thr Val Leu Pro Glu Ala Leu Glu Lys Trp
                375                 380                 385

Ser Tyr Glu Leu Met Gln Lys Leu Leu Pro Arg His Val Glu Ile Ile
            390                 395                 400

Glu Ala Ile Asp Glu Glu Leu Val His Glu Ile Val Leu Lys Tyr Gly
        405                 410                 415

Ser Met Asp Leu Asn Lys Leu Glu Glu Lys Leu Thr Thr Met Arg Ile
    420                 425                 430
```

```
Leu Glu Asn Phe Asp Leu Pro Ser Ser Val Ala Glu Leu Phe Ile Lys
435                 440                 445                 450

Pro Glu Ile Ser Val Asp Asp Asp Thr Glu Thr Val Glu Val His Asp
                455                 460                 465

Lys Val Glu Ala Ser Asp Lys Val Val Thr Asn Asp Glu Asp Asp Thr
            470                 475                 480

Gly Lys Lys Thr Ser Val Lys Ile Glu Ala Ala Ala Glu Lys Asp Ile
        485                 490                 495

Asp Lys Lys Thr Pro Val Ser Pro Glu Pro Ala Val Ile Pro Pro Lys
    500                 505                 510

Lys Val Arg Met Ala Asn Leu Cys Val Val Gly Gly His Ala Val Asn
515                 520                 525                 530

Gly Val Ala Glu Ile His Ser Glu Ile Val Lys Glu Glu Val Phe Asn
                535                 540                 545

Asp Phe Tyr Glu Leu Trp Pro Glu Lys Phe Gln Asn Lys Thr Asn Gly
            550                 555                 560

Val Thr Pro Arg Arg Trp Ile Arg Phe Cys Asn Pro Pro Leu Ser Ala
        565                 570                 575

Ile Ile Thr Lys Trp Thr Gly Thr Glu Asp Trp Val Leu Lys Thr Glu
    580                 585                 590

Lys Leu Ala Glu Leu Gln Lys Phe Ala Asp Asn Glu Asp Leu Gln Asn
595                 600                 605                 610

Glu Trp Arg Glu Ala Lys Arg Ser Asn Lys Ile Lys Val Val Ser Phe
                615                 620                 625

Leu Lys Glu Lys Thr Gly Tyr Ser Val Val Pro Asp Ala Met Phe Asp
            630                 635                 640

Ile Gln Val Lys Arg Ile His Glu Tyr Lys Arg Gln Leu Leu Asn Ile
        645                 650                 655

Phe Gly Ile Val Tyr Arg Tyr Lys Lys Met Lys Glu Met Thr Ala Ala
    660                 665                 670

Glu Arg Lys Thr Asn Phe Val Pro Arg Val Cys Ile Phe Gly Gly Lys
675                 680                 685                 690
```

```
Ala Phe Ala Thr Tyr Val Gln Ala Lys Arg Ile Val Lys Phe Ile Thr
                695                 700                 705

Asp Val Gly Ala Thr Ile Asn His Asp Pro Glu Ile Gly Asp Leu Leu
            710                 715                 720

Lys Val Val Phe Val Pro Asp Tyr Asn Val Ser Val Ala Glu Leu Leu
        725                 730                 735

Ile Pro Ala Ser Asp Leu Ser Glu His Ile Ser Thr Ala Gly Met Glu
    740                 745                 750

Ala Ser Gly Thr Ser Asn Met Lys Phe Ala Met Asn Gly Cys Ile Gln
755                 760                 765                 770

Ile Gly Thr Leu Asp Gly Ala Asn Val Glu Ile Arg Glu Glu Val Gly
                775                 780                 785

Glu Glu Asn Phe Phe Leu Phe Gly Ala Gln Ala His Glu Ile Ala Gly
            790                 795                 800

Leu Arg Lys Glu Arg Ala Asp Gly Lys Phe Val Pro Asp Glu Arg Phe
        805                 810                 815

Glu Glu Val Lys Glu Phe Val Arg Ser Gly Ala Phe Gly Ser Tyr Asn
    820                 825                 830

Tyr Asp Asp Leu Ile Gly Ser Leu Glu Gly Asn Glu Gly Phe Gly Arg
835                 840                 845                 850

Ala Asp Tyr Phe Leu Val Gly Lys Asp Phe Pro Ser Tyr Ile Glu Cys
                855                 860                 865

Gln Glu Lys Val Asp Glu Ala Tyr Arg Asp Gln Lys Arg Trp Thr Thr
            870                 875                 880

Met Ser Ile Leu Asn Thr Ala Gly Ser Tyr Lys Phe Ser Ser Asp Arg
        885                 890                 895

Thr Ile His Glu Tyr Ala Lys Asp Ile Trp Asn Ile Glu Ala Val Glu
    900                 905                 910

Ile Ala
915

<210>  5
```

```
<211>  2151
<212>  DNA
<213>  Thermococcus zilligii


<220>
<221>  CDS
<222>  (1)..(2151)

<400>  5
atg gcg gac gtt tcc cac aca gtt gag aat tta ata agg gcg aag ctc        48
Met Ala Asp Val Ser His Thr Val Glu Asn Leu Ile Arg Ala Lys Leu
1               5                   10                  15

ccc tac ccg ctg gag aat ctc gca gaa ctg gcc tat aac tac tgg tgg        96
Pro Tyr Pro Leu Glu Asn Leu Ala Glu Leu Ala Tyr Asn Tyr Trp Trp
            20                  25                  30

agc tgg aac aga cgt gcc acc cgg ctc tgg gag tac ata gac tcc gaa       144
Ser Trp Asn Arg Arg Ala Thr Arg Leu Trp Glu Tyr Ile Asp Ser Glu
        35                  40                  45

cac tgg cgt gag tac aag aac ccg gtc aag ctc ctt ctt gac acg ccc       192
His Trp Arg Glu Tyr Lys Asn Pro Val Lys Leu Leu Leu Asp Thr Pro
    50                  55                  60

gaa gag agg ttc tgg gag ctc ctc aag gat gac gac ttc atg aac ctc       240
Glu Glu Arg Phe Trp Glu Leu Leu Lys Asp Asp Asp Phe Met Asn Leu
65                  70                  75                  80

tac gag ctc gtt atg gac cag ttt acc gca tac atg aac ccg aaa tca       288
Tyr Glu Leu Val Met Asp Gln Phe Thr Ala Tyr Met Asn Pro Lys Ser
                85                  90                  95

acg tgg ttc tcg acc aac tac ccc aaa tgg gac aaa ccg ata gtc tat       336
Thr Trp Phe Ser Thr Asn Tyr Pro Lys Trp Asp Lys Pro Ile Val Tyr
            100                 105                 110

ctc tgc atg gag tac ggc ata agc aag agc ctc ccg att tac tcc ggt       384
Leu Cys Met Glu Tyr Gly Ile Ser Lys Ser Leu Pro Ile Tyr Ser Gly
        115                 120                 125

ggc ctc gga atc ctc gcc ggg gat cac ctc aaa acc gcg agt gac ctc       432
Gly Leu Gly Ile Leu Ala Gly Asp His Leu Lys Thr Ala Ser Asp Leu
    130                 135                 140

ggc ctt ccc ttc ata gct ata ggc ctc ctc tac aag cac ggc tac ttc       480
Gly Leu Pro Phe Ile Ala Ile Gly Leu Leu Tyr Lys His Gly Tyr Phe
145                 150                 155                 160

agg cag gag ata gac agg gac gga agg cag agg gag ata ttc ccg gaa       528
Arg Gln Glu Ile Asp Arg Asp Gly Arg Gln Arg Glu Ile Phe Pro Glu
                165                 170                 175

tac agg ccc gag gaa atg ccg ata aag cct gtt cta agc aaa gac gga       576
Tyr Arg Pro Glu Glu Met Pro Ile Lys Pro Val Leu Ser Lys Asp Gly
            180                 185                 190

aaa ccc cta ctc gtt gag gtc ccc att gaa aac agg atc gtc tac gcg       624
Lys Pro Leu Leu Val Glu Val Pro Ile Glu Asn Arg Ile Val Tyr Ala
        195                 200                 205

agg gcc ttt gag gtc agc gtc ggc atg gcc aag ctc tac ctc ctt gac       672
```

```
Arg Ala Phe Glu Val Ser Val Gly Met Ala Lys Leu Tyr Leu Leu Asp
    210                 215                 220

aca gat gtg ccc gag aac agc ccg gag gat agg gcg atc tgc gac tac      720
Thr Asp Val Pro Glu Asn Ser Pro Glu Asp Arg Ala Ile Cys Asp Tyr
225                 230                 235                 240

ctc tac aac gcc gag atg gac aag aga ata aaa cag gag atc ctc ctc      768
Leu Tyr Asn Ala Glu Met Asp Lys Arg Ile Lys Gln Glu Ile Leu Leu
                245                 250                 255

gga ata ggc gga atg aga ctg ctc cag gcc ctc ggc atc gag ccc ggt      816
Gly Ile Gly Gly Met Arg Leu Leu Gln Ala Leu Gly Ile Glu Pro Gly
            260                 265                 270

gtg gtt cac ctc aac gag ggg cat ccc gcc ttt gcc aac ctc cag aga      864
Val Val His Leu Asn Glu Gly His Pro Ala Phe Ala Asn Leu Gln Arg
        275                 280                 285

ata gcc tgg tac atg gaa aaa ggc ctc aca ttc acg gaa gct ttg agc      912
Ile Ala Trp Tyr Met Glu Lys Gly Leu Thr Phe Thr Glu Ala Leu Ser
    290                 295                 300

atc gtc agg gga acc acg gtt ttc acc acc cac acg ccg gtt cct gca      960
Ile Val Arg Gly Thr Thr Val Phe Thr Thr His Thr Pro Val Pro Ala
305                 310                 315                 320

ggc cac gac agg ttc ccg atc gcg gaa gtt agg aag agg ctt tca aaa     1008
Gly His Asp Arg Phe Pro Ile Ala Glu Val Arg Lys Arg Leu Ser Lys
                325                 330                 335

ttc ctc gag gga agg gag gag cta ctc gaa ctc ggc cgc gag ggc gac     1056
Phe Leu Glu Gly Arg Glu Glu Leu Leu Glu Leu Gly Arg Glu Gly Asp
            340                 345                 350

cag atc aac atg acc ctg ctt gca ata aga act tcg agc tac gtc aac     1104
Gln Ile Asn Met Thr Leu Leu Ala Ile Arg Thr Ser Ser Tyr Val Asn
        355                 360                 365

ggc gta agc cag ctc cac gcg gag gta agc aag cgc atg tgg aaa gac     1152
Gly Val Ser Gln Leu His Ala Glu Val Ser Lys Arg Met Trp Lys Asp
    370                 375                 380

ctc tgg ccc gga gtt ccc ctc aac gag atc cct ata gag ggc atc aca     1200
Leu Trp Pro Gly Val Pro Leu Asn Glu Ile Pro Ile Glu Gly Ile Thr
385                 390                 395                 400

aac ggc gtc cac aca atg acg tgg gtc cac gac gag atg agg aag ctc     1248
Asn Gly Val His Thr Met Thr Trp Val His Asp Glu Met Arg Lys Leu
                405                 410                 415

ttc gac cgc tac atc ggc aag gtc tgg agg gag cac acc aac ata gag     1296
Phe Asp Arg Tyr Ile Gly Lys Val Trp Arg Glu His Thr Asn Ile Glu
            420                 425                 430

ggc atc tgg tac gcc gtg gaa agg atc ccg gat gaa gag ctc tgg gga     1344
Gly Ile Trp Tyr Ala Val Glu Arg Ile Pro Asp Glu Glu Leu Trp Gly
        435                 440                 445

gcc cac ctc gag gcc aag aga cag ctc ata gag ttc ctc agg gag aag     1392
Ala His Leu Glu Ala Lys Arg Gln Leu Ile Glu Phe Leu Arg Glu Lys
    450                 455                 460
```

```
aca atg gag agg aac agg agg ctc gga acc gat gac ccg ata ccg gag      1440
Thr Met Glu Arg Asn Arg Arg Leu Gly Thr Asp Asp Pro Ile Pro Glu
465                 470                 475                 480

ata gac gag aac gcc ctc ata atc ggc ttt gcc aga cgc ttt gcg acc      1488
Ile Asp Glu Asn Ala Leu Ile Ile Gly Phe Ala Arg Arg Phe Ala Thr
                485                 490                 495

tat aag agg gca act ctc atc ctg agc gac ctt gag agg ctg aag aaa      1536
Tyr Lys Arg Ala Thr Leu Ile Leu Ser Asp Leu Glu Arg Leu Lys Lys
            500                 505                 510

atc ctc aac aac cca gaa agg ccg gtt tac ata gtc ttc ggc ggg aag      1584
Ile Leu Asn Asn Pro Glu Arg Pro Val Tyr Ile Val Phe Gly Gly Lys
        515                 520                 525

gcc cat ccg cgc gac gag gct ggg aag gag ttc ctg agg agg gtt tac      1632
Ala His Pro Arg Asp Glu Ala Gly Lys Glu Phe Leu Arg Arg Val Tyr
    530                 535                 540

gag gtc agc cag atg ccg gag ttc agg ggc aag ata ttc gtc ctc gag      1680
Glu Val Ser Gln Met Pro Glu Phe Arg Gly Lys Ile Phe Val Leu Glu
545                 550                 555                 560

aac tac gac atg ggg agc gcg agg ctc atg gtc gct gga gtc gac gtc      1728
Asn Tyr Asp Met Gly Ser Ala Arg Leu Met Val Ala Gly Val Asp Val
                565                 570                 575

tgg ctc aac aac ccg cgc aga ccg ctt gaa gca agc ggg acg agc ggc      1776
Trp Leu Asn Asn Pro Arg Arg Pro Leu Glu Ala Ser Gly Thr Ser Gly
            580                 585                 590

atg aag gcc ggc ctc aac ggt gtc ctc aac gcg agc atc ttc gac gga      1824
Met Lys Ala Gly Leu Asn Gly Val Leu Asn Ala Ser Ile Phe Asp Gly
        595                 600                 605

tgg tgg gtt gaa ggc tac aat ggg aaa aac ggc tgg gtc att ggg gag      1872
Trp Trp Val Glu Gly Tyr Asn Gly Lys Asn Gly Trp Val Ile Gly Glu
    610                 615                 620

gag acc acc gag ccg gag agc gaa gag gac gac gcg aag gac gct gag      1920
Glu Thr Thr Glu Pro Glu Ser Glu Glu Asp Asp Ala Lys Asp Ala Glu
625                 630                 635                 640

agc ctc tac acc ctg ctc gag aag gag ata atc ccc acc tac tac ggg      1968
Ser Leu Tyr Thr Leu Leu Glu Lys Glu Ile Ile Pro Thr Tyr Tyr Gly
                645                 650                 655

aac agg agc cgc tgg ata tac atg atg aag gag agc ata aag agc ata      2016
Asn Arg Ser Arg Trp Ile Tyr Met Met Lys Glu Ser Ile Lys Ser Ile
            660                 665                 670

gcc ccg cgc ttc agc acg cac agg atg gtc aag gag tac atg gac agg      2064
Ala Pro Arg Phe Ser Thr His Arg Met Val Lys Glu Tyr Met Asp Arg
        675                 680                 685

ttc tac tcc aag gcc atg agc aac tac atc tgg ctc acg agg gag aac      2112
Phe Tyr Ser Lys Ala Met Ser Asn Tyr Ile Trp Leu Thr Arg Glu Asn
    690                 695                 700

tac aag ggg gcc agg gaa ata gcg gcc tgg aag gag agg                  2151
Tyr Lys Gly Ala Arg Glu Ile Ala Ala Trp Lys Glu Arg
705                 710                 715
```

<210> 6
<211> 717
<212> PRT
<213> Thermococcus zilligii

<400> 6

```
Met Ala Asp Val Ser His Thr Val Glu Asn Leu Ile Arg Ala Lys Leu
1               5                   10                  15

Pro Tyr Pro Leu Glu Asn Leu Ala Glu Leu Ala Tyr Asn Tyr Trp Trp
            20                  25                  30

Ser Trp Asn Arg Arg Ala Thr Arg Leu Trp Glu Tyr Ile Asp Ser Glu
        35                  40                  45

His Trp Arg Glu Tyr Lys Asn Pro Val Lys Leu Leu Leu Asp Thr Pro
    50                  55                  60

Glu Glu Arg Phe Trp Glu Leu Leu Lys Asp Asp Asp Phe Met Asn Leu
65                  70                  75                  80

Tyr Glu Leu Val Met Asp Gln Phe Thr Ala Tyr Met Asn Pro Lys Ser
                85                  90                  95

Thr Trp Phe Ser Thr Asn Tyr Pro Lys Trp Asp Lys Pro Ile Val Tyr
            100                 105                 110

Leu Cys Met Glu Tyr Gly Ile Ser Lys Ser Leu Pro Ile Tyr Ser Gly
        115                 120                 125

Gly Leu Gly Ile Leu Ala Gly Asp His Leu Lys Thr Ala Ser Asp Leu
    130                 135                 140

Gly Leu Pro Phe Ile Ala Ile Gly Leu Leu Tyr Lys His Gly Tyr Phe
145                 150                 155                 160

Arg Gln Glu Ile Asp Arg Asp Gly Arg Gln Arg Glu Ile Phe Pro Glu
                165                 170                 175

Tyr Arg Pro Glu Glu Met Pro Ile Lys Pro Val Leu Ser Lys Asp Gly
            180                 185                 190

Lys Pro Leu Leu Val Glu Val Pro Ile Glu Asn Arg Ile Val Tyr Ala
        195                 200                 205

Arg Ala Phe Glu Val Ser Val Gly Met Ala Lys Leu Tyr Leu Leu Asp
    210                 215                 220
```

Thr Asp Val Pro Glu Asn Ser Pro Glu Asp Arg Ala Ile Cys Asp Tyr
225 230 235 240

Leu Tyr Asn Ala Glu Met Asp Lys Arg Ile Lys Gln Glu Ile Leu Leu
245 250 255

Gly Ile Gly Gly Met Arg Leu Leu Gln Ala Leu Gly Ile Glu Pro Gly
260 265 270

Val Val His Leu Asn Glu Gly His Pro Ala Phe Ala Asn Leu Gln Arg
275 280 285

Ile Ala Trp Tyr Met Glu Lys Gly Leu Thr Phe Thr Glu Ala Leu Ser
290 295 300

Ile Val Arg Gly Thr Thr Val Phe Thr Thr His Thr Pro Val Pro Ala
305 310 315 320

Gly His Asp Arg Phe Pro Ile Ala Glu Val Arg Lys Arg Leu Ser Lys
325 330 335

Phe Leu Glu Gly Arg Glu Glu Leu Leu Glu Leu Gly Arg Glu Gly Asp
340 345 350

Gln Ile Asn Met Thr Leu Leu Ala Ile Arg Thr Ser Ser Tyr Val Asn
355 360 365

Gly Val Ser Gln Leu His Ala Glu Val Ser Lys Arg Met Trp Lys Asp
370 375 380

Leu Trp Pro Gly Val Pro Leu Asn Glu Ile Pro Ile Glu Gly Ile Thr
385 390 395 400

Asn Gly Val His Thr Met Thr Trp Val His Asp Glu Met Arg Lys Leu
405 410 415

Phe Asp Arg Tyr Ile Gly Lys Val Trp Arg Glu His Thr Asn Ile Glu
420 425 430

Gly Ile Trp Tyr Ala Val Glu Arg Ile Pro Asp Glu Glu Leu Trp Gly
435 440 445

Ala His Leu Glu Ala Lys Arg Gln Leu Ile Glu Phe Leu Arg Glu Lys
450 455 460

Thr Met Glu Arg Asn Arg Arg Leu Gly Thr Asp Asp Pro Ile Pro Glu

465 470 475 480

Ile Asp Glu Asn Ala Leu Ile Ile Gly Phe Ala Arg Arg Phe Ala Thr
485 490 495

Tyr Lys Arg Ala Thr Leu Ile Leu Ser Asp Leu Glu Arg Leu Lys Lys
500 505 510

Ile Leu Asn Asn Pro Glu Arg Pro Val Tyr Ile Val Phe Gly Gly Lys
515 520 525

Ala His Pro Arg Asp Glu Ala Gly Lys Glu Phe Leu Arg Arg Val Tyr
530 535 540

Glu Val Ser Gln Met Pro Glu Phe Arg Gly Lys Ile Phe Val Leu Glu
545 550 555 560

Asn Tyr Asp Met Gly Ser Ala Arg Leu Met Val Ala Gly Val Asp Val
565 570 575

Trp Leu Asn Asn Pro Arg Arg Pro Leu Glu Ala Ser Gly Thr Ser Gly
580 585 590

Met Lys Ala Gly Leu Asn Gly Val Leu Asn Ala Ser Ile Phe Asp Gly
595 600 605

Trp Trp Val Glu Gly Tyr Asn Gly Lys Asn Gly Trp Val Ile Gly Glu
610 615 620

Glu Thr Thr Glu Pro Glu Ser Glu Glu Asp Asp Ala Lys Asp Ala Glu
625 630 635 640

Ser Leu Tyr Thr Leu Leu Glu Lys Glu Ile Ile Pro Thr Tyr Tyr Gly
645 650 655

Asn Arg Ser Arg Trp Ile Tyr Met Met Lys Glu Ser Ile Lys Ser Ile
660 665 670

Ala Pro Arg Phe Ser Thr His Arg Met Val Lys Glu Tyr Met Asp Arg
675 680 685

Phe Tyr Ser Lys Ala Met Ser Asn Tyr Ile Trp Leu Thr Arg Glu Asn
690 695 700

Tyr Lys Gly Ala Arg Glu Ile Ala Ala Trp Lys Glu Arg
705 710 715